# EUROPEAN PATENT APPLICATION

(11) **EP 4 545 565 A1**
(43) Date of publication of application: **30.04.2025**
(21) Application number: 23826536.7
(22) Date of filing: 21.06.2023
(51) Int. Cl.: C07K 16/46, C07K 16/28, A61K 39/395, A61P 35/00, C12N 15/13

(54) **BISPECIFIC ANTIBODY BINDING EGFR AND B7-H3**

(30) Priority: 23.06.2022 CN 202210724625; 10.01.2023 CN 202310032503
(71) Applicant: Fortvita Biologics (Singapore) Pte. Ltd., Singapore (SG)
(72) Inventor: HE, Kaijie, Suzhou, Jiangsu 215123 (CN); ZHOU, Shuaixiang, Suzhou, Jiangsu 215123 (CN); GUAN, Jian, Suzhou, Jiangsu 215123 (CN); LI, Yaning, Suzhou, Jiangsu 215123 (CN); LIU, Xiyang, Suzhou, Jiangsu 215123 (CN)
(74) Representative: Sagittarius IP
(86) International application number: PCT/CN2023/101807
(87) International publication number: WO 2023/246891

(57) **Abstract**

The present disclosure provides a novel, artificially designed bispecific antibody molecule, in particular an anti-B7-H3/EGFR bispecific antibody molecule, which can simultaneously bind to B7-H3 and EGFR.

## Description

### FIELD OF THE INVENTION

The present disclosure generally relates to the field of immunology and antibody engineering. Specifically, the present disclosure relates to a novel, artificially designed bispecific antibody molecule, in particular, to a bispecific antibody simultaneously binding to EGFR and B7-H3, a polynucleotide encoding the antibody molecule or chains thereof, a vector comprising the polynucleotide, a host cell comprising the polynucleotide or the vector, an immunoconjugate and a pharmaceutical composition comprising the antibody molecule, and use of the antibody molecule in the immunotherapy, prevention, and/or diagnosis of a disease.

### BACKGROUND

Globally, lung cancer is one of the most common types of cancer tumors, and non-small cell lung cancer (NSCLC) accounts for about 80%-85% of all lung cancer cases. NSCLC can be classified into common types such as lung adenocarcinoma, lung squamous cell carcinoma, and large cell lung carcinoma. Epidermal growth factor receptor (EGFR) is a tyrosine kinase receptor, a large transmembrane glycoprotein with a molecular weight of about 170 kDa, and a member of the ErbB receptor family. EGFR is the most common oncogenic driver gene in NSCLC, with EGFR mutations responsible for about 40% of Asian NSCLC patients and about 15% in Caucasian populations (Gower A., Wang Y, Giaccone G. Oncogenic drivers, targeted therapies, and acquired resistance in non-small-cell lung cancer, J. Mol. Med., 2014; 92: 697-707.). EGFR overexpression and/or abnormal mutational activation is also seen in patients with other cancer tumors, e.g., cancers of epithelial source, such as kidney cancer, prostate cancer, pancreatic cancer, breast cancer, colon cancer, and head and neck cancer.

Aiming at the abnormal activation and amplification of NSCLC-EGFR, targeted therapies with EGFR-TKI small molecule inhibitors (e.g., gefitinib, erlotinib, afatinib, dacomitinib and osimertinib, and almonertinib) and biological monoclonal antibody macromolecules (cetuximab, panitumumab, necitumumab, and nimotuzumab) are available. Although the EGFR-TKI small molecule inhibitors are still the standard method for treating the non-small cell lung cancer (NSCLC), the EGFR-TKI small molecules mainly target patients with structural mutations in the tyrosine kinase that alter its activity and often become ineffective due to resistance caused by mutations in the target genes. Therefore, there is a continuous need for the research and development of new targeted drugs against new mutation sites, which greatly limits the clinical use of such drugs and poses a serious challenge to the entire industry of EGFR-TKI small molecules.

An activating mutation region of EGFR mainly occurs in the tyrosine kinase domain of EGFR exons 18-21 (Jiyeon Yun, Soo-Hwan Lee, Seok-Young Kim, et al., Antitumor activity of Amivantamab (JNJ-61186372), an EGFR-MET bispecific antibody, in diverse models of EGFR exon 20 insertion-driven NSCLC, Cancer Discov., 2020; 10: 1194-209.). A binding region of an EGFR antibody is mainly located in an extracellular ligand domain region of EGFR, which can prevent the occurrence of resistance mutations. At the same time, the EGFR antibody can inhibit the growth of tumor cells by inhibiting the binding of EGFR to a ligand, and can also kill tumors with immune cells by utilizing its specific ADCC (antibody-dependent cell-mediated cytotoxicity), such that an anti-tumor killing role can be played jointly through multiple mechanisms of action.

B7-H3 (also referred to as CD276) is a type I transmembrane protein (Picarda E., Ohaegbulam K. C., Zang X., Molecular pathways: targeting B7-H3 (CD276) for human cancer immunotherapy, Clin Cancer Res., 2016; 22: 3425-31. and Yang S., Wei W., Zhao Q., B7-H3, a checkpoint molecule, as a target for cancer immunotherapy, Int J Biol Sci., 202016: 1767-73), which is very similar in structure to PD-L1, both belonging to the B7/CD28 superfamily. B7-H3 is expressed at low levels in most normal human tissues, but abnormally overexpressed in tumor cells, e.g., lung cancer, colorectal cancer, head and neck cancer, breast cancer, ovarian cancer, and pancreatic cancer (Lee Y. H., Martin-Orozco N., Zheng P., Li J., Zhang P., Tan H., et al., Inhibition of the B7-H3 immune checkpoint limits tumor growth by enhancing cytotoxic lymphocyte function, Cell Res., 2017; 27: 1034-45. and Kontos F., Michelakos T., Kurokawa T., Sadagopan A., Schwab J. H., Ferrone C. R., et al., B7-H3: an attractive target for antibody-based immunotherapy, Clin Cancer Res., 2020. https://doi.org/10.1158/1078-0432.CCR-20-2584. and Seaman S., Zhu Z., Saha S., Zhang X. M., Yang M. Y., Hilton M. B., et al., Eradication of tumors through simultaneous ablation of CD276/B7-H3-positive tumor cells and tumor vasculature, Cancer Cell, 2017; 31: 501-15). The B7-H3 receptor has not been identified, but in tumor immunity, B7-H3 may be involved in the immune function regulation of toxic lymphocytes (Kraan J., van den Broek P., Verhoef C., Grunhagen D. J., Taal W., Gratama J. W., et al., Endothelial CD276 (B7-H3) expression is increased in human malignancies and distinguishes between normal and tumor-derived circulating endothelial cells, Br. J. Cancer, 2014; 111: 149-56). There is evidence showing that B7-H3 expression may be correlated with the expression of EGFR gene and the effect of anti-PD-1 therapy (Yonesaka K., Haratani K., Takamura S., Sakai H., Kato R., Takegawa N., et al., B7-H3 negatively modulates CTL-mediated cancer immunity, Clin Cancer Res., 2018; 24: 2653-64). Because B7-H3 has a good selective expression profile, B7-H3 monoclonal monomers or B7-H3-ADC drugs have been developed by biological research and development companies and are used for research and treatment in the field of related tumor diseases.

Antibody molecules capable of targeted specific binding to corresponding antigens thereof are becoming important therapeutic agents, preventive agents, and/or diagnostic agents for a variety of diseases (e.g., cancers, autoimmune diseases, inflammatory diseases, and infectious diseases).

However, monospecific antibodies against a single target have some limitations in clinical applications. Patients may develop resistance or no response after treatment with monospecific antibodies. With researches on cancers and many other diseases, it is recognized that there are often multiple signal transduction pathways involved in the development and progression of diseases, and a single-target immunotherapy is usually less effective in treating many diseases.

Multispecific antibodies (e.g., bispecific antibodies) can be designed to simultaneously act on signal transduction pathways of two or more different mediators since they are capable of specifically binding to different antigens. These advantages have expanded the application of multispecific antibodies (e.g., bispecific antibodies).

There is still a need in the art for alternative bispecific antibodies having improved properties, which are capable of binding to different antigens simultaneously, in particular EGFR and B7-H3, and retaining the binding activity of antigen-binding sites to the corresponding various epitopes, as well as other properties. At the same time, it is desirable to obtain bispecific antibody formats having certain stability and better productivity and developability; such bispecific antibody formats are physically and biologically stable, which allows for better productivity and developability of the antibody.

### SUMMARY

A first aspect of the present disclosure relates to an antibody, which comprises an antigen-binding region specifically recognizing EGFR (e.g., human EGFR) and an antigen-binding region specifically binding to B7-H3. In some embodiments, the antibody is a multispecific antibody, e.g., a bispecific antibody.

For the antibody or the antigen-binding fragment thereof binding to EGFR and B7-H3 provided by the present disclosure, the efficacy and safety selectivity of the antibody molecule are improved by reducing the affinity for EGFR and increasing the affinity for B7-H3; meanwhile, antibody-dependent cell-mediated cytotoxicity (ADCC) is enhanced by using the GlymaxX low-fucose technology. Compared with amivantamab (JNJ-372) used for the NSCLC-EGFR exon 20 insertion mutation (exon20ins), B7-H3 has a broader expression profile than cMET, offering a wider range of applications in tumor treatment; at the same time, the introduction of the B7-H3 parent not only improves the blocking activity of the EGFR antibody within the bispecific antibody but also improves the overall ADCC effect of the bispecific antibody.

The bispecific antibody or the antigen-binding fragment thereof binding to EGFR and B7-H3 provided by the present disclosure has one or more of the following properties:
(a) specifically binding to one or two antigens with high affinity;
(b) easily expressed in culture cells *in vitro,* and chains of the antibody molecule being capable of correct coupling or pairing;
(c) having good physical stability, in particular, having good long-term thermal stability, and being capable of maintaining biological activities for a long time;
(d) exerting biological functions through regulating (e.g., inhibiting or activating) signaling pathways involving one or two antigens after specifically binding thereto;
(e) exerting effector functions;
(f) having better anti-tumor activity.

In one embodiment, different antigen-binding sites bind to the same epitope or different epitopes on the same antigen.

In one embodiment, a first antigen-binding region or a second antigen-binding region is derived from a human, or humanized, or chimeric antibody.

In some embodiments, the antibody of the present disclosure, e.g., a bispecific antibody, further comprises a heavy chain constant region. In one embodiment, the heavy chain constant domain is derived from IgG1. It should be understood that an Fc in the constant domain may be mutated to stabilize the antibody or to enhance the effector function.

In one embodiment, the first antigen-binding region is specific for a first antigen; in one embodiment, the first antigen is EGFR.

In one embodiment, the second antigen-binding region is specific for a second antigen; in one embodiment, the second antigen is B7-H3.

The antibody of the present disclosure may also comprise other antigen-binding regions binding to other antigens to constitute multispecific antibodies. The types of other antigens to which the antibody molecule of the present disclosure specifically binds are not particularly limited, and the antigen may be, e.g., a cytokine, a growth factor, a hormone, a signaling protein, an inflammatory mediator, a ligand, a cell surface receptor, or a fragment thereof. In one embodiment, the other antigens to which the antibody molecule of the present disclosure specifically binds are selected from tumor-associated antigens, immune checkpoint molecules, angiogenesis-inducing factors, members of the tumor necrosis factor receptor superfamily, and co-stimulatory molecules in the immune system, as well as ligands and/or receptors for these molecules.

In one aspect, the present disclosure provides a nucleic acid encoding any one or more polypeptide chains in the antibody molecule of the present disclosure, a vector comprising the nucleic acid, and a host cell comprising the nucleic acid or the vector.

In one aspect, the present disclosure provides a vector comprising a polynucleotide encoding any one or more polypeptide chains in the antibody molecule of the present disclosure; preferably, the vector is an expression vector, e.g., pcDNA3.1.

In one aspect, the present disclosure provides a method for producing the antibody molecule or the fragment thereof of the present disclosure.

In some embodiments, the present disclosure provides an immunoconjugate, a pharmaceutical composition, a kit, a combination product, or an article of manufacture comprising the antibody of the present disclosure.

In some embodiments, the antibody, the pharmaceutical composition, the immunoconjugate, the combination product, or the kit of the present disclosure is used for preventing or treating a disease, e.g., an acute or a chronic inflammatory disease, an infection (e.g., chronic infection), or a tumor. For example, the disease is a tumor (e.g., cancer) or an infection. In some embodiments, the tumor is a tumor immune escape. Preferably, the tumor is a gastrointestinal tumor, a lung tumor, or a skin tumor. In some embodiments, the infection is a chronic infection.

In another aspect, the present disclosure relates to a method for preventing or treating a disease in a subject or an individual, wherein the method comprises administering to the subject an effective amount of any of the antibodies or the fragments thereof, the pharmaceutical composition, the immunoconjugate, the combination product, or the kit described herein. For example, the disease is a tumor (e.g., cancer) or an infection. In some embodiments, the tumor is a tumor immune escape. In one embodiment, the tumor is a gastrointestinal tumor, a lung tumor, or a skin tumor. In one embodiment, the infection is a chronic infection.

In another aspect, the present disclosure also relates to use of any of the antibodies or fragments thereof or the immunoconjugate described herein in the preparation of a drug, a pharmaceutical composition, a kit, or a combination product for treating a tumor (e.g., cancer) or an infection in a subject. In some embodiments, the tumor is a tumor immune escape. In one embodiment, the tumor is a gastrointestinal tumor, a lung tumor, or a skin tumor. In one embodiment, the infection is a chronic infection.

The present disclosure also relates to a method for detecting an antigen in a sample.

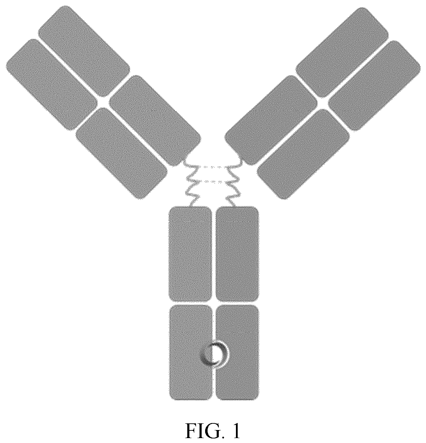

In another aspect, the present disclosure relates to the following specific embodiments.
1. A bispecific antibody binding to EGFR and B7-H3, which comprises a first antigen-binding region and a second antigen-binding region, wherein the first antigen-binding region specifically binds to EGFR, and the second antigen-binding region specifically binds to B7H3.
2. The bispecific antibody according to embodiment 1, wherein the second antigen-binding region comprises sequences of an HCDR1, an HCDR2, and an HCDR3 of a heavy chain variable region set forth in SEQ ID NO: 3, 5, or 7, and sequences of an LCDR1, an LCDR2, and an LCDR3 of a light chain variable region set forth in SEQ ID NO: 4, 6, or 8; preferably, the HCDR1 is based on the Abm scheme, and the HCDR2, the HCDR3, the LCDR1, the LCDR2, and the LCDR3 are based on the Kabat scheme.
3. The bispecific antibody according to embodiment 1, wherein the second antigen-binding region comprises an HCDR1, an HCDR2, and an HCDR3 of a heavy chain variable region VH, and an LCDR1, an LCDR2, and an LCDR3 of a light chain variable region VL, wherein
   (i) the HCDR1, the HCDR2, and the HCDR3 are three complementarity determining regions HCDR1, HCDR2, and HCDR3 comprised in a VH set forth in SEQ ID NO: 3; and the LCDR1, the LCDR2, and the LCDR3 are three complementarity determining regions LCDR1, LCDR2, and LCDR3 comprised in a VL set forth in SEQ ID NO: 4;
   (ii) the HCDR1, the HCDR2, and the HCDR3 are three complementarity determining regions HCDR1, HCDR2, and HCDR3 comprised in a VH set forth in SEQ ID NO: 5; and the LCDR1, the LCDR2, and the LCDR3 are three complementarity determining regions LCDR1, LCDR2, and LCDR3 comprised in a VL set forth in SEQ ID NO: 6; or
   (iii) the HCDR1, the HCDR2, and the HCDR3 are three complementarity determining regions HCDR1, HCDR2, and HCDR3 comprised in a VH set forth in SEQ ID NO: 7; and the LCDR1, the LCDR2, and the LCDR3 are three complementarity determining regions LCDR1, LCDR2, and LCDR3 comprised in a VL set forth in SEQ ID NO: 8;
   preferably, the HCDR1 is based on the Abm scheme, and the HCDR2, the HCDR3, the LCDR1, the LCDR2, and the LCDR3 are based on the Kabat scheme.
4. The bispecific antibody according to embodiment 1, wherein the second antigen-binding region comprises an HCDR1, an HCDR2, and an HCDR3 of a heavy chain variable region VH, and an LCDR1, an LCDR2, and an LCDR3 of a light chain variable region VL, wherein
   (i) the HCDR1 comprises or consists of an amino acid sequence set forth in SEQ ID NO: 15, the HCDR2 comprises or consists of an amino acid sequence set forth in SEQ ID NO: 16, and the HCDR3 comprises or consists of an amino acid sequence set forth in SEQ ID NO: 17;
      the LCDR1 comprises or consists of an amino acid sequence set forth in SEQ ID NO: 18, the LCDR2 comprises or consists of an amino acid sequence set forth in SEQ ID NO: 19, and the LCDR3 comprises or consists of an amino acid sequence set forth in SEQ ID NO: 20; or,
   (ii) the HCDR1 comprises or consists of an amino acid sequence set forth in SEQ ID NO: 21, the HCDR2 comprises or consists of an amino acid sequence set forth in SEQ ID NO: 22, and the HCDR3 comprises or consists of an amino acid sequence set forth in SEQ ID NO: 23;
      the LCDR1 comprises or consists of an amino acid sequence set forth in SEQ ID NO: 24, the LCDR2 comprises or consists of an amino acid sequence set forth in SEQ ID NO: 25, and the LCDR3 comprises or consists of an amino acid sequence set forth in SEQ ID NO: 26; or,
   (iii) the HCDR1 comprises or consists of an amino acid sequence set forth in SEQ ID NO: 27, the HCDR2 comprises or consists of an amino acid sequence set forth in SEQ ID NO: 28, and the HCDR3 comprises or consists of an amino acid sequence set forth in SEQ ID NO: 29;
   the LCDR1 comprises or consists of an amino acid sequence set forth in SEQ ID NO: 30, the LCDR2 comprises or consists of an amino acid sequence set forth in SEQ ID NO: 31, and the LCDR3 comprises or consists of an amino acid sequence set forth in SEQ ID NO: 32.
5. The bispecific antibody according to any one of embodiments 1-4, wherein the second antigen-binding region comprises a heavy chain variable region VH, wherein the VH comprises or consists of an amino acid sequence set forth in SEQ ID NO: 3, 5, or 7, or an amino acid sequence having at least 80%, 85%, 90%, 91%, 92%, 93%, 94%, 95%, 96%, 97%, 98%, or 99% sequence identity to the amino acid sequence set forth in SEQ ID NO: 3, 5, or 7.
6. The bispecific antibody according to any one of embodiments 1-5, wherein the second antigen-binding region comprises a light chain variable region VL, wherein the VL comprises or consists of an amino acid sequence set forth in SEQ ID NO: 4, 6, or 8, or an amino acid sequence having at least 80%, 85%, 90%, 91%, 92%, 93%, 94%, 95%, 96%, 97%, 98%, or 99% sequence identity to the amino acid sequence set forth in SEQ ID NO: 4, 6, or 8.
7. The bispecific antibody according to any one of embodiments 1-6, wherein the second antigen-binding region comprises a heavy chain variable region VH and a light chain variable region VL, wherein
   (i) the VH comprises or consists of an amino acid sequence set forth in SEQ ID NO: 3 or an amino acid sequence having at least 80%, 85%, 90%, 91%, 92%, 93%, 94%, 95%, 96%, 97%, 98%, or 99% identity to the amino acid sequence of SEQ ID NO: 3, and the VL comprises or consists of an amino acid sequence set forth in SEQ ID NO: 4 or an amino acid sequence having at least 80%, 85%, 90%, 91%, 92%, 93%, 94%, 95%, 96%, 97%, 98%, or 99% identity to the amino acid sequence of SEQ ID NO: 4;
   (ii) the VH comprises or consists of an amino acid sequence set forth in SEQ ID NO: 5 or an amino acid sequence having at least 80%, 85%, 90%, 91%, 92%, 93%, 94%, 95%, 96%, 97%, 98%, or 99% identity to the amino acid sequence of SEQ ID NO: 5, and the VL comprises or consists of an amino acid sequence set forth in SEQ ID NO: 6 or an amino acid sequence having at least 80%, 85%, 90%, 91%, 92%, 93%, 94%, 95%, 96%, 97%, 98%, or 99% identity to the amino acid sequence of SEQ ID NO: 6; or
   (iii) the VH comprises or consists of an amino acid sequence set forth in SEQ ID NO: 7 or an amino acid sequence having at least 80%, 85%, 90%, 91%, 92%, 93%, 94%, 95%, 96%, 97%, 98%, or 99% identity to the amino acid sequence of SEQ ID NO: 7, and the VL comprises or consists of an amino acid sequence set forth in SEQ ID NO: 8 or an amino acid sequence having at least 80%, 85%, 90%, 91%, 92%, 93%, 94%, 95%, 96%, 97%, 98%, or 99% identity to the amino acid sequence of SEQ ID NO: 8.
8. The bispecific antibody according to any one of embodiments 1-7, wherein the second antigen-binding region comprises a heavy chain variable region VH and a light chain variable region VL, wherein the VH and the VL comprise or consist of, respectively, amino acid sequences set forth in:
   SEQ ID NO: 3 and SEQ ID NO: 4;
   SEQ ID NO: 5 and SEQ ID NO: 6; or
   SEQ ID NO: 7 and SEQ ID NO: 8.
9. The bispecific antibody according to any one of embodiments 1-7, wherein the first antigen-binding region comprises an HCDR1, an HCDR2, and an HCDR3 of a heavy chain variable region VH, and an LCDR1, an LCDR2, and an LCDR3 of a light chain variable region VL, wherein
   the HCDR1, the HCDR2, and the HCDR3 are three complementarity determining regions HCDR1, HCDR2, and HCDR3 comprised in a VH set forth in SEQ ID NO: 1; and the LCDR1, the LCDR2, and the LCDR3 are three complementarity determining regions LCDR1, LCDR2, and LCDR3 comprised in a VL set forth in SEQ ID NO: 2;
   preferably, the CDRs are determined using the Kabat scheme.
10. The bispecific antibody according to embodiment 9, wherein the HCDR1 of the first antigen-binding region comprises or consists of an amino acid sequence of SEQ ID NO: 9; the HCDR2 comprises or consists of an amino acid sequence of SEQ ID NO: 10; the HCDR3 comprises or consists of an amino acid sequence of SEQ ID NO: 11; and the LCDR1 of the first antigen-binding region comprises or consists of an amino acid sequence of SEQ ID NO: 12; the LCDR2 comprises or consists of an amino acid sequence of SEQ ID NO: 13; and the LCDR3 comprises or consists of an amino acid sequence of SEQ ID NO: 14.
11. The bispecific antibody according to embodiment 9 or 10, wherein the first antigen-binding region comprises a heavy chain variable region VH, wherein the VH comprises or consists of an amino acid sequence set forth in SEQ ID NO: 1 or an amino acid sequence having at least 80%, 85%, 90%, 91%, 92%, 93%, 94%, 95%, 96%, 97%, 98%, or 99% identity to the amino acid sequence of SEQ ID NO: 1.
12. The bispecific antibody according to any one of embodiments 9-11, wherein the first antigen-binding region comprises a light chain variable region VL, wherein the VL comprises or consists of an amino acid sequence set forth in SEQ ID NO: 2 or an amino acid sequence having at least 80%, 85%, 90%, 91%, 92%, 93%, 94%, 95%, 96%, 97%, 98%, or 99% identity to the amino acid sequence of SEQ ID NO: 2.
13. The bispecific antibody according to any one of embodiments 9-12, wherein the first antigen-binding region comprises a heavy chain variable region VH and a light chain variable region VL, wherein the VH comprises or consists of an amino acid sequence set forth in SEQ ID NO: 1 or an amino acid sequence having at least 80%, 85%, 90%, 91%, 92%, 93%, 94%, 95%, 96%, 97%, 98%, or 99% identity to the amino acid sequence of SEQ ID NO: 1, and the VL comprises or consists of an amino acid sequence set forth in SEQ ID NO: 2 or an amino acid sequence having at least 80%, 85%, 90%, 91%, 92%, 93%, 94%, 95%, 96%, 97%, 98%, or 99% identity to the amino acid sequence of SEQ ID NO: 2.
14. The bispecific antibody according to any one of embodiments 9-13, wherein the first antigen-binding region comprises a heavy chain variable region VH and a light chain variable region VL, wherein the VH and the VL comprise or consist of, respectively, amino acid sequences set forth in: SEQ ID NO: 1 and SEQ ID NO: 2.
15. The bispecific antibody according to any one of embodiments 1-14, wherein the first antigen-binding region specifically binding to EGFR comprises an HCDR1, an HCDR2, and an HCDR3 of a heavy chain variable region VH, and an LCDR1, an LCDR2, and an LCDR3 of a light chain variable region VL, wherein
   the HCDR1 comprises or consists of an amino acid sequence of SEQ ID NO: 9;
   the HCDR2 comprises or consists of an amino acid sequence of SEQ ID NO: 10;
   the HCDR3 comprises or consists of an amino acid sequence of SEQ ID NO: 11;
   the LCDR1 comprises or consists of an amino acid sequence of SEQ ID NO: 12;
   the LCDR2 comprises or consists of an amino acid sequence of SEQ ID NO: 13; and
   the LCDR3 comprises or consists of an amino acid sequence of SEQ ID NO: 14;
      and
   the second antigen-binding region specifically binds to B7H3, and comprises an HCDR1, an HCDR2, and an HCDR3 of a heavy chain variable region VH, and an LCDR1, an LCDR2, and an LCDR3 of a light chain variable region VL, wherein
      (i) the HCDR1 comprises or consists of an amino acid sequence of SEQ ID NO: 15;
         the HCDR2 comprises or consists of an amino acid sequence of SEQ ID NO: 16;
         the HCDR3 comprises or consists of an amino acid sequence of SEQ ID NO: 17;
         the LCDR1 comprises or consists of an amino acid sequence of SEQ ID NO: 18;
         the LCDR2 comprises or consists of an amino acid sequence of SEQ ID NO: 19; and
         the LCDR3 comprises or consists of an amino acid sequence of SEQ ID NO: 20;
      (ii) the HCDR1 comprises or consists of an amino acid sequence of SEQ ID NO: 21;
         the HCDR2 comprises or consists of an amino acid sequence of SEQ ID NO: 22;
         the HCDR3 comprises or consists of an amino acid sequence of SEQ ID NO: 23;
         the LCDR1 comprises or consists of an amino acid sequence of SEQ ID NO: 24;
         the LCDR2 comprises or consists of an amino acid sequence of SEQ ID NO: 25; and
         the LCDR3 comprises or consists of an amino acid sequence of SEQ ID NO: 26; or
      (iii) the HCDR1 comprises or consists of an amino acid sequence of SEQ ID NO: 27;
         the HCDR2 comprises or consists of an amino acid sequence of SEQ ID NO: 28;
         the HCDR3 comprises or consists of an amino acid sequence of SEQ ID NO: 29;
         the LCDR1 comprises or consists of an amino acid sequence of SEQ ID NO: 30;
         the LCDR2 comprises or consists of an amino acid sequence of SEQ ID NO: 31; and
         the LCDR3 comprises or consists of an amino acid sequence of SEQ ID NO: 32.
16. The bispecific antibody according to embodiment 15, wherein the first antigen-binding region comprises a VH comprising or consisting of an amino acid sequence set forth in SEQ ID NO: 1 and a VL comprising or consisting of an amino acid sequence set forth in SEQ ID NO: 2, and the second antigen-binding region comprises a VH and a VL comprising or consisting of, respectively, amino acid sequences set forth in:
   SEQ ID NO: 3 and SEQ ID NO: 4;
   SEQ ID NO: 5 and SEQ ID NO: 6; or
   SEQ ID NO: 7 and SEQ ID NO: 8.
17. The bispecific antibody according to any one of embodiments 1-16, which comprises an Fc region, wherein preferably, the Fc region has low fucosylation, e.g., low fucosylation obtained after treatment by the GlymaxX technology.
18. The bispecific antibody according to embodiment 17, which comprises a first Fc region and a second Fc region, wherein the first Fc region and the second Fc region are identical or different.
19. The bispecific antibody according to embodiment 17 or 18, wherein the first Fc region and the second Fc region are each a human IgG Fc, e.g., human IgG1 Fc, human IgG2 Fc, human IgG3 Fc, or human IgG4 Fc, e.g., comprising or consisting of an amino acid sequence set forth in SEQ ID NO: 46 or 47, or an amino acid sequence having at least 90% identity, e.g., 95%, 96%, 97%, 99% or more identity, thereto.
20. The bispecific antibody according to embodiment 18 or 19, wherein mutations that promote heterodimerization of the first Fc region and the second Fc region are introduced in the first Fc region and the second Fc region.
21. The bispecific antibody according to embodiment 20, wherein the mutations are introduced on the basis of the Innobody technology.
22. The bispecific antibody according to embodiment 21, wherein a CH3 of one Fc region comprises S364R and D399K mutations, and a CH3 of the other Fc region comprises Y349T, K370S, and K409D mutations.
23. The bispecific antibody according to embodiment 22, wherein
   a) one Fc region polypeptide comprises or consists of an amino acid sequence set forth in SEQ ID NO: 49 or 50, and the other Fc region polypeptide comprises or consists of an amino acid sequence set forth in SEQ ID NO: 52 or 53;
   b) one Fc region polypeptide comprises an amino acid sequence having at least 85%, 90%, 95%, 96%, 97%, 98%, or 99% identity to the amino acid sequence set forth in SEQ ID NO: 49 or 50, and the other Fc region polypeptide comprises an amino acid sequence having at least 85%, 90%, 95%, 96%, 97%, 98%, or 99% identity to the amino acid sequence set forth in SEQ ID NO: 52 or 53; or
   c) one Fc region polypeptide comprises an amino acid sequence having at least 85%, 90%, 95%, 96%, 97%, 98%, or 99% identity to the amino acid sequence set forth in SEQ ID NO: 49 or 50 and comprises mutations Y349T, K370S, and K409D, and the other Fc region comprises an amino acid sequence having at least 85%, 90%, 95%, 96%, 97%, 98%, or 99% identity to the amino acid sequence set forth in SEQ ID NO: 52 or 53 and comprises mutations S364R and D399K.
24. The bispecific antibody according to embodiment 20, wherein the mutations are introduced based on the Knob-into-Hole technology, wherein corresponding Knob and Hole mutations are introduced into the first Fc region and the second Fc region.
25. The bispecific antibody according to embodiment 24, wherein
   a) one Fc region polypeptide comprises a mutation T366W, and the other Fc region polypeptide comprises T366S, L368A, and Y407V (numbering according to EU index), or
   b) one Fc region comprises amino acid substitutions S354C and T366W, and the other Fc region comprises amino acid substitutions Y349C, T366S, L368A, and Y407V (numbering according to EU index).
26. The bispecific antibody according to any one of embodiments 1-25, wherein the first and/or second antigen-binding regions (e.g., the heavy chain variable region therein) may also be linked to 1 or 2 heavy chain constant regions (e.g., a heavy chain constant region of human IgG1, human IgG2, human IgG3, or human IgG4) comprising a CH1 and an Fc region, via or not via a hinge region, for example, the C-terminus of the heavy chain variable region is linked to the N-terminus of the CH1 of the heavy chain constant region.
27. The bispecific antibody according to embodiment 26, wherein the CH1 comprises or consists of an amino acid sequence set forth in SEQ ID NO: 42 or an amino acid sequence having at least 80%, 85%, 90%, 91%, 92%, 93%, 94%, 95%, 96%, 97%, 98%, or 99% identity to the amino acid sequence of SEQ ID NO: 42.
28. The bispecific antibody according to embodiments 1-27, wherein the first and/or second antigen-binding regions (e.g., the light chain variable region therein) may also be linked to a light chain constant region, for example, the C-terminus of the light chain variable region is linked to the N-terminus of the light chain constant region.
29. The bispecific antibody according to embodiment 28, wherein the light chain constant region is a kappa light chain constant region or a lambda light chain constant region.
30. The bispecific antibody according to embodiment 20, wherein the light chain constant region comprises or consists of an amino acid sequence set forth in SEQ ID NO: 54 or an amino acid sequence having at least 80%, 85%, 90%, 91%, 92%, 93%, 94%, 95%, 96%, 97%, 98%, or 99% identity to the amino acid sequence of SEQ ID NO: 54.
31. The bispecific antibody according to any one of embodiments 1-30, wherein the bispecific antibody is an IgG-like antibody having a configuration shown in FIG. 1.
32. The bispecific antibody according to embodiment 31, which comprises a heavy chain 1 and a light chain 1, and a heavy chain 2 and a light chain 2, wherein the heavy chain 1 and the light chain 1 constitute a first half antibody, and the heavy chain 2 and the light chain 2 constitute a second half antibody, wherein
   the heavy chain 1 comprises the heavy chain variable region of the first antigen-binding region and a first heavy chain constant region; the light chain 1 comprises the light chain variable region of the first antigen-binding region and a first light chain constant region; and the heavy chain 2 comprises the heavy chain variable region of the second antigen-binding region and a second heavy chain constant region; the light chain 2 comprises the light chain variable region of the second antigen-binding region and a second light chain constant region.
33. The bispecific antibody according to embodiment 32, wherein the heavy chain 1 comprises or consists of an amino acid sequence set forth in SEQ ID NO: 33 or an amino acid sequence having at least 80%, 85%, 90%, 91%, 92%, 93%, 94%, 95%, 96%, 97%, 98%, or 99% identity to the amino acid sequence of SEQ ID NO: 33.
34. The bispecific antibody according to embodiment 32 or 33, wherein the light chain 1 comprises or consists of an amino acid sequence set forth in SEQ ID NO: 34 or an amino acid sequence having at least 80%, 85%, 90%, 91%, 92%, 93%, 94%, 95%, 96%, 97%, 98%, or 99% identity to the amino acid sequence of SEQ ID NO: 34.
35. The bispecific antibody according to any one of embodiments 32-34, wherein the heavy chain 1 comprises or consists of an amino acid sequence set forth in SEQ ID NO: 33 or an amino acid sequence having at least 80%, 85%, 90%, 91%, 92%, 93%, 94%, 95%, 96%, 97%, 98%, or 99% identity to the amino acid sequence of SEQ ID NO: 33, and the light chain 1 comprises or consists of an amino acid sequence set forth in SEQ ID NO: 34 or an amino acid sequence having at least 80%, 85%, 90%, 91%, 92%, 93%, 94%, 95%, 96%, 97%, 98%, or 99% identity to the amino acid sequence of SEQ ID NO: 34.
36. The bispecific antibody according to any one of embodiments 32-35, wherein the heavy chain 2 comprises or consists of an amino acid sequence set forth in SEQ ID NO: 35, 37, or 39 or an amino acid sequence having at least 80%, 85%, 90%, 91%, 92%, 93%, 94%, 95%, 96%, 97%, 98%, or 99% identity to the amino acid sequence set forth in SEQ ID NO: 35, 37, or 39.
37. The bispecific antibody according to any one of embodiments 32-36, wherein the light chain 2 comprises or consists of an amino acid sequence set forth in SEQ ID NO: 36, 38, or 40 or an amino acid sequence having at least 80%, 85%, 90%, 91%, 92%, 93%, 94%, 95%, 96%, 97%, 98%, or 99% identity to the amino acid sequence set forth in SEQ ID NO: 36, 38, or 40.
38. The bispecific antibody according to any one of embodiments 32-37, wherein
   (1) the heavy chain 2 comprises or consists of an amino acid sequence set forth in SEQ ID NO: 35 or an amino acid sequence having at least 80%, 85%, 90%, 91%, 92%, 93%, 94%, 95%, 96%, 97%, 98%, or 99% identity to the amino acid sequence set forth in SEQ ID NO: 35; and the light chain 2 comprises or consists of an amino acid sequence set forth in SEQ ID NO: 36 or an amino acid sequence having at least 80%, 85%, 90%, 91%, 92%, 93%, 94%, 95%, 96%, 97%, 98%, or 99% identity to the amino acid sequence set forth in SEQ ID NO: 36;
   (2) the heavy chain 2 comprises or consists of an amino acid sequence set forth in SEQ ID NO: 37 or an amino acid sequence having at least 80%, 85%, 90%, 91%, 92%, 93%, 94%, 95%, 96%, 97%, 98%, or 99% identity to the amino acid sequence set forth in SEQ ID NO: 37; and the light chain 2 comprises or consists of an amino acid sequence set forth in SEQ ID NO: 38 or an amino acid sequence having at least 80%, 85%, 90%, 91%, 92%, 93%, 94%, 95%, 96%, 97%, 98%, or 99% identity to the amino acid sequence set forth in SEQ ID NO: 38;
   (3) the heavy chain 2 comprises or consists of an amino acid sequence set forth in SEQ ID NO: 39 or an amino acid sequence having at least 80%, 85%, 90%, 91%, 92%, 93%, 94%, 95%, 96%, 97%, 98%, or 99% identity to the amino acid sequence set forth in SEQ ID NO: 39; and the light chain 2 comprises or consists of an amino acid sequence set forth in SEQ ID NO: 40 or an amino acid sequence having at least 80%, 85%, 90%, 91%, 92%, 93%, 94%, 95%, 96%, 97%, 98%, or 99% identity to the amino acid sequence set forth in SEQ ID NO: 40.
39. The bispecific antibody according to any one of embodiments 32-38, wherein
   the heavy chain 1 comprises or consists of an amino acid sequence set forth in SEQ ID NO: 33 or an amino acid sequence having at least 80%, 85%, 90%, 91%, 92%, 93%, 94%, 95%, 96%, 97%, 98%, or 99% identity to the amino acid sequence of SEQ ID NO: 33, and the light chain 1 comprises or consists of an amino acid sequence set forth in SEQ ID NO: 34 or an amino acid sequence having at least 80%, 85%, 90%, 91%, 92%, 93%, 94%, 95%, 96%, 97%, 98%, or 99% identity to the amino acid sequence of SEQ ID NO: 34; and the heavy chain 2 and the light chain 2 comprise or consist of, respectively, amino acid sequences set forth in the following SEQ ID NOs or amino acid sequences having at least 85%, 90%, 95%, 96%, 97%, 98%, or 99% identity thereto:
   i) SEQ ID NO: 35 and SEQ ID NO: 36;
   ii) SEQ ID NO: 37 and SEQ ID NO: 38;
   iii) SEQ ID NO: 39 and SEQ ID NO: 40.
40. The bispecific antibody according to any one of embodiments 32-39, wherein,
   (i) the heavy chain 1 comprises or consists of an amino acid sequence set forth in SEQ ID NO: 33, and the light chain 1 comprises or consists of an amino acid sequence set forth in SEQ ID NO: 34;
      the heavy chain 2 comprises or consists of an amino acid sequence set forth in SEQ ID NO: 35, and the light chain 2 comprises or consists of an amino acid sequence set forth in SEQ ID NO: 36; or,
   (ii) the heavy chain 1 comprises or consists of an amino acid sequence set forth in SEQ ID NO: 33, and the light chain 1 comprises or consists of an amino acid sequence set forth in SEQ ID NO: 34;
      the heavy chain 2 comprises or consists of an amino acid sequence set forth in SEQ ID NO: 37, and the light chain 2 comprises or consists of the amino acid sequence set forth in SEQ ID NO: 38; or,
   (iii) the heavy chain 1 comprises or consists of an amino acid sequence set forth in SEQ ID NO: 33, and the light chain 1 comprises or consists of an amino acid sequence set forth in SEQ ID NO: 34;
   the heavy chain 2 comprises or consists of an amino acid sequence set forth in SEQ ID NO: 39, and the light chain 2 comprises or consists of the amino acid sequence set forth in SEQ ID NO: 40.
41. The bispecific antibody or the antigen-binding fragment thereof binding to EGFR and B7-H3 according to any one of embodiments 1-40, wherein the antibody or the antigen-binding fragment thereof has one or more of the following properties:
   (i) in one aspect, the antibody can block the binding of an EGFR ligand to EGFR, thereby inhibiting biological signaling and blocking the corresponding biological activity of a tumor; in another aspect, the antibody stimulates the endocytosis of EGFR and thus enables the final degradation of EGFR by intracellular lysosomes and the like;
   (ii) the antibody adopts an EGFR antibody parent sequence with a low affinity for EGFR, thereby greatly reducing toxic and side effects of a series of monoclonal EGFR antibodies on normal epithelial tissues such as the skin;
   (iii) the antibody introduces a parent antibody sequence with a high affinity for B7-H3 on the basis of the low affinity for EGER, thereby greatly improving the blocking activity against EGFR signaling and improving the pharmacodynamic biological activity and the efficacy and safety windows of the bispecific antibody of the present disclosure;
   (iv) the antibody is a low-fucosylated antibody;
   (ii) the antibody has relatively high pharmacodynamic biological activity and safety;
   (v) the antibody has excellent tumor killing and inhibitory effects;
   (vi) the antibody has excellent *in-vivo* and *in-vitro* ADCC pharmacodynamic activity;
   (vii) the antibody has an excellent synergistic anti-tumor effect when used in combination with a KRAS small-molecule inhibitor.
42. An isolated nucleic acid encoding any one of the chains of the bispecific antibody binding to EGFR and B7-H3 according to any one of embodiments 1-41.
43. A vector, which comprises the nucleic acid according to embodiment 42, wherein preferably, the vector is an expression vector; preferably, the expression vector is a pcDNA, e.g., pcDNA3.1.
44. A host cell, which comprises the nucleic acid according to embodiment 42 or the vector according to embodiment 43, wherein preferably, the host cell is prokaryotic or eukaryotic, more preferably a yeast cell or a mammalian cell (e.g., a 293 cell or a CHO cell, e.g., a 293F cell, a 293T cell, or a CHO-S cell).
45. The host cell according to embodiment 44, wherein the host cell is glycoengineered to express an RMD enzyme; preferably, the host cell is a CHO cell.
46. The host cell according to embodiment 45, which comprises a nucleic acid encoding the RMD enzyme.
47. The host cell according to embodiment 46, wherein the RMD enzyme comprises or consists of an amino acid sequence set forth in SEQ ID NO: 41 or an amino acid sequence having at least 90% identity thereto; preferably, the RMD enzyme is derived from *Pseudomonas aeruginosa.*
48. A method for preparing a bispecific antibody binding to EGFR and B7-H3, wherein the method comprises culturing the host cell according to any one of embodiments 44-47 under conditions suitable for expressing the nucleic acid encoding the bispecific antibody according to any one of embodiments 1-41, and optionally, isolating the antibody or the antigen-binding fragment thereof, and optionally, the method further comprises recovering the antibody or the antigen-binding fragment thereof from the host cell (or host cell culture medium).
49. An immunoconjugate, which comprises the bispecific antibody according to any one of embodiments 1-41 conjugated to a therapeutic agent or a diagnostic agent.
50. A pharmaceutical composition, which comprises the bispecific antibody according to any one of embodiments 1-41 or the immunoconjugate according to embodiment 49, and optionally, a pharmaceutical supplementary material.
51. The pharmaceutical composition according to embodiment 50, further comprising a second therapeutic agent, wherein preferably, the second therapeutic agent is selected from an anti-angiogenic agent, a chemotherapeutic agent, an additional antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is selected from a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.
52. A pharmaceutical combination product, which comprises the bispecific antibody according to any one of embodiments 1-41 or the immunoconjugate according to embodiment 49 or the pharmaceutical composition according to embodiment 50, and one or more second therapeutic agents, wherein preferably, the second therapeutic agent is selected from an anti-angiogenic agent, a chemotherapeutic agent, an additional antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is selected from a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.
53. A method for preventing or treating a tumor or an infectious disease in a subject, wherein the method comprises administering to the subject an effective amount of the bispecific antibody according to any one of embodiments 1-41, or the immunoconjugate according to embodiment 49, or the pharmaceutical composition according to embodiment 50.
54. The method according to embodiment 53, further comprising co-administering to the subject one or more additional therapies, wherein the therapy, for example, comprises a therapeutic modality and/or an additional therapeutic agent; preferably, the therapeutic modality comprises surgical treatment and/or radiotherapy, or the therapeutic agent is selected from an anti-angiogenic agent, a chemotherapeutic agent, another antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is selected from a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.
55. A method for preventing or treating a tumor or an infectious disease in a subject, wherein the method comprises administering to the subject an effective amount of the pharmaceutical composition according to embodiment 51 or the pharmaceutical combination product according to embodiment 52.
56. The method according to any of embodiments 53-55, wherein the tumor is a cancer, e.g., a solid tumor or a hematological tumor, including a cancer of epithelial source, e.g., gastrointestinal tumor or lung tumor or skin tumor, e.g., skin cancer (e.g., cutaneous squamous cell carcinoma or head and neck cancer such as head and neck squamous cell carcinoma), esophageal cancer (e.g., esophageal squamous cell carcinoma), intestinal cancer (e.g., colon cancer, rectal cancer, or colorectal cancer), or lung cancer (e.g., non-small cell lung cancer, lung squamous carcinoma, or lung adenocarcinoma).
57. The method according to any one of embodiments 53-56, wherein tumor cells of the tumor
   (i) overexpress wild-type EGFR (e.g., wild-type EGFR with an increased nucleic acid or protein level) and/or express mutant EGFR compared with normal cells of an adjacent tissue or compared with normal cells of the same tissue in a healthy subject, wherein preferably, the mutant EGFR comprises one or more mutations selected from R521K, L858R, T790M, G719X, C797S, Y1069C, an Exon19 deletion (Del19), and Exon20ins (e.g., S768_D770dup), and preferably, the mutant EGFR comprises R521K/Y1069C, R521K, L858R/T790M/C797S, Del19/T790M/C797S, or S768_D770dup;
   (ii) overexpress wild-type KRAS (e.g., wild-type KRAS with an increased nucleic acid or protein level) or express mutant KRAS compared with normal cells of an adjacent tissue or compared with normal cells of the same tissue in a healthy subject, wherein preferably, the mutant KRAS comprises a mutation at position G12 or G13, e.g., G12D or G12C;
   (iii) have B7-H3 with an increased nucleic acid or protein level compared with normal cells of an adjacent tissue or compared with normal cells of the same tissue in a healthy subject; and/or
   (iv) the tumor cells are resistant to a tyrosine kinase inhibitor, e.g., to the first-generation (erlotinib) and the third-generation (osimertinib), e.g., to osimertinib.
58. The method according to embodiment 57, wherein the tumor cells of the tumor express the mutant EGFR and the mutant KRAS, e.g., comprise EGFR having a mutation R521K and KRAS having a mutation G120D.
59. A method for detecting antigens EGFR and/or B7-H3 in a sample, wherein the method comprises
   (a) bring a sample into contact with the bispecific antibody according to any one of embodiments 1-41; and
   (b) detecting a complex formed by the antibody or the antigen-binding fragment thereof with EGFR and/or B7-H3, wherein optionally, the antibody is detectably labeled.
60. Use of the antibody or the antigen-binding fragment thereof according to any one of embodiments 1-41, and/or the isolated nucleic acid according to embodiment 42, and/or the vector according to embodiment 43, and/or the host cell according to any one of embodiments 44-47, and/or the immunoconjugate according to embodiment 49, and/or the pharmaceutical composition according to embodiment 50 or 51 or the pharmaceutical combination product according to embodiment 52 in the preparation of a drug for preventing and/or treating a disease in a subject.

The present disclosure also encompasses any combination of the embodiments described herein. Any of the embodiments described herein or any combination thereof is applicable to any and all of the antibodies or the fragments thereof, the immunoconjugate, the pharmaceutical composition, the combination product, the kit, the method, and the use described herein.

### BRIEF DESCRIPTION OF THE DRAWINGS

The preferred embodiments of the present disclosure described in detail below will be better understood when read in conjunction with the following drawings. For the purpose of illustrating the present disclosure, currently, the preferred embodiments are shown in the drawings. However, it should be understood that the present disclosure is not limited to the embodiments shown in the drawings.
FIG. 1 is a structural schematic diagram of the anti-B7-H3/EGFR bispecific antibodies of the present disclosure.
FIG. 2 shows the screening proliferation inhibition assay of the anti-B7-H3/EGFR bispecific antibodies of the present disclosure.
FIG. 3 shows the screening ADCC assay of the anti-B7-H3/EGFR bispecific antibodies of the present disclosure.
FIG. 4 shows the expression detection of B7H3 and EGFR on different cell lines for Hz20G5.26/Zalu bsAb of the present disclosure.
FIG. 5 shows the results for the proliferation inhibition assay of Hz20G5.26/Zalu bsAb of the present disclosure in CRC tumor cell lines CCK-81, NCI-H508, HT-55, and LS180, with the antibodies diluted as follows:
   CCK-81, NCI-H508, and HT-55: among the final concentrations after the dilution of the antibodies, the highest is 125 nM, with a 4-fold dilution;
   LS180: among the final concentrations after the dilution of the antibodies, the highest is 300 nM, with a 4-fold dilution.
FIG. 6 shows the results for the proliferation inhibition assay of Hz20G5.26/Zalu bsAb of the present disclosure in HNSCC tumor cell lines TE-1 and Colo680, with the antibodies diluted as follows: TE-1 and Colo680: among the final concentrations after the dilution of the antibodies, the highest is 300 nM, with a 3.16-fold dilution.
FIG. 7.1 shows the results for the proliferation inhibition assay of Hz20G5.26/Zalu bsAb of the present disclosure in different NSCLC-EGFR^{WT} tumor cell lines.
FIG. 7.2 shows the results for the proliferation inhibition assay of Hz20G5.26/Zalu bsAb of the present disclosure in NSCLC-EGFR classic mutation tumor cell lines.
FIG. 7.3 shows the results for the proliferation inhibition assay of Hz20G5.26/Zalu bsAb of the present disclosure in NSCLC-EGFR abnormally amplified tumor cell lines.
FIG. 7.4 shows the results for the proliferation inhibition experimental of Hz20G5.26/Zalu bsAb of the present disclosure in rare NSCLC-EGFR mutant tumor cell lines.
FIG. 7.5 shows the results for the comparative experiment on the efficacy of Hz20G5.26/Zalu bsAb of the present disclosure in combination with gp120/Zalu and gp120/hz20G5.26,
with the antibodies or the small molecule TKI (osimertinib or erlotinib) diluted as follows:

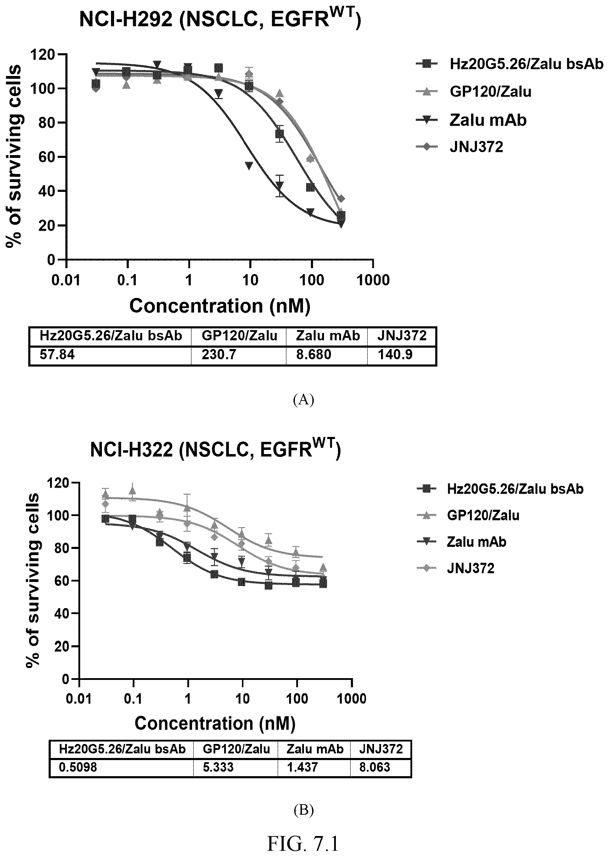

FIG. 7.1: NCI-292 and NCI-H322: among the final concentrations after the dilution of the antibodies, the highest is 300 nM, with a 3.16-fold dilution;

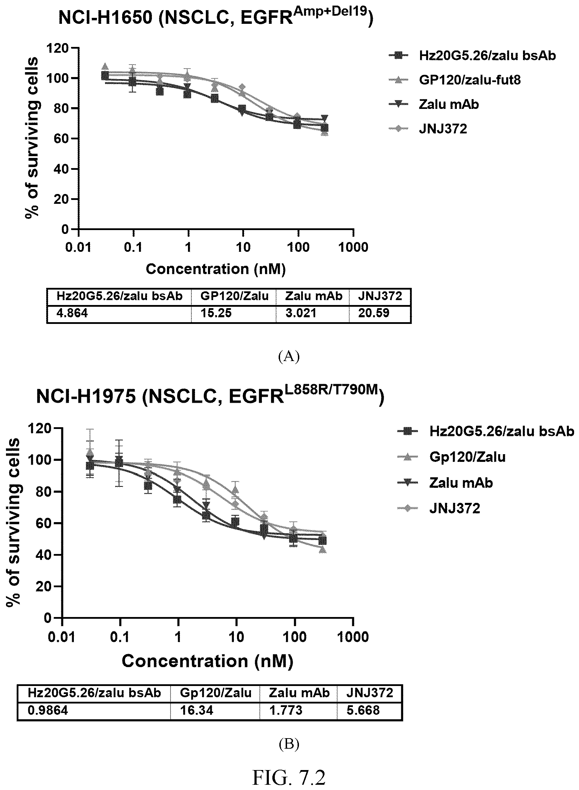

FIG. 7.2: NCI-H1650 and NCI-H1975: among the final concentrations after the dilution of the antibodies, the highest is 300 nM, with a 3.16-fold dilution;

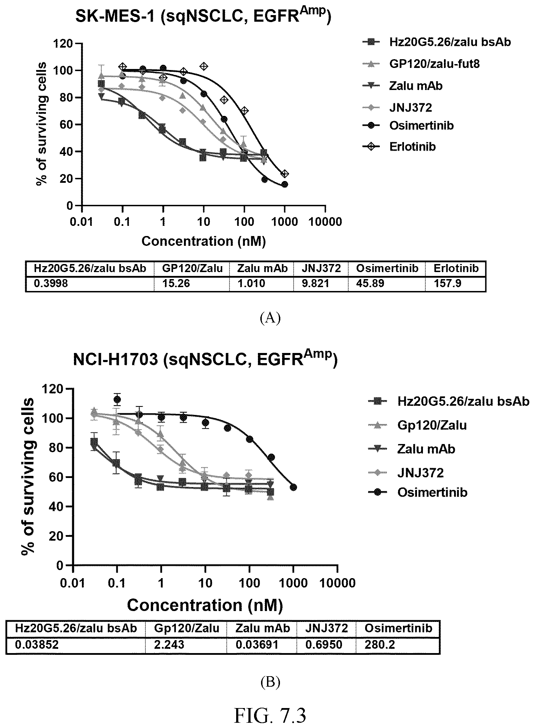

FIG. 7.3: NCI-H1703 and SK-MES-1: among the final concentrations after the dilution of the antibody molecules, the highest is 300 nM, with a 3.16-fold dilution; among the final concentrations after the dilution of small molecule TKIs (osimertinib and erlotinib), the highest is 1000 nM, with a 3.16-fold dilution;

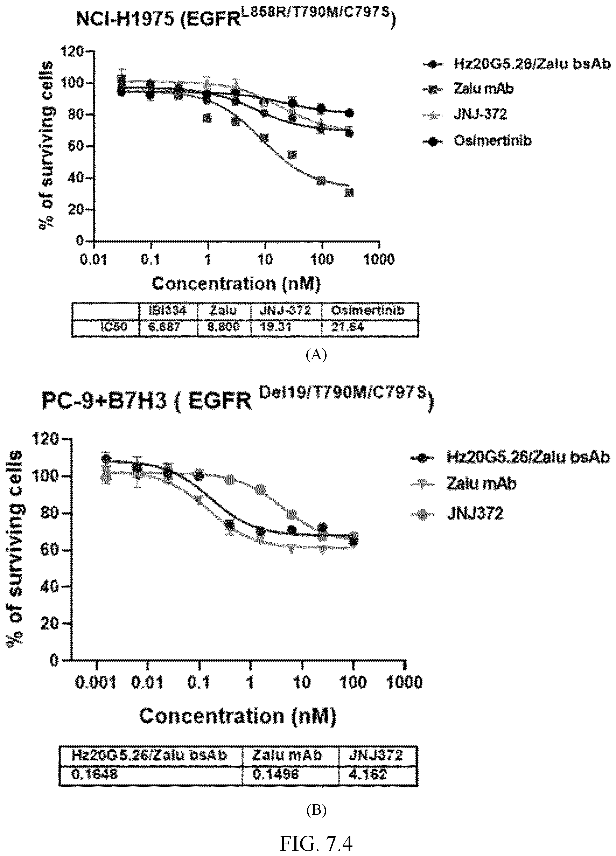

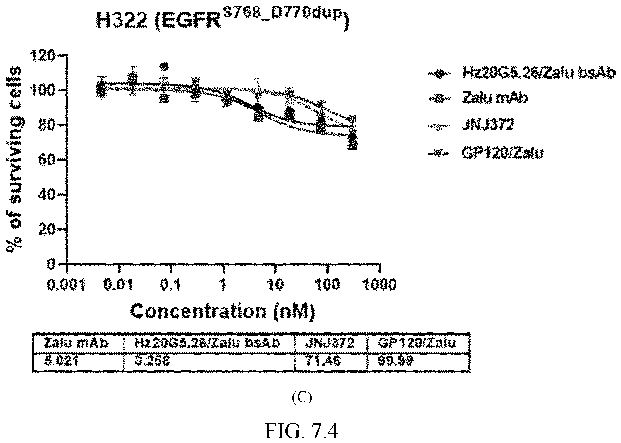

FIG. 7.4: H1975 (EGFR^{L858R/T790M/C797S}): among the final concentrations after the dilution of the antibody molecules and osimertinib, the highest is 300 nM, with a 3.16-fold dilution; H322 (EGFR^{S768-D77dup}): among the final concentrations after the dilution of the antibody molecules and osimertinib, the highest is 300 nM, with a 4-fold dilution; PC9 + B7H3 (EGFR^{Del19/T790M/C79S}): among the final concentrations after the dilution of the antibody molecules and osimertinib, the highest is 100 nM, with a 4-fold dilution;

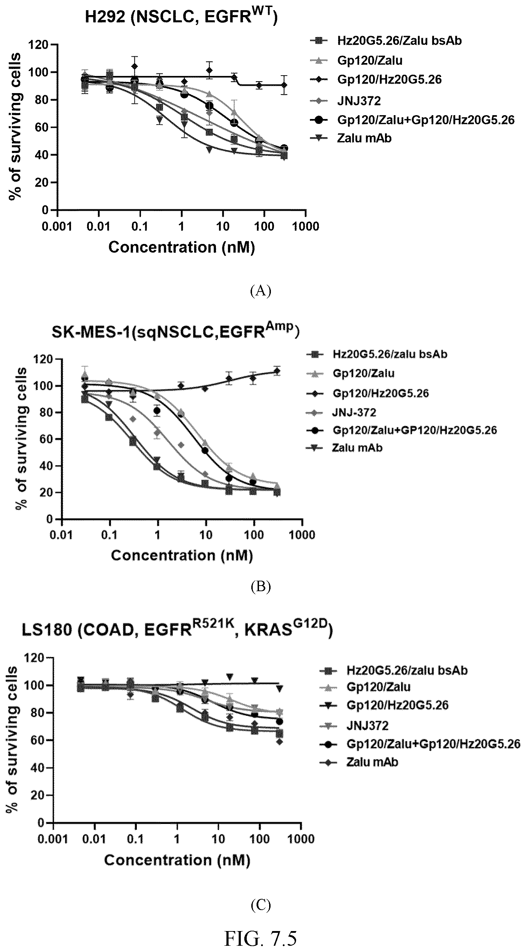

FIG. 7.5: LS180 and H292: among the final concentrations after the dilution of the antibodies, the highest is 300 nM, with a 4-fold dilution; SK-MES-1: among the final concentrations, the highest is 300 nM, with a 3.16-fold dilution.

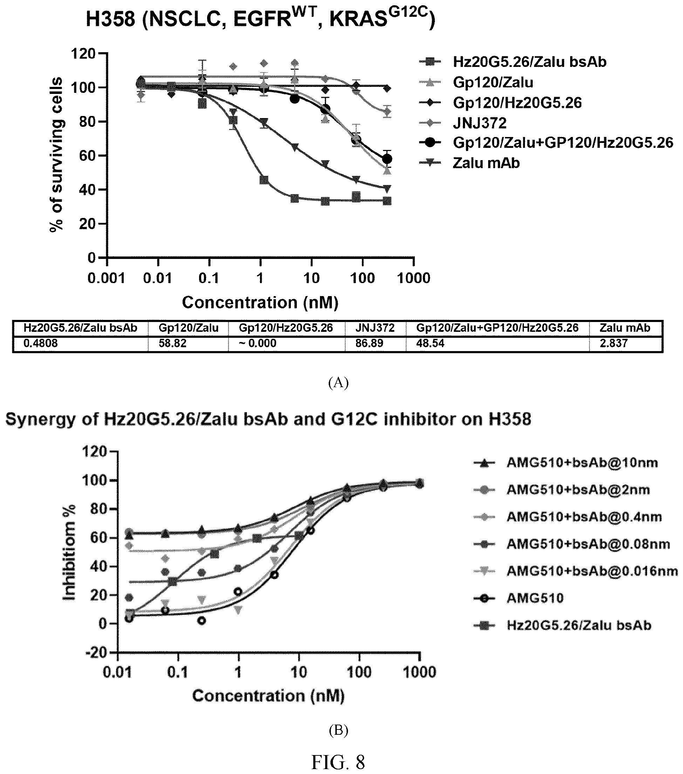

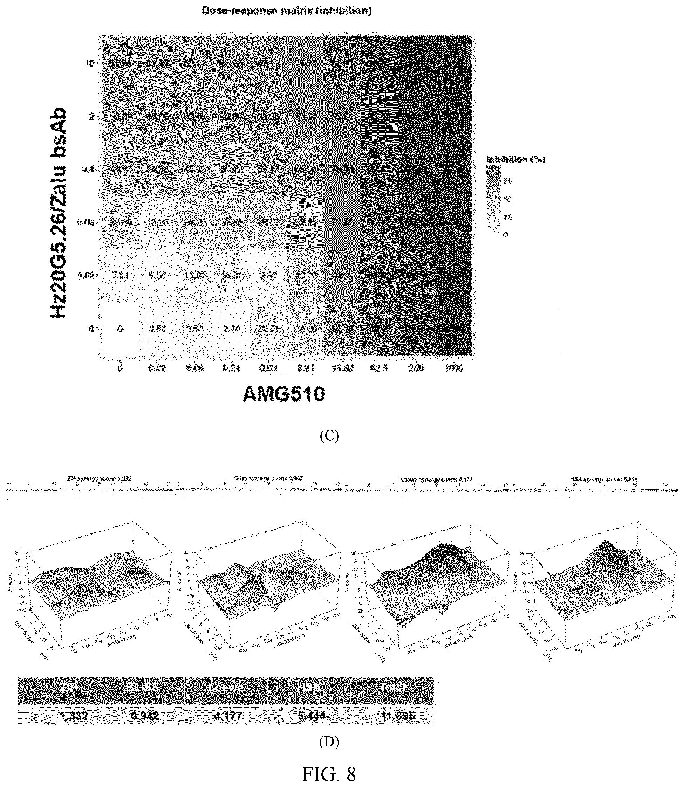

FIG. 8 shows the results for the comparative experiment on the synergistic administration of Hz20G5.26/Zalu bsAb of the present disclosure in combination with the AMG510 small molecule inhibitor in H358 NSCLC cell lines, in which
FIG. 8(A): *in-vitro* proliferation inhibition assay in H358: among the final concentrations of the antibody molecules, the highest is 300 nM, with a 4-fold dilution;
FIG. 8(B): the experiment on the administration of Hz20G5.26/Zalu bsAb in combination with the KRAS G12C inhibitor AMG510 (MedChem Express, HY-114277): AMG510+bsAb@10nm indicates that Hz20G5.26/Zalu bsAb at a fixed concentration of 10 nM is added to each well, and AMG510 gradiently diluted (final concentration: 1000 nM, 4-fold dilution) is added to each well; AMG510-only: among the final concentrations, the highest final concentration is 1000 nM, with a 4-fold dilution; Hz20G5.26/Zalu bsAb-only: among the final concentrations, the highest is 10 nM, with a 5-fold dilution; a total of 5 concentration gradients are obtained.

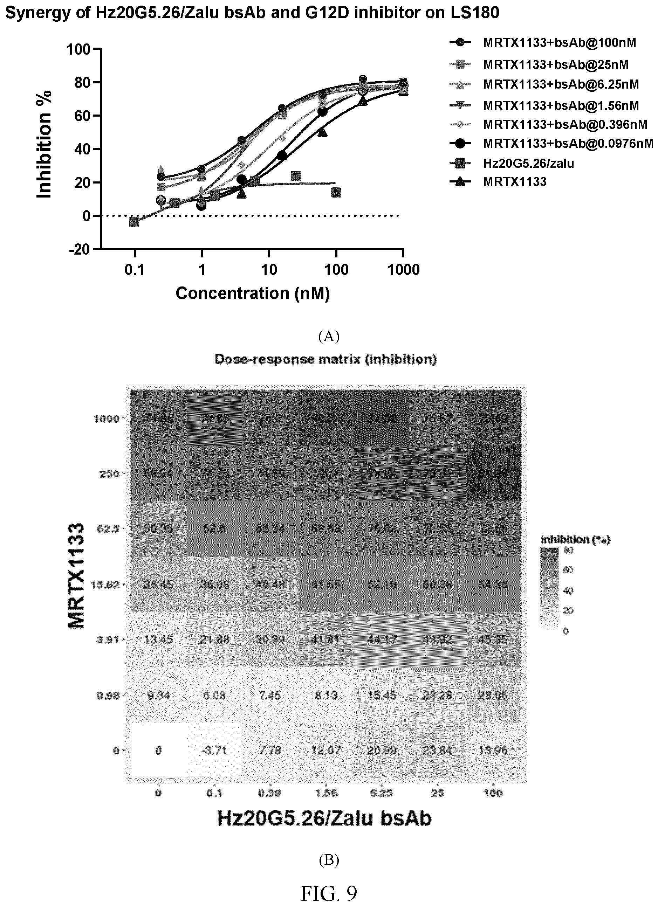

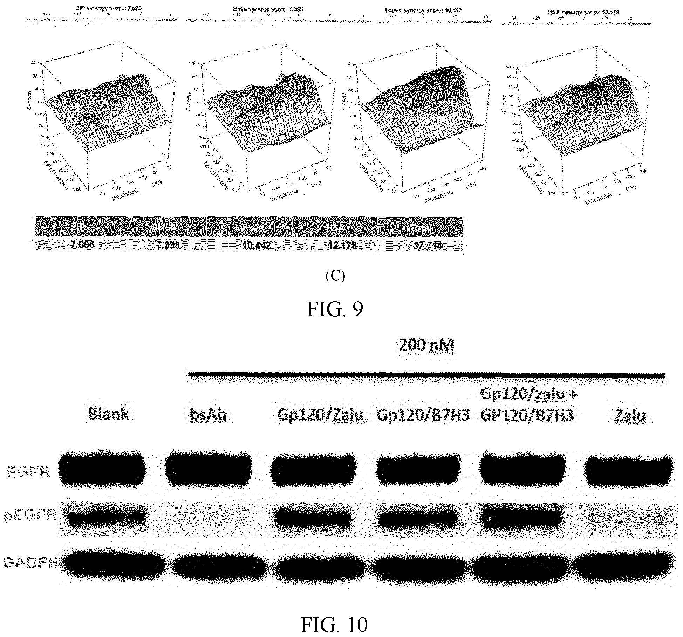

FIG. 9 shows the results for the comparative experiment on the synergistic administration of Hz20G5.26/Zalu bsAb of the present disclosure in combination with the KRAS G12D small molecule inhibitor MRTX1133 in LS180 CRC cell lines, in which
FIG. 9(A): the experiment on the administration of Hz20G5.26/Zalu bsAb in combination with the G12D inhibitor MRTX1133 (MCE, cat: HY-134813A-10mg): MRTX1133+bsAb@100nm indicates that Hz20G5.26/Zalu bsAb at a fixed concentration of 100 nM is added to each well, and MRTX1133 gradiently diluted (final concentration: 1000 nM, 4-fold dilution) is added to each well; MRTX1133-only: among the final concentrations, the highest is 1000 nM, with a 4-fold dilution; Hz20G5.26/Zalu bsAb-only: among the final concentrations, the highest is 100 nM, with a 4-fold dilution; a total of 6 concentration gradients are obtained.
FIG. 10 shows the experimental results for the blocking effect of Hz20G5.26/Zalu bsAb of the present disclosure on EGFR phosphorylation signals in the H358 cell line.

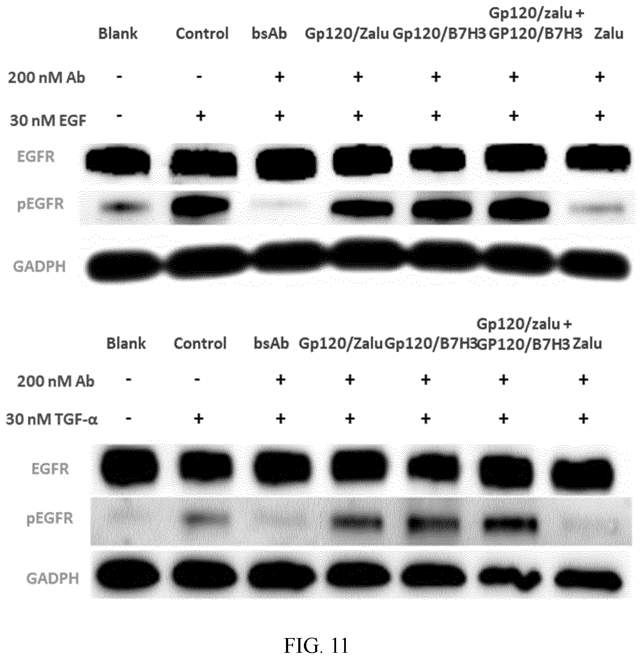

FIG. 11 shows a graph of the experimental results of the blocking effect of Hz20G5.26/Zalu bsAb of the present disclosure on EGFR phosphorylation signals induced by the EGFR ligand (EGF or TGF-α) in the H358 cell line.

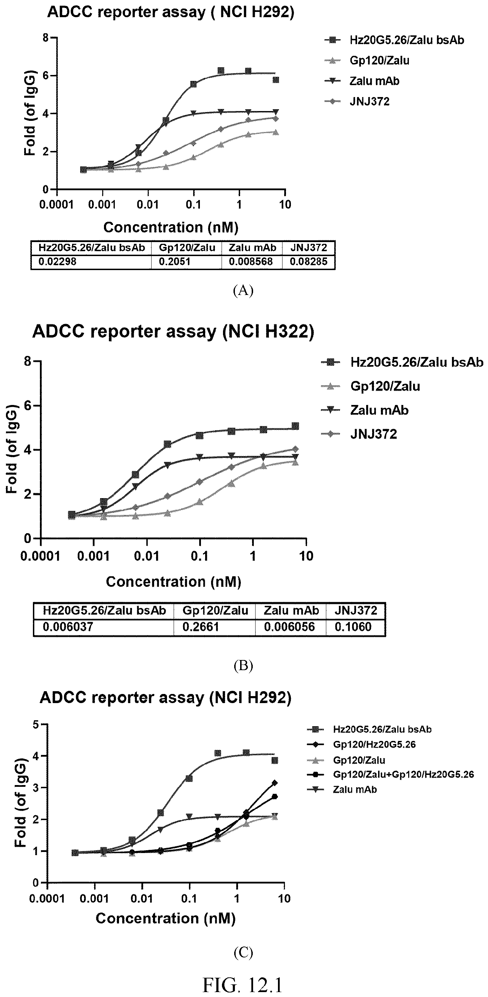

FIG. 12.1 shows the results for the ADCC reporter assay of Hz20G5.26/Zalu bsAb of the present disclosure in the NSCLC-EGFR^{WT} tumor cell lines, in which in 3 cell lines, among the final concentrations after the dilution of the antibody molecules, the highest is 6.25 nM, with a 4-fold dilution.

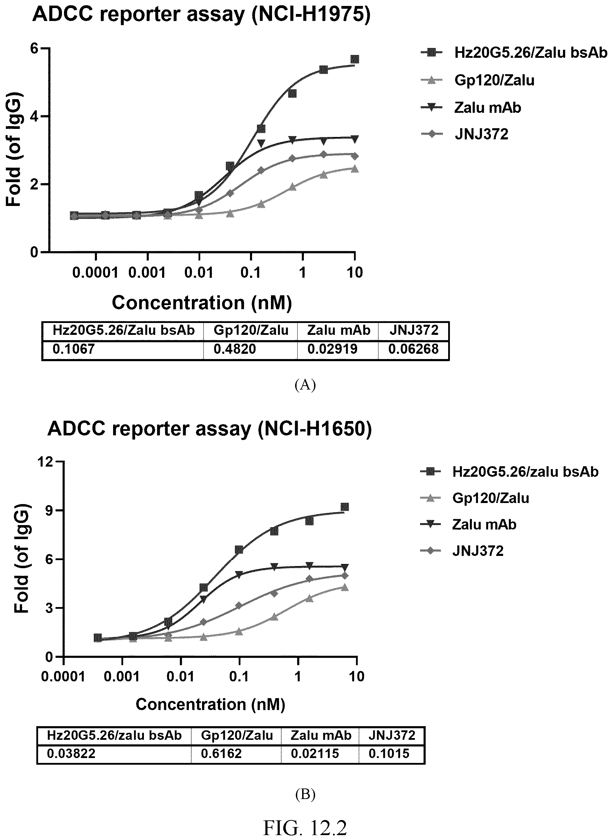

FIG. 12.2 shows the results for the ADCC reporter assay of Hz20G5.26/Zalu bsAb of the present disclosure in the NSCLC-EGFR mutant tumor cell lines, in which in the H1975 cell line, among the concentrations after the dilution of the antibody molecules, the highest is 10 nM, with a 4-fold dilution; and in the H1975 cell line, among the concentrations after the dilution of the antibody molecules, the highest is 6.25 nM, with a 4-fold dilution.

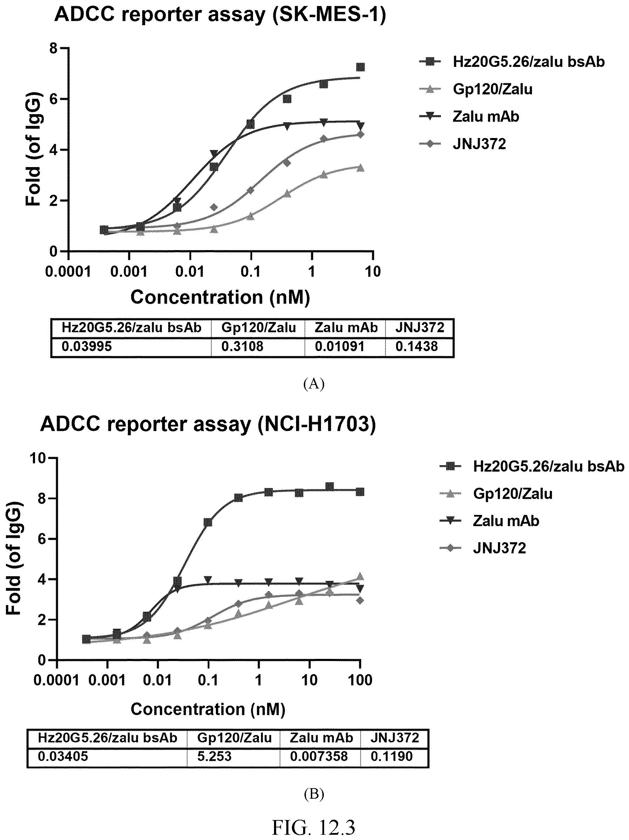

FIG. 12.3 shows the results for the ADCC reporter assay of Hz20G5.26/Zalu bsAb of the present disclosure in NSCLC-EGFR abnormally amplified tumor cell lines, in which in the SK-MES-1 cell line, among the concentrations after the dilution of the antibody molecules, the highest is 6.25 nM, with a 4-fold dilution; and in the H1703 cell line, among the concentrations after the dilution of the antibody molecules, the highest is 100 nM, with a 4-fold dilution.

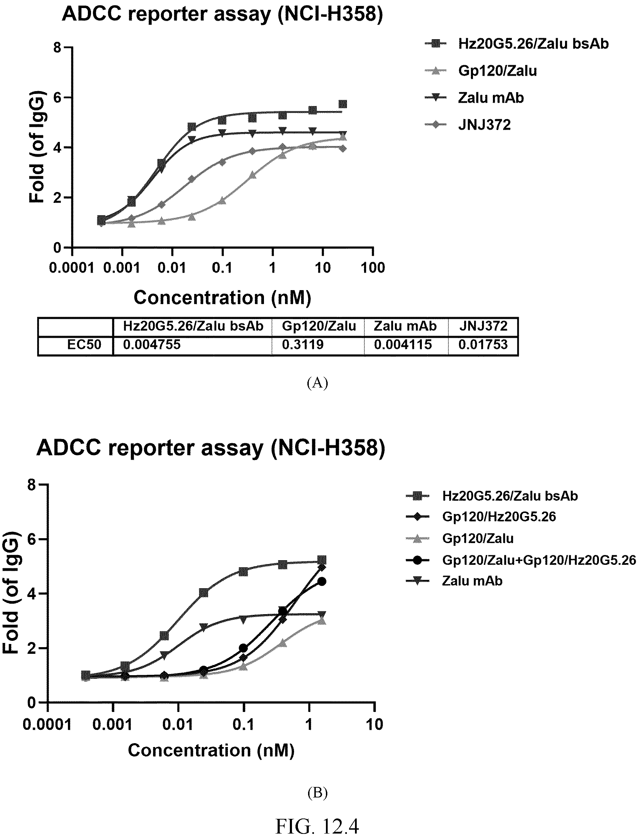

FIG. 12.4 shows the results for the ADCC reporter assay of Hz20G5.26/Zalu bsAb of the present disclosure in NSCLC-KRAS mutant tumor cell lines, wherein in the H358 cell line, among the concentrations after the dilution of the antibody molecules, the highest is 25 nM, with a 4-fold dilution (FIG. 12.4(A)); and in the H358 cell line, among the concentrations after the dilution of the antibody molecules, the highest is 1.56 nM, with a 4-fold dilution (FIG. 12.4(B)).

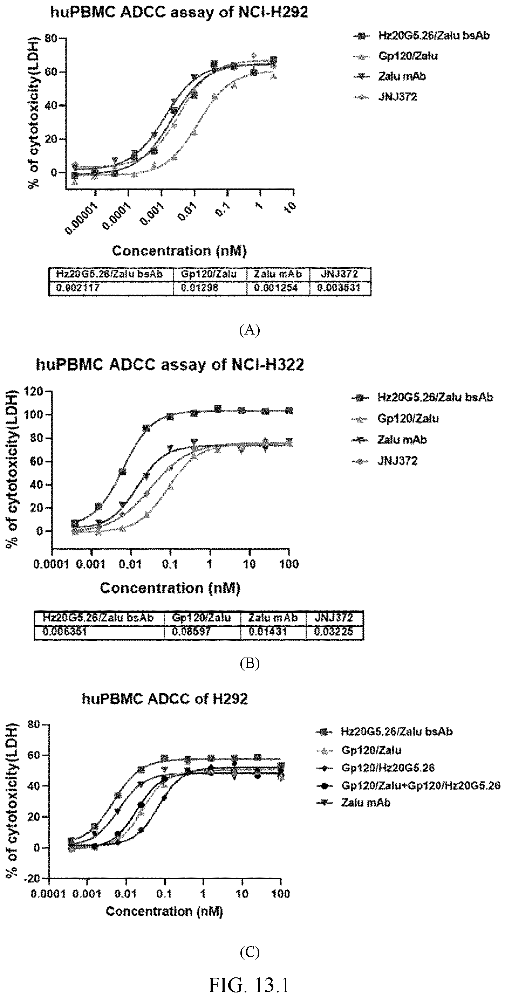

FIG. 13.1 shows the results for the huPBMC ADCC reporter assay of Hz20G5.26/Zalu bsAb of the present disclosure in NSCLC-EGFR wild-type tumor cell lines, in which
FIG. 13.1(A): in the H292 cell line, among the final concentrations after the dilution of the antibody molecules, the highest is 2.5 nM, with a 4-fold dilution;
FIG. 13.1(B): in the H322 cell line, among the final concentrations after the dilution of the antibody molecules, the highest is 100 nM, with a 4-fold dilution;
FIG. 13.1(C): in the H292 cell line, among the final concentrations after the dilution of the antibody molecules, the highest is 100 nM, with a 4-fold dilution;

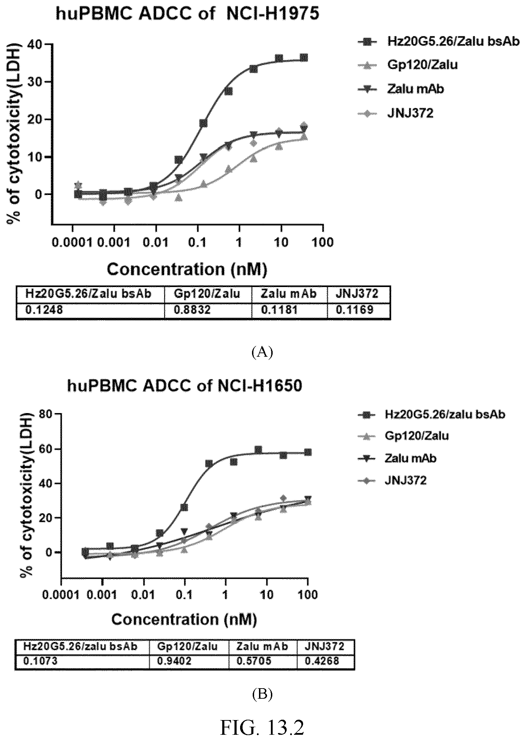

FIG. 13.2 shows the results for the huPBMC ADCC reporter assay of Hz20G5.26/Zalu bsAb of the present disclosure in the NSCLC-EGFR mutant tumor cell lines, in which in the H1975 cell line, among the concentrations after the dilution of the antibody molecules, the highest is 35 nM, with a 4-fold dilution; in the H1650 cell line, among the concentrations after the dilution of the antibody molecules, the highest is 100 nM, with a 4-fold dilution.

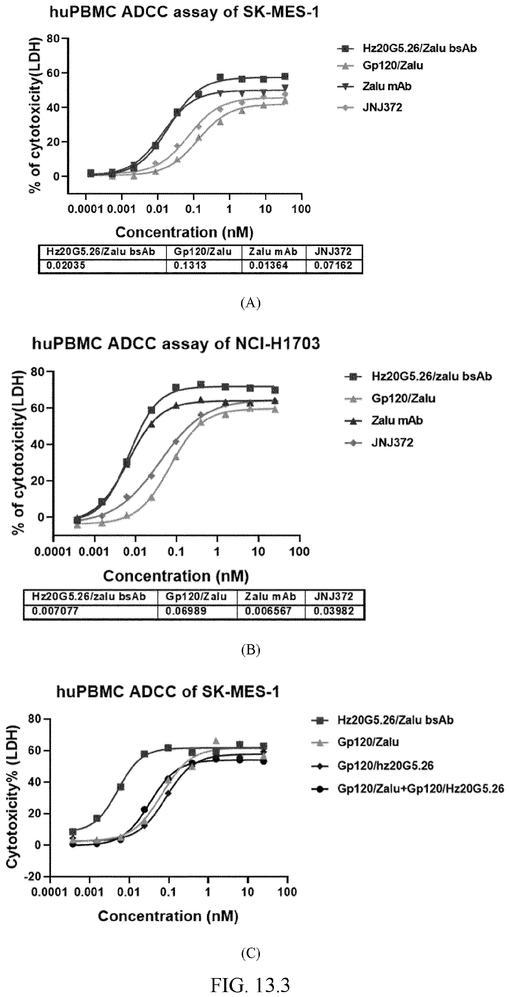

FIG. 13.3 shows the results for the huPBMC ADCC reporter assay of Hz20G5.26/Zalu bsAb of the present disclosure in NSCLC-EGFR abnormally amplified tumor cell lines, in which
FIG. 13.3(A): in the SK-MES-1 cell line, among the final concentrations after the dilution of the antibody molecules, the highest is 35 nM, with a 4-fold dilution;
FIG. 13.3(B): in the H1703 cell line, among the final concentrations after the dilution of the antibody molecules, the highest is 25 nM, with a 4-fold dilution;
FIG. 13.3(C): in the SK-MES-1 cell line, among the final concentrations after the dilution of the antibody molecules, the highest is 25 nM, with a 4-fold dilution;

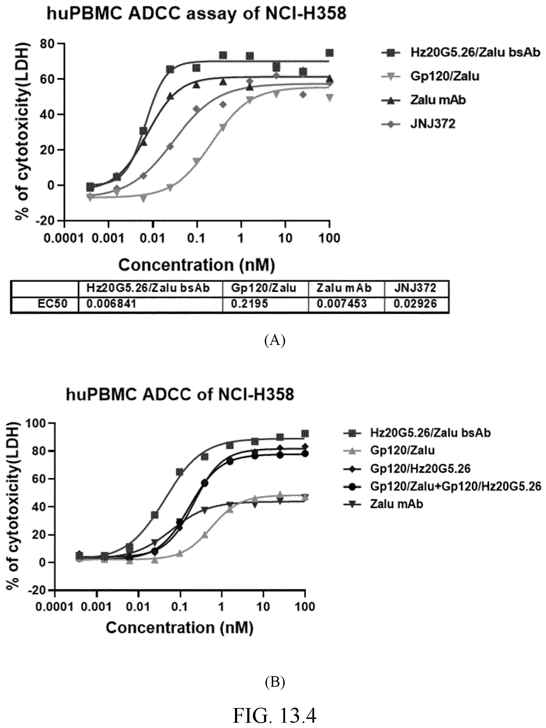

FIG. 13.4 shows the results for the huPBMC ADCC reporter assay of Hz20G5.26/Zalu bsAb of the present disclosure in NSCLC-KRAS mutant tumor cell lines, wherein in each of the two cell lines, among the final concentrations after the dilution of the antibody molecules, the highest is 35 nM, with a 4-fold dilution.

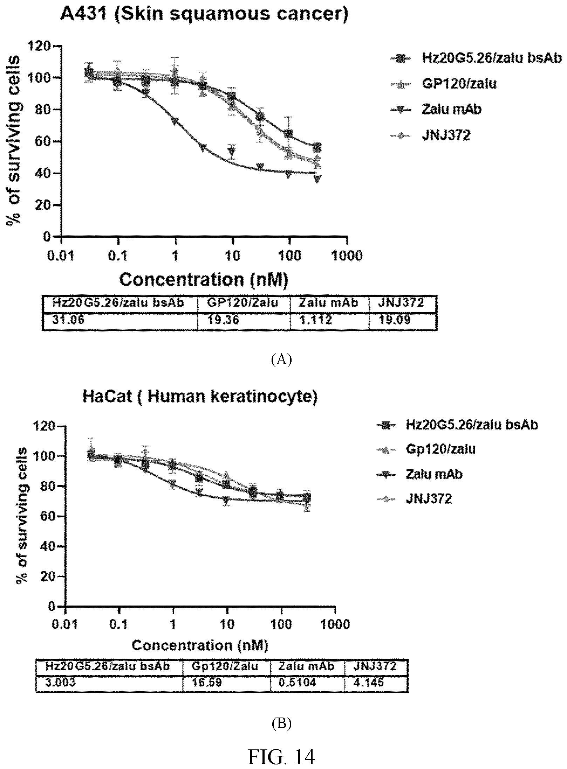

FIG. 14 shows the experimental results for the *in-vitro* study of simulated skin toxicity of the Hz20G5.26/Zalu bsAb drug of the present disclosure, in which the antibody molecules are diluted with a cell culture medium, and in each of the two human skin cell lines, among the final concentrations after the dilution of the antibody molecules, the highest is 300 nM, with a 3.16-fold dilution.

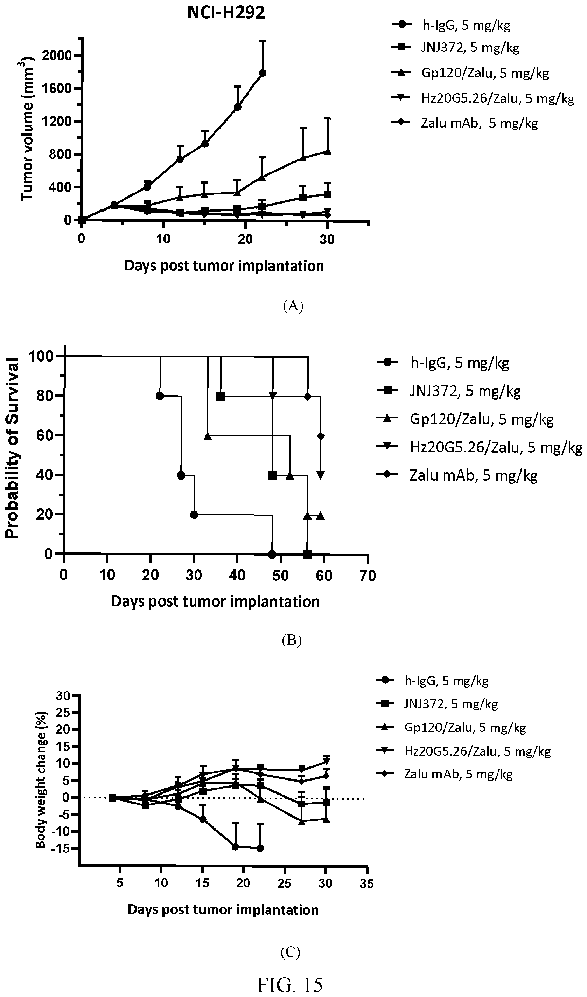

FIG. 15A shows the experimental results for the anti-tumor effect of Hz20G5.26/Zalu bsAb of the present disclosure on the NCl-H292 tumor-bearing mice; FIG. 15B shows the experimental results for the survival curve versus the anti-tumor effect of Hz20G5.26/Zalu bsAb of the present disclosure on the NCl-H292 tumor-bearing mice; FIG. 15C shows the experimental results for the effect of Hz20G5.26/Zalu bsAb of the present disclosure on the body weight of the NCl-H292 tumor-bearing mice.

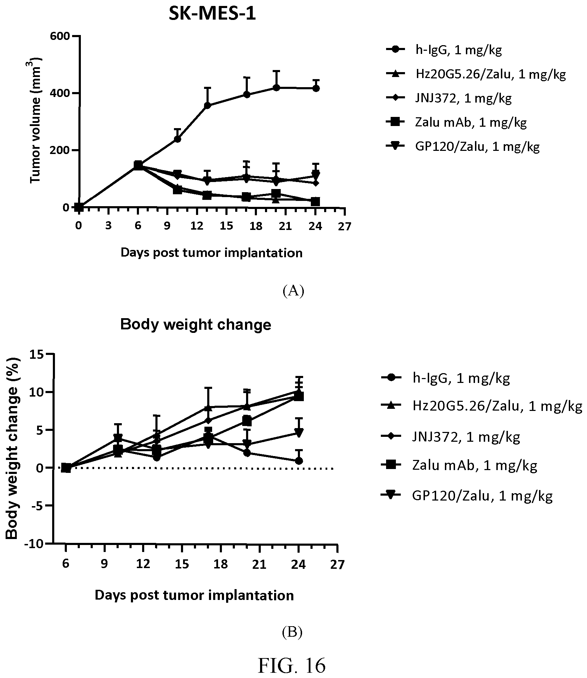

FIG. 16A shows the experimental results for the anti-tumor effect of Hz20G5.26/Zalu bsAb of the present disclosure on the SK-MES-1 tumor-bearing mice; FIG. 16B shows the experimental results for the effect of Hz20G5.26/Zalu bsAb of the present disclosure on the body weight of the SK-MES-1 tumor-bearing mice.

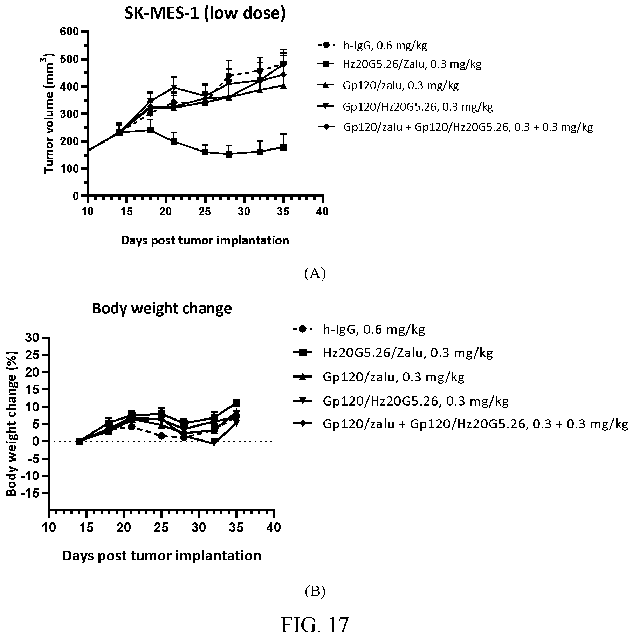

FIG. 17A shows the experimental results for the anti-tumor effect of Hz20G5.26/Zalu bsAb of the present disclosure on the SK-MES-1 tumor-bearing mice; FIG. 17B shows the experimental results for the effect of Hz20G5.26/Zalu bsAb of the present disclosure on the body weight of the SK-MES-1 tumor-bearing mice.

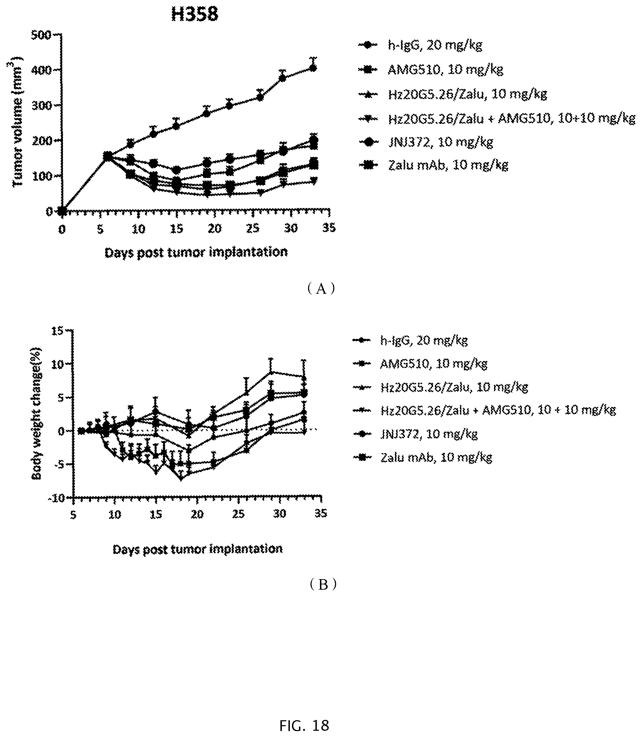

FIG. 18A shows the experimental results for the anti-tumor effect of Hz20G5.26/Zalu bsAb of the present disclosure in combination with AMG510 on the NCI-H358 tumor-bearing mice; FIG. 18B shows the experimental results for the effect of Hz20G5.26/Zalu bsAb of the present disclosure in combination with AMG510 on the body weight of the NCI-H358 tumor-bearing mice.

### DETAILED DESCRIPTION

Unless otherwise defined, all technical and scientific terms used herein have the same meaning as commonly understood by those of ordinary skill in the art to which the present disclosure belongs. All publications, patent applications, patents, and other references mentioned herein are incorporated by reference in their entireties. In addition, the materials, methods, and examples described herein are illustrative only and are not intended to be limiting. Other features, objectives, and advantages of the present disclosure will be apparent from the specification and drawings, and from the appended claims.

### I. Definitions

It should be understood that the present disclosure is not limited to the particular methodology, protocols, and reagents described herein, as these may vary. It should also be understood that the terms used herein are only intended to describe specific embodiments rather than limit the scope of the present disclosure, which will be limited only by the appended claims. Unless otherwise defined, any technical and scientific term used herein has the same meaning as commonly understood by those of ordinary skill in the art to which the present disclosure belongs.

For the purpose of explaining this specification, the following definitions will be used, and wherever appropriate, terms used in the singular form may also include the plural form, and vice versa. It should be understood that the terms used herein are for the purpose of describing specific embodiments only and are not intended to be limiting.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or any two or more of the options.

As used herein, the term "comprise" or "include" is intended to mean that the described elements, integers, or steps are included, but not to the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

When referring to "first" and "second" herein, it is only to distinguish two domains or two chains, and does not indicate the location of the two domains in any way.

As used herein, the amino acid positions of all variable regions of the heavy and light chains are numbered according to the Kabat numbering system described in Kabat et al., Sequences of Proteins of Immunological Interest, 5th Ed., Public Health Service, National Institutes of Health, Bethesda, MD (1991) and referred to herein as "Kabat numbering".

As used herein, when used to refer to an amino acid position in a domain of an antibody other than a variable region (e.g., a constant region, such as an Fc region), it is numbered according to the EU numbering system described in Kabat, E. A. et al., Sequences of Proteins of Immunological Interest, 5th Ed., Public Health Service, National Institutes of Health, Bethesda, MD (1991) and referred to herein as "EU numbering". When a position number and/or an amino acid residue is assigned to a particular antibody isotype, it is intended to apply to the corresponding position and/or amino acid residue of any other antibody isotype, which is known to those skilled in the art.

General information on the nucleotide sequences of the human immunoglobulin light and heavy chains is given in Kabat, E. A. et al., Sequences of Proteins of Immunological Interest, 5^{th} Ed., Public Health Service, National Institutes of Health, Bethesda, MD (1991).

The term "antibody" is used herein in the broadest sense, refers to a protein comprising an antigen-binding site, and encompasses natural and artificial antibodies with various structures, including, but not limited to, monoclonal antibodies, polyclonal antibodies, multispecific antibodies (e.g., bispecific antibodies), single-chain antibodies, intact antibodies, and antibody fragments.

The terms "whole antibody", "full-length antibody", "complete antibody" and "intact antibody" are used interchangeably herein to refer to a naturally occurring glycoprotein comprising at least two heavy (H) chains and two light (L) chains interconnected by disulfide bonds. Each of the heavy chains consists of a heavy chain variable region (abbreviated herein as VH) and a heavy chain constant region. The heavy chain constant region consists of 3 domains CH1, CH2, and CH3. Each of the light chains consists of a light chain variable region (abbreviated herein as VL) and a light chain constant region. The light chain constant region consists of one domain CL. The VH region and the VL region can be further divided into hypervariable regions (complementarity-determining regions, or CDRs), with relatively conservative regions (framework regions, or FRs) inserted therebetween. Each VH or VL consists of three CDRs and 4 FRs, arranged from amino-terminus to carboxyl-terminus in the following order: FR1, CDR1, FR2, CDR2, FR3, CDR3, and FR4. The constant regions are not directly involved in the binding of antibodies to antigens, but exhibit a variety of effector functions.

The term "half antibody" or "hemimer" refers to a monovalent antigen-binding polypeptide. In some embodiments, the half antibody or hemimer comprises a VH/VL unit and optionally at least a portion of an immunoglobulin constant domain. In some embodiments, the half antibody or hemimer comprises one immunoglobulin heavy chain associated with one immunoglobulin light chain, or an antigen-binding fragment thereof. In some embodiments, the half antibody or hemimer is monospecific; that is, it binds to a single antigen or epitope. In some specific embodiments, the half antibody binds to EGFR and does not bind to B7-H3. In some specific embodiments, the half antibody binds to B7-H3 and does not bind to EGFR. Those skilled in the art will readily understand that a half antibody may have an antigen-binding domain consisting of a single variable domain (e.g., derived from camelidae).

The term "antigen-binding fragment" of an antibody is a molecule different from a full-length antibody, which comprises a portion of the full-length antibody, but is capable of binding to an antigen of the full-length antibody or competes with the full-length antibody (i.e., a full-length antibody from which the antigen-binding fragment is derived) for binding to an antigen. The antigen-binding fragment may be prepared by recombinant DNA techniques, or by enzymatic or chemical cleavage of an intact antibody. The antigen-binding fragment includes, but is not limited to, a Fab, a Fab', a F(ab')2, an Fv, a single-chain Fv, a diabody, a single-domain antibody (sdAb), and a nanobody.

The "Fab fragment" and "Fab" are used interchangeably herein to refer to an immunoglobulin fragment consisting of two polypeptide chains and comprising a heavy chain variable region VH, a heavy chain constant domain CH1, a light chain variable region VL, and a light chain constant domain CL of an immunoglobulin, in which one polypeptide chain comprises, from N-terminus to C-terminus, a VH and one constant region selected from a CH1 and a CL, and the other polypeptide chain comprises, from N-terminus to C-terminus, a VL and the other constant region selected from a CL and a CH1, wherein the VH and VL domains pair to form an antigen-binding site. A Fab' fragment differs from the Fab fragment due to the addition of some residues (including one or more cysteines from an antibody hinge region) to the carboxyl-terminus of the heavy chain CH1 domain. A Fab'-SH refers to a Fab' in which the cysteine residue of the constant domain carries a free thiol group. A F(ab')₂ antibody fragment was originally generated as paired Fab' fragments with hinge cysteines between the Fab' fragments. Other chemical couplings of antibody fragments are also known.

As used herein, the term "bispecific antibody" comprises antigen-binding domains that specifically bind to two kinds of antigens or two kinds of epitopes. Unless otherwise stated, the order of antigens bound to the bispecific antibody in the listed name of the bispecific antibody is arbitrary. That is, in some embodiments, the terms "anti-EGFR/B7-H3 bispecific antibody" and "anti-B7-H3/EGFR bispecific antibody" are used interchangeably. In some embodiments, the bispecific antibody comprises two half antibodies, each of which comprises a single heavy chain variable region and optionally at least a portion of a heavy chain constant region, and comprises a single light chain variable region and optionally at least a portion of a light chain constant region. In some embodiments, the bispecific antibody comprises two half antibodies, each of which comprises a single heavy chain variable region and a single light chain variable region but does not comprise more than one single heavy chain variable region and does not comprise more than one single light chain variable region. In some embodiments, the bispecific antibody comprises two half antibodies, each of which comprises a single heavy chain variable region and a single light chain variable region, in which a first half antibody binds to a first antigen/epitope but does not bind to a second antigen/epitope, and a second half antibody binds to the second antigen but does not bind to the first antigen.

The multispecific antibody of the present disclosure may comprise a linker. The term "linker" as used herein refers to any molecule that enables direct connection of different portions of a multispecific antibody. Examples of linkers to establish covalent linkages between different portions of a multispecific antibody include peptide linkers and non-proteinaceous polymers including, but not limited to, polyethylene glycol (PEG), polypropylene glycol, polyalkylene oxide, and copolymers of polyethylene glycol and polypropylene glycol. In some embodiments, the term "peptide linker" according to the present disclosure refers to an amino acid sequence, wherein the sequence links the amino acid sequences of various portions of a multispecific antibody together. Preferably, the peptide linker has a length sufficient to link two entities in a manner that maintains their conformation relative to each other without interference with the desired activities. The peptide linker may or may not predominantly comprise the following amino acid residues: Gly, Ser, Ala, or Thr. Useful linkers include glycine-serine polymers including, for example, (GS)n, (GSGGS)n, (GGGGS)n, (GGGS)n, and (GGGGS)nG, wherein n is an integer of at least 1 (and preferably 2, 3, 4, 5, 6, 7, 8, 9, or 10). Useful linkers also include glycine-alanine polymers, alanine-serine polymers, and other flexible linkers.

The term "Fc domain" or "Fc region" is used herein to define a C-terminus region of an immunoglobulin heavy chain, which comprises at least a portion of a constant region. The term includes Fc regions of natural sequences and variant Fc regions. A natural immunoglobulin "Fc domain" comprises two or three constant domains, i.e., a CH2 domain, a CH3 domain, and an optional CH4 domain. For example, in natural antibodies, an immunoglobulin Fc domain comprises the second and the third constant domains (CH2 domain and CH3 domain) derived from two heavy chains of IgG, IgA, and IgD antibodies, or comprises the second, the third, and the fourth constant domains (CH2 domain, CH3 domain, and CH4 domain) derived from two heavy chains of IgM and IgE antibodies. Unless otherwise stated herein, amino acid residues in the Fc region or heavy chain constant region are numbered according to the EU numbering system (also known as the EU Index) as described in, e.g., Kabat et al., Sequences of Proteins of Immunological Interes, 5th Ed., Public Health Service, National Institutes of Health, Bethesda, MD, 1991. However, the C-terminal lysine (Lys447) of the Fc region may or may not be present. Two Fc regions can constitute a dimeric Fc by dimerization, and two different Fc form a heterodimeric Fc by heterodimerization. As used herein, the terms "Fc region", "Fc portion", and "dimeric Fc (e.g., heterodimeric Fc)" do not comprise a heavy chain variable region VH and a light chain variable region VL as well as a heavy chain constant region CH1 and a light chain constant region CL of an immunoglobulin, but in some cases, they can comprise a hinge region at the N-terminus of the heavy chain constant region. In one embodiment, a human IgG heavy chain Fc region extends from Asp221 or from Cys226 or from Asp231 to the carboxyl-terminus of the heavy chain. In one embodiment, the Fc region is a human Fc region. In one embodiment, the Fc region belongs to a human IgG4 subclass. In one embodiment, the Fc region belongs to a human IgG1 subclass.

Herein, "Fc dimerization" refers to the formation of a dimer of two Fc regions by the dimerization. "Fc heterodimerization" refers to the formation of a dimer of two different Fc regions by the dimerization. Heterodimeric Fc regions constitute an Fc scaffold in bispecific antibody or multispecific antibody. Therefore, "heterodimeric Fc scaffold" refers to a scaffold comprising two different Fc regions or formed by the dimerization of two different Fc regions, which may be linked to a domain that binds to an antigen (e.g., a heavy and/or light chain variable region of an antibody or an antigen-binding fragment of an antibody that can bind to a target molecule, or a soluble moiety of a ligand or receptor that can bind to a target molecule) at its N-terminus or C-terminus to constitute a multispecific antibody, e.g., a bispecific antibody.

An amino acid mutation is indicated by "original amino acid, amino acid position, mutated amino acid". For example, when the mutation site is located in the Fc region, "T366W" means that T located at EU numbering position 366 is substituted with W. When describing combinations of mutations, the combined mutations are joined by "and" or "/"*.* "R521K/Y1069C" indicates that both mutations R521K and Y1069C are contained. It should be noted that when describing a mutation, a particular position encompasses its corresponding amino acid positions on other polypeptide chains. For example, if C220 is mentioned, it encompasses an amino acid at position 220 of the IgG1 heavy chain according to EU numbering, as well as the corresponding amino acids in other heavy chains, e.g., an amino acid at position 131 in IgG2, IgG3, or IgG4. When describing a mutation, the original amino acid at a particular position may be the described amino acid or may be other amino acids at the corresponding positions.

"Complementarity determining region" or "CDR region" or "CDR" is a region in an antibody variable domain that is highly variable in sequence and forms a structurally defined loop ("hypervariable loop") and/or comprises antigen-contacting residues ("antigen contact sites"). CDRs are primarily responsible for binding to antigen epitopes. CDRs of heavy and light chains are generally referred to as CDR1, CDR2, and CDR3, and are numbered sequentially from the N-terminus. The CDRs located in a heavy chain variable region of an antibody are referred to as HCDR1, HCDR2, and HCDR3, whereas the CDRs located in a light chain variable region of an antibody are referred to as LCDR1, LCDR2, and LCDR3. In a given amino acid sequence of a light chain variable region or a heavy chain variable region, the exact amino acid sequence boundary of each CDR may be determined using any one or a combination of many well-known antibody CDR assignment systems including, e.g., Chothia based on the three-dimensional structure of antibodies and the topology of the CDR loops (Chothia et al., (1989) Nature, 342: 877-883; Al-Lazikani et al., "Standard conformations for the canonical structures of immunoglobulins", Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

For example, according to different CDR determination schemes, the residues of each CDR are described as follows.

| CDR | Kabat scheme | AbM scheme | Chothia scheme | Contact scheme |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 | H31-H35B | H26-H35B | H26-H32 | H30-H35B |
| (Kabat numbering system) | | | | |
| HCDR1 | H31-H35 | H26-H35 | H26-H32 | H30-H35 |
| (Chothia numbering system) | | | | |
| HCDR2 | H50-H65 | H50-H58 | H53-H55 | H47-H58 |
| HCDR3 | H95-H102 | H95-H102 | H96-H101 | H93-H101 |
| (Kabat numbering system) | | | | |

CDRs can also be determined based on having the same Kabat numbering positions as a reference CDR sequence (e.g., any one of the exemplary CDRs of the present disclosure).

Unless otherwise stated, the term "CDR" or "CDR sequence" used herein encompasses CDR sequences determined by any one of the schemes described above.

Unless otherwise stated, residue positions of an antibody variable region (including heavy chain variable region residues and light chain variable region residues) used herein are numbered according to the Kabat numbering system (Kabat et al., Sequences of Proteins of Immunological Interest, 5th Ed., Public Health Service, National Institutes of Health, Bethesda, Md. (1991)).

In one embodiment, the boundaries of CDRs of the antibody of the present disclosure are determined by the Kabat rule, the AbM rule, or a combination thereof.

In one embodiment of the present disclosure, the HCDR1 of the VH in the antigen-binding region binding to B7-H3 of the present disclosure is determined by the AbM rule, and the HCDR2 and the HCDR3 are determined by the Kabat rule, and the CDRs of the VL are determined by the Kabat rule.

In one embodiment of the present disclosure, the CDRs of the VH and the VL in the antigen-binding region binding to EGFR in the present disclosure are determined by the Kabat rule.

The term "hinge region" refers to a portion of an antibody heavy chain polypeptide that links to the CH1 and CH2 regions in the wild-type antibody heavy chain, e.g., an IgG1 hinge region, e.g., a sequence from D221 to P230 according to EU numbering. The hinge regions of other IgG subclasses can be determined by alignment with the cysteine residues in the hinge region of the IgG1 subclass sequence.

The term "binding site", "antigen-binding site", or "antigen-binding region" as used herein refers to any portion of an antibody molecule, e.g., a multispecific antibody, e.g., a bispecific antibody, that binds to a specific target or antigen. The antigen-binding region may be, for example, an antibody or immunoglobulin per se or an antibody fragment. Such antigen-binding regions may or may not have a tertiary structure independent of the remainder of BsAB, and may or may not bind to their antigens/epitopes as separate entities. In some embodiments, the antigen-binding region comprises an antibody light chain variable region (VL) and an antibody heavy chain variable region (VH) that constitute a VH/VL pair, a heavy chain variable region derived from a camelidae heavy chain antibody, a VH-like single domain (v-NAR) of IgNAR from a shark, a camelized human VH domain, and a humanized camelidae antibody heavy chain variable region.

When referring to a "first antigen-binding region" in a multispecific antibody or a bispecific antibody, it refers to a binding region that binds to a first antigen and is not intended to limit the number of such antigen-binding regions contained in the antibody, for example, the multispecific antibody or the bispecific antibody can comprise one or more first antigen-binding regions. For example, the bispecific antibody comprises a first antigen-binding region and a second antigen-binding region but may comprise one or more first antigen-binding regions and one or more second antigen-binding regions.

When referring to "an antigen-binding region derived from an antibody", it is meant that the binding domain constituting the antigen-binding region is or is derived from a binding domain of the antibody that specifically binds to the antigen, for example, the fragment, such as Fab, of the antigen-binding region that specifically binds to the antigen is or is derived from a corresponding fragment, such as Fab, of the antibody, or the heavy chain variable region and/or the light chain variable region of the antigen-binding region is or is derived from a heavy chain variable region and/or a light chain variable region of the antibody, or 1, 2, 3, 4, 5, or 6 CDRs of the antigen-binding region are CDRs of the antibody. In some embodiments, the antigen-binding region is an antigen-binding fragment of an antibody.

As used herein, the term "multispecific antibody" refers to an antibody having at least two antigen-binding sites, each of which binds to a different epitope of the same antigen or a different epitope of a different antigen. The antibody provided herein is generally a multispecific antibody, such as a bispecific antibody. A multispecific antibody is an antibody having binding specificities for at least two different epitopes. In one embodiment, provided herein is a bispecific antibody having binding specificities for a first antigen and a second antigen.

The term "immunoglobulin molecule" refers to a protein having a structure of a naturally existing antibody. For example, an IgG is a heterotetrameric glycoprotein of about 150,000 Daltons consisting of two light chains and two heavy chains which are disulfide-bonded. Each immunoglobulin heavy chain has a heavy chain variable region (VH), also called a heavy chain variable domain, followed by three heavy chain constant domains (CH1, CH2, and CH3), from N-terminus to C-terminus. Similarly, each immunoglobulin light chain has a light chain variable region (VL), also called a light chain variable domain, followed by a light chain constant domain (CL) from N-terminus to C-terminus. The heavy chains of an immunoglobulin can be assigned to one of 5 classes, α (IgA), δ (IgD), ε (IgE), γ (IgG), or µ (IgM), in which some classes can be further divided into subclasses such as γ₁ (IgG1), γ₂ (IgG2), γ₃ (IgG₃), γ₄ (IgG₄), α₁ (IgA₁), and α₂ (IgA₂). The light chains of an immunoglobulin can be divided into one of the two classes, κ or λ, based on the amino acid sequence of constant domains thereof. An immunoglobulin consists essentially of two Fab molecules and one Fc domain linked by an immunoglobulin hinge region.

The term "effector function" refers to biological activities attributed to an immunoglobulin Fc region that vary with immunoglobulin isotype. Examples of immunoglobulin effector functions include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (such as B-cell receptors), and B-cell activation.

The term "...valent" antibody refers to the number of antigen-binding sites present in an antibody molecule. "Bivalent", "trivalent", and "tetravalent" antibodies refer to the presence of 2 antigen-binding sites, 3 antigen-binding sites, and 4 antigen-binding sites in an antibody molecule, respectively.

As used herein, the term "bind to" or "specifically bind to" means that the binding effect is selective for antigens and may be distinguished from unwanted or non-specific interactions. The ability of an antigen-binding site to bind to a particular antigen may be determined by enzyme-linked immunosorbent assay (ELISA) or conventional binding assays known in the art, such as radioimmunoassay (RIA), bio-layer interferometry, MSD assay, or surface plasmon resonance (SPR).

"Affinity" or "binding affinity" refers to the inherent binding affinity that reflects the interaction between members of a binding pair. The affinity of molecule X for its partner Y can be generally represented by a dissociation constant (K_{D}), which is a ratio of the dissociation rate constant to the association rate constant (K_{dis} and Kₒₙ, respectively). Affinity can be measured by common methods known in the art. One specific method for measuring affinity is the ForteBio kinetic binding assay described herein.

A "knob-in-hole" mutation is used herein to refer to the introduction of mutations in a first Fc polypeptide and a second Fc polypeptide, respectively, using the "knob-in-hole" technique to form a protuberance ("knob") and a complementary cavity ("hole") at the interface of the first Fc polypeptide and the interface of the second Fc polypeptide. It is known in the art that the "knob-in-hole" technique enables the engineering of the interface between different chains of an antibody molecule to promote the correct association of the chains of the antibody molecule. Generally, this technique involves introducing a "protuberance/knob" at the interface of one chain, and introducing a corresponding "cavity/hole" at the interface of the other chain to be paired with, such that the protuberance can be placed at the cavity. A preferred interface comprises the CH3 domain from the heavy chain constant domains of one chain and the CH3 domain from the heavy chain constant domains of the other chain to be paired with. The protuberance can be constructed by replacing small amino acid side chains at an interface of the CH3 domain from the heavy chain constant domains of one chain with large side chains, such as tyrosine or tryptophan. The compensating cavity of the same size as, or a similar size to, the protuberance is constructed at an interface of the CH3 domain from the heavy chain constant domains of the other chain to be paired with, by replacing large amino acid side chains with small side chains (e.g., alanine or threonine). Another optional interface comprises a light chain CL domain and a heavy chain CH1 domain of the Fab fragment described above, and the correct heterodimerization between the two chains of the Fab fragment is promoted by constructing a protuberance-cavity interaction.

Herein, an antibody constant region or antibody constant domain, including CH1, CL, and Fc domains, and CH2, CH3, and optional CH4 domains that constitute the Fc domain, may be selected according to the intended function of the antibody molecule. For example, the constant region may be an IgA, IgD, IgE, IgG, or IgM region, particularly an immunoglobulin constant domain of human IgG, such as a constant domain of human IgG1, IgG2, IgG3, or IgG4, preferably a constant domain of human IgG1. For another example, a Fab fragment of the antibody may comprise CH and CL constant regions derived from IgG1. For another example, an Fc region of the antibody may comprise CH2 and CH3 domains derived from IgG1. The immunoglobulin constant region may have a natural sequence or a variant sequence.

The term "antigen" refers to a molecule that induces an immune response. Such an immune response may involve antibody production or activation of specific immune cells, or both. Those skilled will understand that any macromolecules, including essentially all proteins or peptides, can be used as antigens. In addition, an antigen may be derived from recombinant or genomic DNA. In some embodiments described herein, the first antigen and the second antigen are two different antigens.

The terms "tumor-associated antigen" and "cancer antigen" are used interchangeably to refer to molecules (generally proteins, carbohydrates, or lipids) preferably expressed completely or as fragments (e.g., MHC/peptide) on the surface of cancer cells, compared to normal cells, and the molecules can be used in the preferential targeting of cancer cells by the agent.

The term "immune checkpoint molecule" means a class of inhibitory signaling molecules that are present in the immune system, which avoid tissue damage by regulating the persistence and strength of the immune response in peripheral tissues, and participate in maintaining the tolerance to self-antigens.

The term "cytokine" is a generic term for proteins that are released by a cell population and act as intercellular mediators on another cell.

"Immunoconjugate" is an antibody conjugated to one or more other substances, including but not limited to cytotoxic agents or labels.

As used herein, the term "EGFR" refers to the epidermal growth factor receptor, which is a tyrosine kinase receptor, a large transmembrane glycoprotein with a molecular weight of about 170 kDa, and a member of the ErbB receptor family. EGFR is the most common oncogenic driver gene in NSCLC. An activating mutation region of EGFR mainly occurs in the tyrosine kinase domain of exons 18-21 of EGFR. A binding region of an EGFR antibody is mainly located in an extracellular ligand domain region of EGFR, which can prevent the occurrence of resistance mutations. At the same time, the EGFR antibody can inhibit the growth of tumor cells by inhibiting the binding of EGFR to a ligand, and can also kill tumor cells with immune cells by utilizing its specific ADCC (antibody-dependent cell-mediated cytotoxicity), such that an anti-tumor killing role can be jointly played through multiple mechanisms of action.

The terms "B7-H3", "B7H3", and "CD276" are used interchangeably herein. B7-H3 is a type I transmembrane glycoprotein, which is a member of the B7/CD28 superfamily, and is similar in sequence to the extracellular domain of PD-L1. B7-H3 has 316 amino acids and comprises a putative signal peptide consisting of 28 amino acids, an extracellular region consisting of 217 amino acids, a transmembrane region, and a cytoplasmic domain consisting of 45 amino acids, with a molecular weight of about 45-66 kDa. In humans, the extracellular structure of B7-H3 may be an IgV-IgC-like domain (2Ig-B7-H3) or an IgV-IgC-IgV-IgC-like domain (4Ig-B7-H3) due to exon duplication. The sequence of cynomolgus monkey B7-H3 has about 90% homology with its human counterpart.

"Effector function" refers to biological activities which can be attributed to the antibody Fc region and vary with the antibody isotype. Examples of antibody effector functions include: C1q binding and complement-dependent cytotoxicity (CDC), Fc receptor binding, antibody-dependent cell-mediated cytotoxicity (ADCC), phagocytosis, cell surface receptors (such as B cell receptors) down-regulation, and B cell activation.

The term "GlymaxX technology" is a technology platform developed by ProBioGen corporation that increases the ADCC effect of a bispecific antibody. It is a CHO host cell that stably overexpresses a bacterial RMD protein, and finally blocks the fucose modification of the Fc region by interfering with the formation of the substrate GDP-fucose, thereby enhancing the ADCC effect (WO2011035884A1).

The term "effective amount" refers to an amount or dosage of the antibody, fragment, conjugate, or composition of the present disclosure which generates expected effects in a patient in need of treatment or prevention after being administered to the patient in a single or multiple doses. The effective amount can be easily determined by an attending physician as a person skilled in the art by considering a variety of factors as follows: species such as mammals; its size, age, and general health condition; the specific disease involved; the extent or severity of the disease; response in an individual patient; specific antibody administered; route of administration; bioavailability characteristics of the administered formulation; selected dosing regimen; and use of any concomitant therapy.

"Therapeutically effective amount" refers to an amount effective to achieve a desired therapeutic result at a necessary dose for a necessary period of time. The therapeutically effective amount of an antibody or antibody fragment, or a conjugate or composition thereof may vary depending on a variety of factors such as disease state, age, sex, and weight of an individual, and the ability of the antibody or antibody portion to elicit a desired response in an individual. The therapeutically effective amount is also such an amount that any toxic or adverse effect of the antibody or antibody fragment, or the conjugate or composition thereof is inferior to the therapeutically beneficial effect. The "therapeutically effective amount" inhibits a measurable parameter (e.g., tumor growth rate) by preferably at least about 20%, more preferably at least about 40%, even more preferably at least about 50%, 60%, or 70%, and still more preferably at least about 80%, relative to untreated subjects. The ability of a compound to inhibit a measurable parameter (e.g., cancer) can be evaluated in an animal model system that predicts efficacy in human tumors. Optionally, such property of a composition can be evaluated by examining the inhibition ability of

the compound, which can be measured *in vitro* by assays known to those skilled.

"Prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

The term "variable region" or "variable domain" refers to a domain of a heavy chain or light chain of an antibody involved in the binding of the antibody to an antigen. Variable domains of heavy chains and light chains of natural antibodies typically have similar structures, wherein each domain comprises four conserved framework regions (FRs) and three complementarity determining regions (CDRs). (See, e.g., Kindt et al., Kuby Immunology, 6th ed., WH. Freeman and Co., p. 91 (2007)). A single VH or VL domain may be sufficient to provide antigen-binding specificity. In addition, a VH or VL domain from an antibody binding to a particular antigen can be used to isolate antibodies that bind to the antigen, so as to screen for libraries of complementary VL or VH domains.

The term "host cell" refers to a cell into which an exogenous polynucleotide has been introduced, including the progeny of such cells. Host cells include "transformants" and "transformed cells", which include primary transformed cells and progeny derived therefrom, regardless of the number of passages. Progeny may not be exactly the same as parent cells in terms of nucleic acid content, and may comprise mutations. Mutant progenies having the same function or biological activities that are screened or selected from the initially transformed cells are included herein. Host cells are any type of cell system that can be used to produce the antibody molecule of the present disclosure, including eukaryotic cells, e.g., mammalian cells, insect cells, and yeast cells; and prokaryotic cells, e.g., E. *coli* cells. Host cells include cultured cells, as well as cells within a transgenic animal, a transgenic plant, or cultured plant tissue or animal tissue.

The term "anti-tumor effect" refers to a biological effect that can be demonstrated by a variety of means, including but not limited to, for example, a decrease in tumor volume, a decrease in number of tumor cells, a decrease in tumor cell proliferation, or a decrease in tumor cell viability.

The terms "tumor" and "cancer" are used interchangeably herein and encompass solid and liquid tumors.

The terms "cancer" and "cancerous" refer to or describe a physiological disease in mammals that is typically characterized by unregulated cell growth. In certain embodiments, cancers suitable for treatment with the antibody of the present disclosure include a cancer of epithelial origin, e.g., skin cancer (e.g., cutaneous squamous cell carcinoma or head and neck cancer such as head and neck squamous cell carcinoma), esophageal cancer (e.g., esophageal squamous carcinoma), intestinal cancer (e.g., colon cancer, rectal cancer, or colorectal cancer), or lung cancer (e.g., non-small cell lung cancer, lung squamous cell carcinoma, or lung adenocarcinoma), including metastatic forms of such cancers.

The term "tumor" refers to all neoplastic cell growth and proliferation, whether malignant or benign, and all pre-cancerous and cancerous cells and tissues. The terms "cancer", "cancerous", and "tumor" are not mutually exclusive when referred to herein.

The term "infectious disease" refers to a disease caused by a pathogen, including, e.g., viral infection, bacterial infection, fungal infection, or caused by a protozoan, e.g., parasitic infection.

The term "chronic infection" refers to an infection in which an infectious agent (e.g., a pathogen such as a virus, a bacterium, a protozan such as a parasite, or a fungus) has induced an immune response in an infected host, but has not been cleared or eliminated from the host as in acute infections.

The term "label" used herein refers to a compound or composition which is directly or indirectly conjugated or fused to an agent, such as a polynucleotide probe or an antibody, and facilitates the detection of the agent to which it is conjugated or fused. The label itself can be detectable (e.g., a radioisotope label or a fluorescent label) or can catalyze a chemical change to a detectable substrate compound or composition in the case of enzymatic labeling. The term is intended to encompass direct labeling of a probe or an antibody by coupling (i.e., physical linking) a detectable substance to the probe or the antibody and indirect labeling of a probe or an antibody by reacting with another reagent which is directly labeled. Examples of indirect labeling include detection of a primary antibody using a fluorescently labeled secondary antibody, and end labeling of a biotinylated DNA probe such that it can be detected with a fluorescently labeled streptavidin.

"Individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human.

An "isolated" antibody is an antibody which has been separated from components of its natural environment. In some embodiments, the antibody is purified to a purity greater than 95% or 99% as determined by, e.g., electrophoresis (e.g., SDS-PAGE, isoelectric focusing (IEF) and capillary electrophoresis) or chromatography (e.g., ion exchange or reverse-phase HPLC). For a review of methods for assessing antibody purity, see, e.g., Flatman et al., J. Chromatogr., B848: 79-87 (2007).

"Isolated" nucleic acid refers to a nucleic acid molecule that has been separated from components of its natural environment. The isolated nucleic acid includes a nucleic acid molecule contained in a cell that typically comprises the nucleic acid molecule, but the nucleic acid molecule exists extrachromosomally or at a chromosomal location that is different from its natural chromosomal location.

The calculation of sequence identity between sequences is performed as follows. To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be discarded for comparison). In one preferred embodiment, for comparison purposes, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate the percent identity between the sequences. In one preferred embodiment, the percent identity between two amino acid sequences is determined with the Needlema and Wunsch algorithm ((1970) J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide sequences is determined with the GAP program of the GCG software package (available at http://www.gcg.com), using the NWSgapdna.CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5. The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0). Additionally or alternatively, the nucleic acid sequences and protein sequences described herein can be further used as "query sequences" to perform searches against public databases to, e.g., identify other family member sequences or related sequences.

The term "pharmaceutical composition" refers to a composition that exists in a form allowing effective biological activity of the active ingredient contained therein and does not contain additional ingredients having unacceptable toxicity to a subject to which the composition is administered.

The term "pharmaceutical supplementary material" refers to diluents, adjuvants (e.g., Freund's adjuvants (complete and incomplete)), carriers, excipients or stabilizers, and the like, which are co-administered with active substance.

As used herein, "treatment", "treat", or "treating" refers to slowing, interrupting, arresting, alleviating, stopping, lowering, or reversing the progression or severity of an existing symptom, condition, disorder, or disease. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis. In some embodiments, the antibody molecule of the present disclosure is used to delay the progression of a disease or to slow the progression of a disease.

As used herein, "prevention", "prevent", or "preventing" includes the inhibition of the development or progression of symptoms of a disease or condition, or a specific disease or condition. In some embodiments, subjects with family history of cancer are candidates for preventive regimens. Generally, in the context of cancer, the term "prevention" "prevent", or "preventing" refers to the administration of a drug prior to the onset of signs or symptoms of cancer, particularly in subjects at risk of cancer.

The term "effective amount" refers to an amount or dosage of the antibody, fragment, composition, or combination of the present disclosure which generates expected effects in a patient in need of treatment or prevention after being administered to the patient in a single dose or multiple doses.

"Therapeutically effective amount" refers to an amount effective to achieve a desired therapeutic result at a necessary dose for a necessary period of time. The therapeutically effective amount is also such an amount that any toxic or undesired effect of the antibody, fragment thereof, composition, or combination is inferior to the therapeutically beneficial effect. The "therapeutically effective amount" preferably inhibits a measurable parameter or improves a measurable parameter by at least about 40%, and even more preferably by at least about 50%, 55%, 60%, 65%, 70%, 75%, 80%, 85%, 90%, or even 100%, relative to an untreated subject.

"Prophylactically effective amount" refers to an amount effective to achieve a desired prophylactic result at a necessary dose for a necessary period of time. Generally, since a prophylactic dose is administered in a subject before or at an earlier stage of a disease, a prophylactically effective amount will be less than a therapeutically effective amount.

The term "therapeutic agent" described herein encompasses any substance effective in preventing or treating diseases, such as tumors (e.g., cancer) and infections (e.g., chronic infection), including anti-angiogenic agents, chemotherapeutic agents, cytotoxic agents, vaccines, additional antibodies, anti-infective active agents, small molecule drugs, or immunomodulatory agents.

"Chemotherapeutic agent" includes chemical compounds useful in the treatment of cancer, including but not limited to anti-tumor agents including alkylating agents, antimetabolites, natural products, antibiotics, enzymes, miscellaneous agents, hormones and antagonists, antiestrogens, antiandrogens, and non-steroidal antiandrogens, and the like.

The term "immunomodulatory agent" used herein refers to a natural or synthetic active agent or drug that suppresses or modulates an immune response. The immune response may be a humoral response or a cellular response. The immunomodulatory agents include an inhibitor of an immune checkpoint molecule and an activator of a co-stimulatory molecule.

The term "small molecule drug" refers to an organic compound having a low molecular weight and capable of regulating biological processes.

As used herein, the term "cytotoxic agent" refers to a substance that inhibits or prevents cell function and/or causes cell death or destruction. The cytotoxic agent includes, but is not limited to, radioisotopes; chemotherapeutic agents or drugs; growth inhibitors; enzymes and fragments thereof such as nucleolytic enzymes; antibiotics; toxins such as small molecule toxins or enzymatic toxins of bacterial, fungal, plant, or animal sources, including fragments and/or variants thereof.

"Tumor immune escape" refers to a process by which tumors evade immune recognition and clearance. As such, as a therapeutic concept, tumor immunity is "treated" when such evasion diminishes, and the tumor is recognized and attacked by the immune system. Examples of tumor recognition include tumor binding, tumor shrinkage, and tumor clearance.

"Immunogenicity" refers to the ability of a particular substance to elicit an immune response. Tumors are immunogenic and enhancing tumor immunogenicity helps to eliminate tumor cells by the immune response.

As used herein, "agonist activity of an antibody" refers to the ability of an antibody to activate the biological activity of the antigen to which it binds.

"Anti-angiogenic agent" refers to a compound that blocks or interferes to some extent with vascular development. The anti-angiogenic agent may be, for example, a small molecule or an antibody binding to a growth factor or growth factor receptor involved in promoting angiogenesis.

The term "pharmaceutical combination or combination product" refers to a non-fixed combination product or a fixed combination product, including but not limited to, a kit and a pharmaceutical composition. The term "non-fixed combination" means that the active ingredients (e.g., (i) the immunoconjugate of the present disclosure, and (ii) an additional therapeutic agent) are administered, either simultaneously or sequentially (without specific time limitation or at identical or different time intervals), to a patient as separate entities, wherein such administration provides two or more prophylactically or therapeutically effective active agents in the patient. The term "fixed combination" means that two or more active agents are administered to a patient simultaneously in the form of a single entity. The dose and/or time intervals of two or more active agents are preferably selected such that the combined use of the components can result in a therapeutic effect on the disease or condition which is greater than that achieved by the use of either component alone. The ingredients may each take a separate formulation form and such separate formulation forms may be the same or different.

The term "combination therapy" or "combined therapy" means that two or more therapeutic agents are administered to treat a cancer or an infection as described herein. Such administration includes co-administration of these therapeutic agents in a substantially simultaneous manner, for example, in a single capsule with a fixed proportion of active ingredients. Alternatively, such administration includes co-administration or separate administration or sequential administration of active ingredients in a variety of or separate containers (such as tablets, capsules, powder, and liquid). The powder and/or liquid can be reconstituted or diluted to a desired dose before administration. In some embodiments, the administration also includes using each type of therapeutic agents in a sequential manner at approximately the same time or at different times. In any case, the therapeutic regimen will provide the beneficial effect of the pharmaceutical combination in the treatment of conditions or symptoms described herein.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of nucleic acids to which they are operatively linked. Such vectors are referred to as "expression vectors" herein.

"Subject/patient sample" refers to a collection of cells or fluids obtained from a patient or a subject. The source of tissue or cell samples can be solid tissues, e.g., from fresh, frozen, and/or preserved organ or tissue samples or biopsy samples or puncture samples; blood or any blood component; body fluids such as cerebrospinal fluids, amniotic fluids, peritoneal fluids, or interstitial fluids; and cells from a subject at any time during pregnancy or development. Tissue samples may comprise compounds that are naturally not mixed with tissues, such as preservatives, anticoagulants, buffers, fixatives, nutrients, and antibiotics. Examples of tumor samples include but are not limited to tumor biopsies, fine needle aspirates, bronchial lavage fluids, pleural fluids, sputa, urine, surgical specimens, circulating tumor cells, serum, plasma, circulating plasma proteins, ascites, primary cell cultures or cell lines derived from tumors or exhibiting tumor-like properties, and preserved tumor samples such as formalin-fixed, paraffin-embedded tumor samples, or frozen tumors samples.

### II. Antibody molecule of the present disclosure

The present disclosure provides a novel antibody molecule that can be used for immunotherapy, prevention, and/or diagnosis of a variety of diseases. The antibody molecule of the present disclosure comprises at least 2, 3, or 4 antigen-binding regions, and can function as a bispecific antibody or a multispecific antibody, and preferably, function as a bispecific antibody.

In some embodiments, the bispecific antibody or the multispecific antibody of the present disclosure comprises a first binding specificity for EGFR and a second binding specificity for B7H3, and optionally an additional binding specificity.

Therefore, one aspect of the present disclosure relates to a bispecific antibody, which comprises a first antigen-binding region and a second antigen-binding region, wherein the first antigen-binding region specifically binds to EGFR, and/or the second antigen-binding region specifically binds to B7-H3.

The bispecific antibody of the present disclosure can be prepared using bispecific antibody formats or techniques known in the art. Specific exemplary bispecific formats that can be used in the context of the present disclosure are described, e.g., in Labrijn, et al., Bispecific antibodies: a mechanistic review of the pipeline, Nature Reviews Drug Discovery, 2019, 18(8): 1-24. In one embodiment, the bispecific antibody formats include an IgG-like antibody (Fan et al., (2015) Journal of Hematology & Oncology. 8: 130). The most common form of IgG-like antibodies comprises two Fab regions and two Fc regions, the heavy and light chains of each Fab may be from a separate monoclonal antibody. In some embodiments, the bispecific antibody of the present disclosure is an IgG-like bispecific antibody, which comprises a Fab fragment specifically binding to EGFR as an antigen-binding region and a Fab fragment specifically binding to B7H3 as another antigen-binding region.

### ➢ Antigen-binding region specifically binding to EGFR

In some embodiments, the antigen-binding region specifically binding to EGFR is derived from an antibody specifically binding to EGFR, e.g., an EGFR antibody disclosed in WO02100348A2, e.g., zalutumumab.

In some embodiments, the antigen-binding region specifically binding to EGFR comprises 1, 2, 3, 4, 5, or 6 CDRs of a known antibody specifically binding to EGFR, e.g., an EGFR antibody disclosed in WO02100348A2, e.g., zalutumumab.

In some embodiments, the antigen-binding region specifically binding to EGFR comprises 1, 2, and 3 heavy chain variable region CDRs, i.e., HCDR1, HCDR2, and HCDR3, of a known antibody specifically binding to EGFR, e.g., an EGFR antibody disclosed in WO02100348A2, e.g., zalutumumab.

In some embodiments, the antigen-binding region specifically binding to EGFR comprises 1, 2, and 3 light chain variable region CDRs, i.e., LCDR1, LCDR2, and LCDR3, of a known antibody specifically binding to EGFR, e.g., an EGFR antibody disclosed in WO02100348A2, e.g., zalutumumab.

In some embodiments, the antigen-binding region specifically binding to EGFR comprises 3 heavy chain variable region CDRs and 3 light chain variable region CDRs of a known antibody specifically binding to EGFR, e.g., an EGFR antibody disclosed in WO02100348A2, e.g., zalutumumab.

In some embodiments, the antigen-binding region specifically binding to EGFR comprises a heavy chain variable region and a light chain variable region of a known antibody specifically binding to EGFR, e.g., an EGFR antibody disclosed in WO02100348A2, e.g., zalutumumab.

In some embodiments, the antigen-binding region specifically binding to EGFR comprises a Fab of a known antibody specifically binding to EGFR, e.g., an EGFR antibody disclosed in WO02100348A2, e.g., zalutumumab.

In some embodiments, the antigen-binding region specifically binding to EGFR comprises 3 complementarity determining regions from the heavy chain variable region (HCDRs), i.e., HCDR1, HCDR2, and HCDR3. In some embodiments, the antigen-binding region specifically binding to EGFR comprises 3 complementarity determining regions from the light chain variable region (LCDRs), i.e., LCDR1, LCDR2, and LCDR3. In some embodiments, the antigen-binding region specifically binding to EGFR comprises 3 complementarity determining regions from the heavy chain variable region (HCDRs) and 3 complementarity determining regions from the light chain variable region (LCDRs).

In some aspects, the antigen-binding region specifically binding to EGFR comprises a heavy chain variable region (VH). In some aspects, the antigen-binding region specifically binding to EGFR comprises a light chain variable region (VL). In some aspects, the antigen-binding region specifically binding to EGFR comprises a heavy chain variable region (VH) and a light chain variable region (VL). In some embodiments, the heavy chain variable region comprises 3 complementarity determining regions (CDRs) from the heavy chain variable region, i.e., HCDR1, HCDR2, and HCDR3. In some embodiments, the light chain variable region comprises 3 complementarity determining regions (CDRs) from the light chain variable region, i.e., LCDR1, LCDR2, and LCDR3.

In some embodiments, the heavy chain variable region of the antigen-binding region specifically binding to EGFR
(i) comprises or consists of an amino acid sequence having at least 80%, 85%, 90%, 91%, 92%, 93%, 94%, 95%, 96%, 97%, 98%, or 99% identity to the amino acid sequence of SEQ ID NO: 1; or
(ii) comprises or consists of an amino acid sequence of SEQ ID NO: 1; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence of SEQ ID NO: 1, wherein preferably, the amino acid changes do not occur in the CDR regions.

In some embodiments, the light chain variable region of the antigen-binding region specifically binding to EGFR
(i) comprises or consists of an amino acid sequence having at least 80%, 85%, 90%, 91%, 92%, 93%, 94%, 95%, 96%, 97%, 98%, or 99% identity to the amino acid sequence of SEQ ID NO: 2; or
(ii) comprises or consists of an amino acid sequence of SEQ ID NO: 2; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with the amino acid sequence of SEQ ID NO: 2, wherein preferably, the amino acid changes do not occur in the CDR regions.

In some embodiments, the 3 complementarity determining regions from the heavy chain variable region (HCDRs), i.e., HCDR1, HCDR2, and HCDR3, of the antigen-binding region specifically binding to EGFR are selected from
(i) three complementarity determining regions HCDR1, HCDR2, and HCDR3 comprised in a VH set forth in SEQ ID NO: 1, or
(ii) a sequence comprising a total of at least one and no more than 5, 4, 3, 2, or 1 amino acid change (preferably amino acid replacement, and more preferably conservative replacement) in the three HCDR regions relative to any one sequence of (i).

For example, the CDRs are determined by the Kabat scheme.

In some embodiments, the 3 complementarity determining regions from the light chain variable region (LCDRs) of the antigen-binding region specifically binding to EGFR, i.e., LCDR1, LCDR2, and LCDR3, are selected from
(i) three complementarity determining regions LCDR1, LCDR2, and LCDR3 comprised in a VL set forth in SEQ ID NO: 2, or
(ii) a sequence comprising a total of at least one and no more than 5, 4, 3, 2, or 1 amino acid change (preferably amino acid replacement, and more preferably conservative replacement) in the three LCDR regions relative to any one sequence of (i).

For example, the CDRs are determined by the Kabat scheme.

In some embodiments, the antigen-binding region specifically binding to EGFR comprises 3 complementarity determining regions comprised in the heavy chain variable region (HCDRs) consisting of an amino acid sequence of SEQ ID NO: 1 and 3 complementarity determining regions comprised in the light chain variable region (LCDRs) consisting of an amino acid sequence of SEQ ID NO: 2.

In some embodiments, the HCDR1 comprises or consists of an amino acid sequence of SEQ ID NO: 9, or the HCDR1 comprises an amino acid sequence having one, two, or three changes (preferably amino acid replacements, and more preferably conservative replacements) compared with the amino acid sequence of SEQ ID NO: 9.

In some embodiments, the HCDR2 comprises or consists of an amino acid sequence of SEQ ID NO: 10, or the HCDR2 comprises an amino acid sequence having one, two, or three changes (preferably amino acid replacements, and more preferably conservative replacements) compared with the amino acid sequence of SEQ ID NO: 10.

In some embodiments, the HCDR3 comprises or consists of an amino acid sequence of SEQ ID NO: 11, or the HCDR3 comprises an amino acid sequence having one, two, or three changes (preferably amino acid replacements, and more preferably conservative replacements) compared with the amino acid sequence of SEQ ID NO: 11.

In some embodiments, the LCDR1 comprises or consists of an amino acid sequence set forth in SEQ ID NO: 12, or the LCDR1 comprises an amino acid sequence having one, two, or three changes (preferably amino acid replacements, and more preferably conservative replacements) compared with the amino acid sequence set forth in SEQ ID NO: 12.

In some embodiments, the LCDR2 comprises or consists of an amino acid sequence of SEQ ID NO: 13, or the LCDR2 comprises an amino acid sequence having one, two, or three changes (preferably amino acid replacements, and more preferably conservative replacements) compared with the amino acid sequence of SEQ ID NO: 13.

In some embodiments, the LCDR3 comprises or consists of the amino acid sequence set forth in SEQ ID NO: 14, or the LCDR3 comprises an amino acid sequence having one, two, or three changes (preferably amino acid replacements, and more preferably conservative replacements) compared with the amino acid sequence set forth in SEQ ID NO: 14.

In some embodiments of the present disclosure, the antigen-binding region specifically binding to EGFR comprises the HCDR1, the HCDR2, the HCDR3, the LCDR1, the LCDR2, and/or the LCDR3 described above.

In some embodiments of the present disclosure, the antigen-binding region specifically binding to EGFR comprises the HCDR1, the HCDR2, the HCDR3, the LCDR1, the LCDR2, and the LCDR3 described above.

In some embodiments of the present disclosure, the antigen-binding region specifically binding to EGFR comprises an HCDR1, an HCDR2, an HCDR3, an LCDR1, an LCDR2, and an LCDR3, wherein
the HCDR1 comprises or consists of an amino acid sequence of SEQ ID NO: 9;
the HCDR2 comprises or consists of an amino acid sequence of SEQ ID NO: 10;
the HCDR3 comprises or consists of an amino acid sequence of SEQ ID NO: 11;
the LCDR1 comprises or consists of an amino acid sequence of SEQ ID NO: 12;
the LCDR2 comprises or consists of an amino acid sequence of SEQ ID NO: 13; and
the LCDR3 comprises or consists of an amino acid sequence of SEQ ID NO: 14.

In some specific embodiments of the present disclosure, the antigen-binding region specifically binding to EGFR comprises an HCDR1 set forth in SEQ ID NO: 9, an HCDR2 set forth in SEQ ID NO: 10, an HCDR3 set forth in SEQ ID NO: 11, an LCDR1 set forth in SEQ ID NO: 12, an LCDR2 set forth in SEQ ID NO: 13, and an LCDR3 set forth in SEQ ID NO: 14.

In some specific embodiments of the present disclosure, the antigen-binding region specifically binding to EGFR comprises a VH and a VL, wherein
the VH comprises or consists of an amino acid sequence set forth in SEQ ID NO: 1 or an amino acid sequence having at least 80%, 85%, 90%, 91%, 92%, 93%, 94%, 95%, 96%, 97%, 98%, or 99% identity thereto, and the VL comprises or consists of an amino acid sequence set forth in SEQ ID NO: 2 or an amino acid sequence having at least 80%, 85%, 90%, 91%, 92%, 93%, 94%, 95%, 96%, 97%, 98%, or 99% identity thereto.

In some specific embodiments of the present disclosure, the antigen-binding region specifically binding to EGFR comprises or consists of a VH and a VL, wherein the VH and the VL comprise or consist of, respectively, amino acid sequences set forth in: SEQ ID NO: 1 and SEQ ID NO: 2.

### ➢ Antigen-binding region specifically binding to B7-H3

In some embodiments, the antigen-binding region specifically binding to B7-H3 is derived from an antibody specifically binding to B7-H3, e.g., a B7-H3 antibody described in PCT/CN2021/140449, e.g., the monoclonal antibody numbered Hz20G5 therein (e.g., Hz20G5.26 mentioned in the examples of the present disclosure).

In some embodiments, the antigen-binding region specifically binding to B7-H3 comprises 1, 2, 3, 4, 5, or 6 CDRs of a known antibody specifically binding to B7-H3, e.g., a B7-H3 antibody described in PCT/CN2021/140449, e.g., the monoclonal antibody numbered Hz20G5 therein.

In some embodiments, the antigen-binding region specifically binding to B7-H3 comprises 1, 2, and 3 heavy chain variable region CDRs, i.e., HCDR1, HCDR2, and HCDR3, of a known antibody specifically binding to B7-H3, e.g., a B7-H3 antibody described in PCT/CN2021/140449, e.g., the monoclonal antibody numbered Hz20G5 therein.

In some embodiments, the antigen-binding region specifically binding to B7-H3 comprises 1, 2, and 3 light chain variable region CDRs, i.e., LCDR1, LCDR2, and LCDR3, of a known antibody specifically binding to B7-H3, e.g., a B7-H3 antibody described in PCT/CN2021/140449, e.g., the monoclonal antibody numbered Hz20G5 therein.

In some embodiments, the antigen-binding region specifically binding to B7-H3 comprises 3 heavy chain variable region CDRs and 3 light chain variable region CDRs of a known antibody specifically binding to B7-H3, e.g., a B7-H3 antibody described in PCT/CN2021/140449, e.g., the monoclonal antibody numbered Hz20G5 therein.

In some embodiments, the antigen-binding region specifically binding to B7-H3 comprises a heavy chain variable region and a light chain variable region of a known antibody specifically binding to B7-H3, e.g., a B7-H3 antibody described in PCT/CN2021/140449, e.g., the monoclonal antibody numbered Hz20G5 therein.

In some embodiments, the antigen-binding region specifically binding to B7-H3 comprises a Fab of a known antibody specifically binding to B7-H3, e.g., a B7-H3 antibody described in PCT/CN2021/140449, e.g., the monoclonal antibody numbered Hz20G5 therein.

In some embodiments, the antigen-binding region specifically binding to B7-H3 comprises 3 complementarity determining regions from the heavy chain variable region (HCDRs), i.e., HCDR1, HCDR2, and HCDR3. In some embodiments, the antigen-binding region specifically binding to B7-H3 comprises 3 complementarity determining regions from the light chain variable region (LCDRs), i.e., LCDR1, LCDR2, and LCDR3. In some embodiments, the antigen-binding region specifically binding to B7-H3 comprises 3 complementarity determining regions from the heavy chain variable region (HCDRs) and 3 complementarity determining regions from the light chain variable region (LCDRs).

In some aspects, the antigen-binding region specifically binding to B7-H3 comprises a heavy chain variable region (VH). In some aspects, the antigen-binding region specifically binding to B7-H3 comprises a light chain variable region (VL). In some aspects, the antigen-binding region specifically binding to B7-H3 comprises a heavy chain variable region (VH) and a light chain variable region (VL). In some embodiments, the heavy chain variable region comprises 3 complementarity determining regions (CDRs) from the heavy chain variable region, i.e., HCDR1, HCDR2, and HCDR3. In some embodiments, the light chain variable region comprises 3 complementarity determining regions (CDRs) from the light chain variable region, i.e., LCDR1, LCDR2, and LCDR3.

In some embodiments, the heavy chain variable region of the antigen-binding region specifically binding to B7-H3
(i) comprises or consists of an amino acid sequence having at least 80%, 85%, 90%, 91%, 92%, 93%, 94%, 95%, 96%, 97%, 98%, or 99% identity to an amino acid sequence of SEQ ID NO: 3, 5, or 7; or
(ii) comprises or consists of an amino acid sequence of SEQ ID NO: 3, 5, or 7; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with an amino acid sequence of SEQ ID NO: 3, 5, or 7, wherein preferably, the amino acid changes do not occur in the CDRs.

In some embodiments, the light chain variable region of the antigen-binding region specifically binding to B7-H3
(i) comprises or consists of an amino acid sequence having at least 80%, 85%, 90%, 91%, 92%, 93%, 94%, 95%, 96%, 97%, 98%, or 99% identity to an amino acid sequence of SEQ ID NO: 4, 6, or 8; or
(ii) comprises or consists of an amino acid sequence of SEQ ID NO: 4, 6, or 8; or
(iii) comprises or consists of an amino acid sequence having one or more (preferably no more than 10, and more preferably no more than 5, 4, 3, 2, or 1) amino acid changes (preferably amino acid replacements, and more preferably conservative amino acid replacements) compared with an amino acid sequence of SEQ ID NO: 4, 6, or 8, wherein preferably, the amino acid changes do not occur in the CDRs.

In some embodiments, the 3 complementarity determining regions from the heavy chain variable region (HCDRs), i.e., HCDR1, HCDR2, and HCDR3, of the antigen-binding region specifically binding to B7-H3 are selected from
(i) three complementarity determining regions HCDR1, HCDR2, and HCDR3 comprised in a VH set forth in SEQ ID NO: 3, 5, or 7, or
(ii) a sequence comprising a total of at least one and no more than 5, 4, 3, 2, or 1 amino acid change (preferably amino acid replacement, and more preferably conservative replacement) in the three HCDR regions relative to any one sequence of (i).

For example, the HCDR1 is based on the Abm scheme, and the HCDR2 and the HCDR3 are determined by the Kabat scheme.

In some embodiments, the 3 complementarity determining regions from the light chain variable region (LCDRs), i.e., LCDR1, LCDR2, and LCDR3, of the antigen-binding region specifically binding to B7-H3 are selected from
(i) three complementarity determining regions LCDR1, LCDR2, and LCDR3 comprised in a VL set forth in SEQ ID NO: 4, 6, or 8, or
(ii) a sequence comprising a total of at least one and no more than 5, 4, 3, 2, or 1 amino acid change (preferably amino acid replacement, and more preferably conservative replacement) in the three LCDR regions relative to any one sequence of (i).

For example, the CDRs are determined by the Kabat scheme.

In some embodiments, the antigen-binding region specifically binding to B7-H3 comprises 3 complementarity determining regions comprised in the heavy chain variable region (HCDRs) consisting of an amino acid sequence of SEQ ID NO: 3, 5, or 7 and 3 complementarity determining regions comprised in the light chain variable region (LCDRs) consisting of an amino acid sequence of SEQ ID NO: 4, 6, or 8.

In some embodiments, the HCDR1 comprises or consists of an amino acid sequence of SEQ ID NO: 15, 21, or 27, or the HCDR1 comprises an amino acid sequence having one, two, or three changes (preferably amino acid replacements, and more preferably conservative replacements) compared with the amino acid sequence of SEQ ID NO: 15, 21, or 27.

In some embodiments, the HCDR2 comprises or consists of an amino acid sequence of SEQ ID NO: 16, 22, or 28, or the HCDR2 comprises an amino acid sequence having one, two, or three changes (preferably amino acid replacements, and more preferably conservative replacements) compared with the amino acid sequence of SEQ ID NO: 16, 22, or 28.

In some embodiments, the HCDR3 comprises or consists of an amino acid sequence of SEQ ID NO: 17, 23, or 29, or the HCDR3 comprises an amino acid sequence having one, two, or three changes (preferably amino acid replacements, and more preferably conservative replacements) compared with the amino acid sequence of SEQ ID NO: 17, 23, or 29.

In some embodiments, the LCDR1 comprises or consists of an amino acid sequence of SEQ ID NO: 18, 24, or 30, or the LCDR1 comprises an amino acid sequence having one, two, or three changes (preferably amino acid replacements, and more preferably conservative replacements) compared with the amino acid sequence of SEQ ID NO: 18, 24, or 30.

In some embodiments, the LCDR2 comprises or consists of an amino acid sequence of SEQ ID NO: 19, 25, or 31, or the LCDR2 comprises an amino acid sequence having one, two, or three changes (preferably amino acid replacements, and more preferably conservative replacements) compared with the amino acid sequence of SEQ ID NO: 19, 25, or 31.

In some embodiments, the LCDR3 comprises or consists of an amino acid sequence of SEQ ID NO: 20, 26, or 32, or the LCDR3 comprises an amino acid sequence having one, two, or three changes (preferably amino acid replacements, and more preferably conservative replacements) compared with the amino acid sequence of SEQ ID NO: 20, 26, or 32.

In some specific embodiments of the present disclosure, the antigen-binding region specifically binding to B7-H3 comprises the HCDR1, the HCDR2, the HCDR3, the LCDR1, the LCDR2, and/or the LCDR3 described above.

In some specific embodiments of the present disclosure, the antigen-binding region specifically binding to B7-H3 comprises the HCDR1, the HCDR2, the HCDR3, the LCDR1, the LCDR2, and the LCDR3 described above.

In some specific embodiments of the present disclosure, the antigen-binding region specifically binding to B7-H3 comprises the HCDR1, the HCDR2, the HCDR3, the LCDR1, the LCDR2, and the LCDR3, wherein
(i) the HCDR1 comprises or consists of an amino acid sequence set forth in SEQ ID NO: 15, the HCDR2 comprises or consists of an amino acid sequence set forth in SEQ ID NO: 16, and the HCDR3 comprises or consists of an amino acid sequence set forth in SEQ ID NO: 17;
   the LCDR1 comprises or consists of an amino acid sequence set forth in SEQ ID NO: 18, the LCDR2 comprises or consists of an amino acid sequence set forth in SEQ ID NO: 19, and the LCDR3 comprises or consists of an amino acid sequence set forth in SEQ ID NO: 20; or,
(ii) the HCDR1 comprises or consists of an amino acid sequence set forth in SEQ ID NO: 21, the HCDR2 comprises or consists of an amino acid sequence set forth in SEQ ID NO: 22, and the HCDR3 comprises or consists of an amino acid sequence set forth in SEQ ID NO: 23;
   the LCDR1 comprises or consists of an amino acid sequence set forth in SEQ ID NO: 24, the LCDR2 comprises or consists of an amino acid sequence set forth in SEQ ID NO: 25, and the LCDR3 comprises or consists of an amino acid sequence set forth in SEQ ID NO: 26; or,
(iii) the HCDR1 comprises or consists of an amino acid sequence set forth in SEQ ID NO: 27, the HCDR2 comprises or consists of an amino acid sequence set forth in SEQ ID NO: 28, and the HCDR3 comprises or consists of an amino acid sequence set forth in SEQ ID NO: 29;
the LCDR1 comprises or consists of an amino acid sequence set forth in SEQ ID NO: 30, the LCDR2 comprises or consists of an amino acid sequence set forth in SEQ ID NO: 31, and the LCDR3 comprises or consists of an amino acid sequence set forth in SEQ ID NO: 32.

In some specific embodiments of the present disclosure, the antigen-binding region specifically binding to B7-H3 comprises
(i) an HCDR1 set forth in SEQ ID NO: 15, an HCDR2 set forth in SEQ ID NO: 16, an HCDR3 set forth in SEQ ID NO: 17; an LCDR1 set forth in SEQ ID NO: 18, an LCDR2 set forth in SEQ ID NO: 19, and an LCDR3 set forth in SEQ ID NO: 20;
(ii) an HCDR1 set forth in SEQ ID NO: 21, an HCDR2 set forth in SEQ ID NO: 22, an HCDR3 set forth in SEQ ID NO: 23; an LCDR1 set forth in SEQ ID NO: 24, an LCDR2 set forth in SEQ ID NO: 25, and an LCDR3 set forth in SEQ ID NO: 26; or
(iii) an HCDR1 set forth in SEQ ID NO: 27, an HCDR2 set forth in SEQ ID NO: 28, an HCDR3 set forth in SEQ ID NO: 29; an LCDR1 set forth in SEQ ID NO: 30, an LCDR2 set forth in SEQ ID NO: 31, and an LCDR3 set forth in SEQ ID NO: 32.

In some specific embodiments of the present disclosure, the antigen-binding region specifically binding to B7-H3 comprises a VH and a VL, wherein
(i) the VH comprises or consists of an amino acid sequence set forth in SEQ ID NO: 3 or an amino acid sequence having at least 80%, 85%, 90%, 91%, 92%, 93%, 94%, 95%, 96%, 97%, 98%, or 99% identity to the amino acid sequence of SEQ ID NO: 3, and the VL comprises or consists of an amino acid sequence set forth in SEQ ID NO: 4 or an amino acid sequence having at least 80%, 85%, 90%, 91%, 92%, 93%, 94%, 95%, 96%, 97%, 98%, or 99% identity to the amino acid sequence of SEQ ID NO: 4;
(ii) the VH comprises or consists of an amino acid sequence set forth in SEQ ID NO: 5 or an amino acid sequence having at least 80%, 85%, 90%, 91%, 92%, 93%, 94%, 95%, 96%, 97%, 98%, or 99% identity to the amino acid sequence of SEQ ID NO: 5, and the VL comprises or consists of an amino acid sequence set forth in SEQ ID NO: 6 or an amino acid sequence having at least 80%, 85%, 90%, 91%, 92%, 93%, 94%, 95%, 96%, 97%, 98%, or 99% identity to the amino acid sequence of SEQ ID NO: 6; or
(iii) the VH comprises or consists of an amino acid sequence set forth in SEQ ID NO: 7 or an amino acid sequence having at least 80%, 85%, 90%, 91%, 92%, 93%, 94%, 95%, 96%, 97%, 98%, or 99% identity to the amino acid sequence of SEQ ID NO: 7, and the VL comprises or consists of an amino acid sequence set forth in SEQ ID NO: 8 or an amino acid sequence having at least 80%, 85%, 90%, 91%, 92%, 93%, 94%, 95%, 96%, 97%, 98%, or 99% identity to the amino acid sequence of SEQ ID NO: 8.

In some specific embodiments of the present disclosure, the antigen-binding region specifically binding to B7-H3 comprises a VH and a VL, wherein the VH and the VL comprise or consist of, respectively, amino acid sequences set forth in:
SEQ ID NO: 3 and SEQ ID NO: 4;
SEQ ID NO: 5 and SEQ ID NO: 6; or
SEQ ID NO: 7 and SEQ ID NO: 8.

The present disclosure also relates to an antibody specifically binding to B7-H3, which comprises the antigen-binding region specifically binding to B7-H3 described above, e.g., an antibody comprising the HCDR1, the HCDR2, and the HCDR3, and the LCDR1, the LCDR2, and the LCDR3 as defined herein; or an antibody comprising the VH or the VL as defined herein, or an antibody comprising the HC and the LC as defined herein.

### ➢ Fc region

In some embodiments, the antibody molecule of the present disclosure, e.g., a multispecific antibody (e.g., a bispecific antibody), further comprises Fc regions, wherein the Fc regions comprised may be identical or different.

In some embodiments, the Fc region has low fucosylation, e.g., low fucosylation obtained after treatment by the GlymaxX technology.

In some embodiments, the antibody molecule of the present disclosure comprises a first Fc region and a second Fc region, wherein the first Fc region and the second Fc region are identical or different.

In some embodiments, the first Fc region and the second Fc region are different and can form a heterodimeric Fc scaffold by dimerization.

Herein, an Fc region refers to a C-terminal region of an immunoglobulin heavy chain that contains at least a portion of the constant region, and may include Fc fragments of natural sequences and variant Fc fragments. The Fc regions of natural sequences encompass naturally occurring Fc sequences of various immunoglobulins, such as Fc regions of various Ig subtypes and allotypes thereof (Gestur Vidarsson et al., IgG subclasses and allotypes: from structure to effector functions, 20 October 2014, doi: 10.3389/fimmu.2014.00520). In some embodiments, the Fc region of the present disclosure comprises a CH2 and a CH3 of the antibody. In some embodiments, the antibody Fc region may also bear an IgG hinge region or a partial IgG hinge region at the N-terminus, e.g., an IgG1 hinge region or a partial IgG1 hinge region, e.g., according to EU numbering, a sequence of D221 to P230. The hinge region may comprise mutations.

Unless otherwise indicated herein, amino acid residues in the Fc region are numbered according to the EU numbering system, also called the EU index, as described in Kabat, E.A. et al., Sequences of Proteins of Immunological Interest, 5th Ed., Public Health Service, National Institutes of Health, Bethesda, MD (1991), NIH Publication 91-3242.

In some embodiments, the Fc region is a human IgG Fc, e.g., human IgG1 Fc, human IgG2 Fc, human IgG3 Fc, or human IgG4 Fc. In one embodiment, the Fc region comprises or consists of an amino acid sequence of SEQ ID NO: 46 or 47 or an amino acid sequence having at least 90% identity, e.g., 95%, 96%, 97%, 99% or more identity to the amino acid sequence.

As understood by those skilled in the art, to facilitate the formation of the multispecific antibody of the present disclosure as a heterodimer, the Fc region contained in the multispecific antibody of the present disclosure may comprise mutations that favor the heterodimerization of the first Fc region and the second Fc region. In one embodiment, mutations are introduced into the CH3 regions of the two Fc regions.

Methods for facilitating the heterodimerization of the Fc regions are known in the art. For example, the CH3 region of the first Fc region and the CH3 region of the second Fc region are engineered in a complementary manner, such that each CH3 region (or the heavy chain comprising it) can be no longer homodimerized with itself but forced to be heterodimerized with other CH3 regions that are complementarily engineered (such that the CH3 regions of the first and second Fc regions are heterodimerized and no homodimers are formed between the two first CH3 regions or the two second CH3 regions).

Preferably, based on the Knob-in-Hole technology, corresponding Knob and Hole mutations are introduced into the first Fc region and the second Fc region. See, e.g., US 5,731,168; US 7,695,936; Ridgway et al., Prot Eng 9, 617-621 (1996) and Carter, J Immunol Meth 248, 7-15 (2001) for the technology.

In a particular embodiment, in the CH3 region of one Fc region, the threonine residue at position 366 is substituted with a tryptophan residue (T366W) (knob mutation); in the CH3 region of the other Fc region, the tyrosine residue at position 407 is substituted with a valine residue (Y407V) (hole mutation). Optionally, the threonine residue at position 366 is substituted with a serine residue (T366S) and the tyrosine residue at position 407 is substituted with a valine residue (Y407V) (numbering according to the EU index).

In yet another embodiment, in the CH3 region of one Fc region, the threonine residue at position 366 is substituted with a tryptophan residue (T366W), and the serine residue at position 354 is substituted with a cysteine residue (S354C) or the glutamic acid residue at position 356 is substituted with a cysteine residue (E356C) (in particular, the serine residue at position 354 is substituted with a cysteine residue); in the CH3 region of the other Fc region, the tyrosine residue at position 407 is substituted with a valine residue (Y407V) (hole mutation). Optionally, the threonine residue at position 366 is substituted with a serine residue (T366S) and the leucine residue at position 368 is substituted with an alanine residue (L368A) (numbering according to the EU index), and optionally, the tyrosine residue at position 349 is substituted with a cysteine residue (Y349C) (numbering according to the EU index).

In a specific embodiment, one Fc region comprises an amino acid substitution T366W, and the other Fc region comprises amino acid substitutions T366S, L368A, and Y407V (numbering according to EU index).

In a specific embodiment, one Fc region comprises amino acid substitutions S354C and T366W, and the other Fc region comprises amino acid substitutions Y349C, T366S, L368A, and Y407V (numbering according to EU index).

In addition, based on the Innobody technology, corresponding mutations may be introduced in the first Fc region and the second Fc region. See, e.g., PCT/CN2021/143141 for the technology.

In a particular embodiment,
the first CH3 region comprises an S364R/K (preferably S364R) mutation, and optionally one or more additional mutations. In some embodiments, the second CH3 region comprises a K370S/T/A/V (preferably K370S) mutation, and optionally one or more additional mutations. In some embodiments, the first CH3 region comprises an S364R/K mutation, and the second CH3 region comprises a K370S/T/A/V mutation. In some embodiments, the first CH3 region comprises an S364R mutation, and the second CH3 region comprises a K370S mutation.

In some embodiments, the first CH3 region comprises an S364R/K (preferably S364R) mutation and a D399K/R (preferably D399K) mutation. In some embodiments, the second CH3 region comprises a K370S/T/A/V (preferably K370S) mutation and a K409D/E (preferably K409D) mutation. In some embodiments, the first CH3 region comprises S364R/K+D399K/R, and the second CH3 region comprises K370S/T/A/V+Y349T/S/A/V. In some embodiments, the first CH3 region comprises S364R +D399K, and the second CH3 region comprises K370S + Y349T. In some embodiments, the first CH3 region further comprises E375N/Q (preferably E375N) and/or T350V/A (preferably T350V). In some embodiments, the second CH3 region further comprises K409D/E (preferably K409D), Q347D/E (preferably Q347D) and/or T350V/A (preferably T350V).

In some embodiments, the first CH3 region comprises S364R + D399K, and the second CH3 region comprises K370S + Y349T + K409D. In some embodiments, the first CH3 region further comprises E357N. In some embodiments, the second CH3 region further comprises Q347D. In some embodiments, the first CH3 region further comprises E357N, and the second CH3 region further comprises Q347D. In some embodiments, the first CH3 region and the second CH3 region further each comprise T350V, or both comprise T350V.

Thus, in some embodiments, the first CH3 region comprises S364R + D399K, and the second CH3 region comprises K370S + Y349T + K409D + Q347D. In some embodiments, the first CH3 region comprises S364R + D399K + E357N, and the second CH3 region comprises K370S + Y349T + K409D + Q347D. In some embodiments, the first CH3 region comprises S364R + D399K + E357N + T350V, and the second CH3 region comprises K370S + Y349T + K409D + Q347D + T350V.

In some embodiments, the first CH3 region comprises K409E/D (preferably K409E). In some embodiments, the second CH3 region comprises D399K/R (preferably D399K) or K370T/S/A/V (preferably K370T). In some embodiments, the first CH3 region comprises K409E/D (preferably K409E), and the second CH3 region comprises D399K/R (preferably D399K). In some embodiments, the first CH3 region further comprises T411R/K (preferably T411R). In some embodiments, the second CH3 region further comprises K370T/S/A/V (preferably K370T). In some embodiments, the first CH3 region comprises K409E/D + T411R/K, and the second CH3 region comprises D399K/R + K370T/S/A/V. In some embodiments, the first CH3 region comprises K409E + T411R, and the second CH3 region comprises D399K + K370T.

In some specific embodiments, the first and second CH3 regions have the following combinations of mutations:

| The first CH3 region | The second CH3 region |
|---|---|
| S364R, D399K, E357N, T350V | K370S, Y349T, Q347D, K409D, T350V |
| K409E, T411R | D399K, K370T |
| S364R, D399K, E357N | K370S, Y349T, Q347D, K409D |
| S364R, D399K | K370S, Y349T, Q347D, K409D |
| S364R, D399K | K370S, Y349T, K409D |

In one embodiment, a CH3 of one Fc region comprises S364R and D399K mutations, and a CH3 of the other Fc region comprises Y349T, K370S, and K409D mutations.

Thus, in a specific embodiment, the multispecific antibody of the present disclosure comprises two Fc regions that are heterodimerized, wherein
a) one Fc region polypeptide comprises a mutation T366W, and the other Fc region polypeptide comprises mutations T366S, L368A, and Y407V, or
b) one Fc region polypeptide comprises mutations S354C and T366W, and the other Fc region polypeptide comprises mutations Y349C, T366S, L368A, and Y407V, or
c) one Fc region polypeptide comprises mutations S364R and D399K, and the other Fc-region polypeptide comprises mutations Y349T, K370S, and K409D.

Thus, in a specific embodiment, the multispecific antibody of the present disclosure comprises two Fc regions that are heterodimerized, wherein one Fc region polypeptide comprises or consists of an amino acid sequence set forth in SEQ ID NO: 49 or 50, and the other Fc region polypeptide comprises or consists of an amino acid sequence set forth in SEQ ID NO: 52 or 53.

Thus, in a specific embodiment, the multispecific antibody of the present disclosure comprises two Fc regions that are heterodimerized, wherein one Fc region polypeptide comprises an amino acid sequence having at least 85%, 90%, 95%, 96%, 97%, 98%, or 99% identity to an amino acid sequence set forth in SEQ ID NO: 49 or 50, and the other Fc region polypeptide comprises an amino acid sequence having at least 85%, 90%, 95%, 96%, 97%, 98%, or 99% identity to an amino acid sequence set forth in SEQ ID NO: 52 or 53.

Thus, in a specific embodiment, the multispecific antibody of the present disclosure comprises two Fc regions that are heterodimerized, wherein one Fc region polypeptide comprises an amino acid sequence having at least 85%, 90%, 95%, 96%, 97%, 98%, or 99% identity to an amino acid sequence set forth in SEQ ID NO: 49 or 50 and comprises mutations Y349T, K370S, and K409D, and the other Fc region comprises an amino acid sequence having at least 85%, 90%, 95%, 96%, 97%, 98%, or 99% identity to an amino acid sequence set forth in SEQ ID NO: 52 or 53 and comprises mutations S364R and D399K.

In some embodiments, the Fc region further comprises additional mutations that favor the purification of the heterodimer.

### ➢ Antibody modification

In one embodiment of the present disclosure, the amino acid change described herein includes amino acid replacement, insertion, or deletion. Preferably, the amino acid change described herein is an amino acid replacement, preferably a conservative replacement.

In a preferred embodiment, the amino acid change described herein occurs in a region outside the CDR (e.g., in FR). More preferably, the amino acid change described herein occurs in a region outside the heavy chain variable region and/or outside the light chain variable region.

In some embodiments, the replacement is a conservative replacement. A conservative replacement refers to the replacement of an amino acid by another amino acid of the same class, e.g., the replacement of an acidic amino acid by another acidic amino acid, the replacement of a basic amino acid by another basic amino acid, or the replacement of a neutral amino acid by another neutral amino acid. Exemplary replacements are shown in Table A below:

**Table A**

| Original residue | Exemplary replacement | Preferred replacement |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu, Val; Met; Ala; Phe; Nle | Leu |
| Leu (L) | Nle; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Nle | Leu |

In certain embodiments, the antibody provided herein is changed to increase or decrease the extent to which the antibody is glycosylated.

Antibodies with changed classes of glycosylation can be prepared, such as low-fucosylated antibodies with reduced amounts of fucosyl residues or antibodies with increased bisecting GlcNac structures. For example, fucosylation in the constant region (e.g., an Fc region) can be eliminated, e.g., to obtain low or no fucosylation.

Such changed glycosylation patterns have shown the ability to increase ADCC of antibodies. Such carbohydrate modifications can be achieved by, e.g., expressing antibodies in host cells with changed glycosylation systems. For example, afucosylated antibodies are expressed in host cells knocked out for α-1,6-fucosyltransferase 8 (Fut8) (WO2000061739). For example, low-fucosylated or afucosylated antibodies are obtained in host cells into which the gene encoding an enzyme RMD related to glycan chain modification has been introduced (GlymaxX technology, ProBioGen AG, see patent publication No.: WO2011035884A1). Alternatively, fucosidase may be used to cleave off fucose residues of the antibody; for example, fucosidase α-L-fucosidase removes fucosyl residues from the antibody (Tarentino et al. (1975) Biochem. 14: 5516-23).

### ➢ Exemplary bispecific antibody molecule

In some embodiments, the anti-B7-H3/EGFR bispecific antibody of the present disclosure has one or more of the following properties:
(1) in one aspect, the bispecific antibody of the present disclosure can block the binding of an EGFR ligand to EGFR, thereby inhibiting biological signaling and blocking the corresponding biological activity of a tumor; in another aspect, the antibody stimulates the endocytosis of EGFR and thus enables the final degradation of EGFR by intracellular lysosomes and the like;
(2) the bispecific antibody of the present disclosure adopts an EGFR antibody parent sequence with a low affinity for EGFR, thereby greatly reducing toxic and side effects of a series of EGFR monoclonal antibodies on normal epithelial tissues such as the skin;
(3) the bispecific antibody of the present disclosure incorporates an antibody parent sequence with a high affinity for B7-H3 on the basis of the low affinity for EGER, thereby greatly improving the blocking activity against EGFR signaling and improving the pharmacodynamic biological activity and pharmacodynamic safety window of the bispecific antibody of the present disclosure;
(4) the bispecific antibody of the present disclosure is a low-fucosylated antibody;
(5) the bispecific antibody of the present disclosure has relatively high pharmacodynamic biological activity and safety;
(6) the bispecific antibody of the present disclosure has excellent tumor killing and inhibiting effects;
(7) the bispecific antibody of the present disclosure has excellent *in-vivo* and *in-vitro* ADCC pharmacodynamic activity;
(8) the bispecific antibody of the present disclosure has an excellent anti-tumor effect, especially a synergistic effect, when used in combination with a KRAS small molecule inhibitor.

In some embodiments, in the antibody molecule of the present disclosure, the antigen-binding region specifically binding to EGFR is linked to a heavy chain constant region CH, for example, the heavy chain variable region therein is linked to the heavy chain constant region CH, for example, the C-terminus of the heavy chain variable region therein is linked to the N-terminus of the heavy chain constant region CH. In some embodiments, in the antibody molecule of the present disclosure, the antigen-binding region specifically binding to EGFR is linked to a light chain constant region, for example, the light chain variable region therein is linked to the light chain constant region CL, for example, the C-terminus of the light chain variable region therein is linked to the N-terminus of the light chain constant region CL. In some embodiments, in the antibody molecule of the present disclosure, in the antigen-binding region specifically binding to EGFR, the heavy chain variable region is linked to a heavy chain constant region CH and the light chain variable region is linked to a light chain constant region CL.

In some embodiments, in the antibody molecule of the present disclosure, the antigen-binding region specifically binding to B7-H3 is linked to a heavy chain constant region CH, for example, the heavy chain variable region therein is linked to the heavy chain constant region CH, for example, the C-terminus of the heavy chain variable region therein is linked to the N-terminus of the heavy chain constant region CH. In some embodiments, in the antibody molecule of the present disclosure, the antigen-binding region specifically binding to B7-H3 is linked to a light chain constant region, for example, the light chain variable region therein is linked to the light chain constant region CL, for example, the C-terminus of the light chain variable region therein is linked to the N-terminus of the light chain constant region CL. In some embodiments, in the antibody molecule of the present disclosure, in the antigen-binding region specifically binding to B7-H3, the heavy chain variable region is linked to a heavy chain constant region CH and the light chain variable region is linked to a light chain constant region CL.

In some embodiments, the heavy chain constant region comprises a CH1 and an Fc region, which are linked via or not via a hinge region.

In some embodiments, the heavy chain constant region is a heavy chain constant region of IgG1, IgG2, IgG3, or IgG4, e.g., human IgG1, human IgG2, human IgG3, or human IgG4. In some embodiments, the CH1 comprises or consists of an amino acid sequence set forth in SEQ ID NO: 42 or an amino acid sequence having at least 80%, 85%, 90%, 91%, 92%, 93%, 94%, 95%, 96%, 97%, 98%, or 99% identity to the amino acid sequence of SEQ ID NO: 42.

In some embodiments, the light chain constant region is a kappa light chain constant region or a lambda light chain constant region, e.g., a human kappa or human lambda light chain constant region. In some embodiments, the light chain constant region comprises or consists of an amino acid sequence set forth in SEQ ID NO: 54 or an amino acid sequence having at least 80%, 85%, 90%, 91%, 92%, 93%, 94%, 95%, 96%, 97%, 98%, or 99% identity to the amino acid sequence of SEQ ID NO: 54.

In some preferred embodiments, the present disclosure provides a bispecific antibody molecule, which comprises a Fab fragment specifically binding to EGFR, a Fab fragment specifically binding to B7-H3, and an Fc dimer, wherein the Fab fragment specifically binding to EGFR forms a half antibody specifically binding to EGFR with one Fc, and the Fab fragment specifically binding to B7-H3 forms a half antibody specifically binding to B7-H3 with one Fc.

In some embodiments, the bispecific antibody is an IgG-like antibody having a configuration shown in FIG. 1.

In some embodiments, the bispecific antibody comprises a heavy chain 1 and a light chain 1, and a heavy chain 2 and a light chain 2, wherein the heavy chain 1 and the light chain 1 constitute a first half antibody, and the heavy chain 2 and the light chain 2 constitute a second half antibody; wherein the heavy chain 1 comprises the heavy chain variable region of the first antigen-binding region and a first heavy chain constant region; the light chain 1 comprises the light chain variable region of the first antigen-binding region and a first light chain constant region; and the heavy chain 2 comprises the heavy chain variable region of the second antigen-binding region and a second heavy chain constant region; the light chain 2 comprises the light chain variable region of the second antigen-binding region and a second light chain constant region.

In some embodiments, in the bispecific antibody, the heavy chain 1 comprises or consists of an amino acid sequence set forth in SEQ ID NO: 33 or an amino acid sequence having at least 80%, 85%, 90%, 91%, 92%, 93%, 94%, 95%, 96%, 97%, 98%, or 99% identity to the amino acid sequence of SEQ ID NO: 33.

In some embodiments, in the bispecific antibody, the light chain 1 comprises or consists of an amino acid sequence set forth in SEQ ID NO: 34 or an amino acid sequence having at least 90%, 91%, 92%, 93%, 94%, 95%, 96%, 97%, 98%, or 99% identity to the amino acid sequence of SEQ ID NO: 34.

In some embodiments, in the bispecific antibody, the heavy chain 1 comprises or consists of an amino acid sequence set forth in SEQ ID NO: 33 or an amino acid sequence having at least 90%, 91%, 92%, 93%, 94%, 95%, 96%, 97%, 98%, or 99% identity to the amino acid sequence of SEQ ID NO: 33, and the light chain 1 comprises or consists of an amino acid sequence set forth in SEQ ID NO: 34 or an amino acid sequence having at least 90%, 91%, 92%, 93%, 94%, 95%, 96%, 97%, 98%, or 99% identity to the amino acid sequence of SEQ ID NO: 34.

In some embodiments, in the bispecific antibody, the heavy chain 2 comprises or consists of an amino acid sequence set forth in SEQ ID NO: 35, 37, or 39 or an amino acid sequence having at least 90%, 91%, 92%, 93%, 94%, 95%, 96%, 97%, 98%, or 99% identity to an amino acid sequence set forth in SEQ ID NO: 35, 37, or 39.

In some embodiments, in the bispecific antibody, the light chain 2 comprises or consists of an amino acid sequence set forth in SEQ ID NO: 36, 38, or 40 or an amino acid sequence having at least 90%, 91%, 92%, 93%, 94%, 95%, 96%, 97%, 98%, or 99% identity to the amino acid sequence set forth in SEQ ID NO: 36, 38, or 40.

In some embodiments, in the bispecific antibody,
(1) the heavy chain 2 comprises or consists of an amino acid sequence set forth in SEQ ID NO: 35 or an amino acid sequence having at least 90%, 91%, 92%, 93%, 94%, 95%, 96%, 97%, 98%, or 99% identity to the amino acid sequence set forth in SEQ ID NO: 35; and the light chain 2 comprises or consists of an amino acid sequence set forth in SEQ ID NO: 36 or an amino acid sequence having at least 90%, 91%, 92%, 93%, 94%, 95%, 96%, 97%, 98%, or 99% identity to the amino acid sequence set forth in SEQ ID NO: 36;
(2) the heavy chain 2 comprises or consists of an amino acid sequence set forth in SEQ ID NO: 37 or an amino acid sequence having at least 90%, 91%, 92%, 93%, 94%, 95%, 96%, 97%, 98%, or 99% identity to the amino acid sequence set forth in SEQ ID NO: 37; and the light chain 2 comprises or consists of an amino acid sequence set forth in SEQ ID NO: 38 or an amino acid sequence having at least 90%, 91%, 92%, 93%, 94%, 95%, 96%, 97%, 98%, or 99% identity to the amino acid sequence set forth in SEQ ID NO: 38;
(3) the heavy chain 2 comprises or consists of an amino acid sequence set forth in SEQ ID NO: 39 or an amino acid sequence having at least 90%, 91%, 92%, 93%, 94%, 95%, 96%, 97%, 98%, or 99% identity to the amino acid sequence set forth in SEQ ID NO: 39; and the light chain 2 comprises or consists of an amino acid sequence set forth in SEQ ID NO: 40 or an amino acid sequence having at least 90%, 91%, 92%, 93%, 94%, 95%, 96%, 97%, 98%, or 99% identity to the amino acid sequence set forth in SEQ ID NO: 40.

In some embodiments, in the bispecific antibody,
the heavy chain 1 comprises or consists of an amino acid sequence set forth in SEQ ID NO: 33 or an amino acid sequence having at least 90%, 91%, 92%, 93%, 94%, 95%, 96%, 97%, 98%, or 99% identity to the amino acid sequence of SEQ ID NO: 33, and the light chain 1 comprises or consists of an amino acid sequence set forth in SEQ ID NO: 34 or an amino acid sequence having at least 90%, 91%, 92%, 93%, 94%, 95%, 96%, 97%, 98%, or 99% identity to the amino acid sequence of SEQ ID NO: 34; and the heavy chain 2 and the light chain 2 comprise or consist of, respectively, amino acid sequences set forth in the following SEQ ID NOs or amino acid sequences having at least 85%, 90%, 95%, 96%, 97%, 98%, or 99% identity thereto:
i) SEQ ID NO: 35 and SEQ ID NO: 36;
ii) SEQ ID NO: 37 and SEQ ID NO: 38;
iii) SEQ ID NO: 39 and SEQ ID NO: 40.

In some embodiments, in the bispecific antibody,
(i) the heavy chain 1 comprises an amino acid sequence set forth in SEQ ID NO: 33, and the light chain 1 comprises an amino acid sequence set forth in SEQ ID NO: 34, and
   the heavy chain 2 comprises an amino acid sequence set forth in SEQ ID NO: 35, and the light chain 2 comprises an amino acid sequence set forth in SEQ ID NO: 36; or,
(ii) the heavy chain 1 comprises an amino acid sequence set forth in SEQ ID NO: 33, and the light chain 1 comprises an amino acid sequence set forth in SEQ ID NO: 34, and
   the heavy chain 2 comprises an amino acid sequence set forth in SEQ ID NO: 37, and the light chain 2 comprises an amino acid sequence set forth in SEQ ID NO: 38; or,
(iii) the heavy chain 1 comprises an amino acid sequence set forth in SEQ ID NO: 33, and the light chain 1 comprises an amino acid sequence set forth in SEQ ID NO: 34, and
the heavy chain 2 comprises an amino acid sequence set forth in SEQ ID NO: 39, and the light chain 2 comprises an amino acid sequence set forth in SEQ ID NO: 40.

### III. Immunoconjugate

In some embodiments, the present disclosure further encompasses an antibody conjugated to an additional substance. Thus, the present disclosure relates to an immunoconjugate, which comprises an antibody conjugated to an additional substance ("immunoconjugate"). In some embodiments, the additional substance is, e.g., a therapeutic agent or a label, such as a cytotoxic agent, an immunomodulatory agent (e.g., an immunoagonist), or a chemotherapeutic agent. The cytotoxic agent includes any agent that is harmful to cells. Examples of the cytotoxic agent (e.g., chemotherapeutic agent) suitable for forming the immunoconjugate are known in the art. For example, the cytotoxic agent includes, but is not limited to: radioisotopes; growth inhibitors; enzymes and fragments thereof such as nucleases; antibiotics; toxins such as small molecule toxins or enzymatically active toxins of bacterial, fungal, plant, or animal sources, including fragments and/or variants thereof; and a variety of known anti-tumor or anti-cancer agents.

Additionally, the antibody molecule of the present disclosure can be conjugated to a labeled sequence (such as a peptide) to facilitate purification.

In other embodiments, the antibody molecule of the present disclosure is conjugated to a diagnostic agent or a detectable agent. Such antibodies can be used as a part of clinical test methods (e.g., to determine the effect of a particular therapy), for monitoring or predicting the onset, development, progression, and/or severity of a disease or a condition. Such diagnosis and detection can be achieved by coupling the antibody to a detectable substance, which includes, but is not limited to, a variety of enzymes, prosthetic groups, fluorescent substances, luminescent substances, radioactive substances, and positron-emitting metal and non-radioactive paramagnetic metal ions used in various positron emission imaging techniques.

Additionally, the antibody molecule of the present disclosure can be conjugated to a therapeutic moiety or a drug moiety that modulates a given biological response. The therapeutic moiety or the drug moiety includes, but is not limited to, classical chemotherapeutic drugs. For example, the drug moiety may be a protein, a peptide or a polypeptide possessing the desired biological activity. Additionally, the antibody molecule of the present disclosure can be conjugated to a therapeutic moiety such as a radioactive metal ion.

The antibody can also be attached to a solid phase support, which is particularly useful for immunoassays or purification of target antigens.

In some embodiments, the immunoconjugate is for use in the prevention or treatment of a disease, such as acute and chronic inflammatory diseases, infection (e.g., chronic infection), and a tumor. For example, the disease is a tumor (e.g., cancer) or an infection. In some embodiments, the tumor is a tumor immune escape. Preferably, the tumor is a cancer of epithelial origin, e.g., a gastrointestinal tumor, a lung tumor, or a skin tumor, e.g., a skin cancer (e.g., cutaneous squamous cell carcinoma, or head and neck cancer such as squamous cell carcinoma of the head and neck), an esophageal cancer (e.g., esophageal squamous cell carcinoma), an intestinal cancer (e.g., colon cancer, rectal cancer, or colorectal cancer), or a lung cancer (e.g., non-small cell lung cancer, lung squamous cell carcinoma, or lung adenocarcinoma). In some embodiments, the infection is a chronic infection. In some embodiments, the infection is, e.g., a bacterial infection, a viral infection, a fungal infection, a protozoal infection, and the like.

### IV. Nucleic acid of the disclosure and host cell comprising same

In one aspect, the present disclosure provides a nucleic acid encoding any one of the above antibodies or the fragments thereof or any one of the chains thereof. In one embodiment, provided is a vector comprising the nucleic acid. In one embodiment, the vector is an expression vector. In one embodiment, provided is a host cell comprising the nucleic acid or the vector. In one embodiment, the host cell is eukaryotic. In another embodiment, the host cell is selected from yeast cells, mammalian cells (e.g., CHO cells or 293 cells), or other cells suitable for preparing an antibody or an antigen-binding fragment thereof. In another embodiment, the host cell is prokaryotic.

For example, the nucleic acid of the present disclosure comprises a nucleic acid encoding an amino acid sequence selected from any one of SEQ ID NOs: 3-8 and 33-40, or an amino acid sequence having at least 85%, 90%, 91%, 92%, 93%, 94%, 95%, 96%, 97%, 98%, or 99% identity to the amino acid sequence selected from any one of SEQ ID NOs: 3-8 and 33-40.

As will be understood by those skilled in the art, each antibody or polypeptide amino acid sequence can be encoded by a variety of nucleic acid sequences because of codon degeneracy. Nucleic acid sequences encoding the molecules of the present disclosure can be produced using methods well known in the art, for example by *de novo* solid phase DNA synthesis, or by PCR amplification.

In one aspect, the present disclosure provides a nucleic acid encoding any of the above antibodies or any of the antibody chains. When expressed from a suitable expression vector, the polypeptide encoded by the nucleic acid can exhibit human EGFR and/or B7-H3 antigen-binding ability.

In a further aspect, the present disclosure provides a nucleic acid encoding any of the above bispecific antibodies. When expressed from a suitable expression vector, the polypeptide encoded by the nucleic acid can exhibit human EGFR and/or B7-H3 antigen-binding ability. In one embodiment, the nucleic acids encoding the chains of the bispecific antibody can be in the same vector or in different vectors. In another embodiment, the nucleic acids encoding the chains of the bispecific antibody can be introduced into the same or different host cells for expression. Thus, in some embodiments, the method for producing the bispecific antibody of the present disclosure comprises the step of: culturing a host cell comprising nucleic acids encoding chains of the molecule under conditions suitable for the expression of the chains to produce the bispecific antibody of the present disclosure.

In one embodiment, provided are one or more vectors comprising the nucleic acids. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the vector is, e.g., a pcDNA vector, e.g., pcDNA3.1.

Once the expression vector or DNA sequence has been prepared for expression, the expression vector can be transfected or introduced into suitable host cells. Various techniques can be used for this purpose, e.g., protoplast fusion, calcium phosphate precipitation, electroporation, retroviral transduction, viral transfection, biolistics, lipid-based transfection, or other conventional techniques. In the case of protoplast fusion, cells are cultured in a culture medium and screened for appropriate activity. Methods and conditions for culturing the resulting transfected cells and for recovering the resulting antibody molecules are known to those skilled in the art and may be changed or optimized according to the particular expression vector and the particular mammalian host cell used based on the present description and methods known in the prior art.

Additionally, cells having stably incorporated DNA in chromosomes thereof can be selected by introducing one or more markers permitting the selection of transfected host cells.

In one embodiment, provided is a host cell comprising one or more polynucleotides of the present disclosure. In some embodiments, provided is a host cell comprising the expression vector of the present disclosure. In one embodiment, the host cell is eukaryotic. In another embodiment, the host cell is selected from yeast cells, mammalian cells, e.g., CHO cells (e.g., CHO-S, such as ExpiCHO-S) or 293 cells (e.g., a 293F cell or an HEK293 cell), or other cells suitable for preparing an antibody or a fragment thereof. In one embodiment, the host cell is prokaryotic, e.g., a bacterium, e.g., *E.coli.*

Suitable host cells include prokaryotic microorganisms, such as *E. coli.* The host cells may also be eukaryotic microorganisms such as filamentous fungi or yeast, or various eukaryotic cells such as insect cells. Vertebrate cells may also be used as hosts. For example, a mammalian cell line engineered to be suitable for suspension growth may be used. Examples of useful mammalian host cell lines include SV40-transformed monkey kidney CV1 line (COS-7); human embryonic kidney line (HEK293 or 293F cells), 293 cells, baby hamster kidney cells (BHK), monkey kidney cells (CV1), African green monkey kidney cells (VERO-76), human cervical cancer cells (HELA), canine kidney cells (MDCK), buffalo rat liver cells (BRL 3A), human lung cells (W138), human liver cells (Hep G2), Chinese hamster ovary cells (CHO cells), CHOK1SV cells, CHOK1SV GS-KO cells, CHOS cells, NSO cells, myeloma cell line such as Y0, NS0, P3X63, and Sp2/0, and the like. For reviews of mammalian host cell lines suitable for protein production, see, e.g., Yazaki and Wu, Methods in Molecular Biology, vol. 248 (edited by B. K. C. Lo, Humana Press, Totowa, NJ), pp. 255-268 (2003). In a preferred embodiment, the host cell is a CHO cell, e.g., a CHOS cell CHOK1SV cell or CHOK1SV GS-KO, or the host cell is a 293 cell, e.g., a HEK293 cell. In some preferred embodiments, the host cell is a CHO cell. In one embodiment, the host cell of the present disclosure is a glycosylation-engineered host cell, preferably a glycosylation-engineered CHO cell. In one embodiment, the host cell is engineered to express an RMD enzyme. In one embodiment, the host cell comprises a nucleic acid encoding an RMD enzyme. In one embodiment, the RMD enzyme comprises or consists of an amino acid sequence set forth in SEQ ID NO: 41, or an amino acid sequence having at least 90%, 91%, 92%, 93%, 94%, 95%, 96%, 97%, 98%, or 99% identity thereto; preferably, the RMD enzyme is derived from *Pseudomonas aeruginosa.*

In one embodiment, the host cell of the present disclosure comprises a nucleic acid encoding one or more or all of chains of the antibody molecule of the disclosure and a nucleic acid encoding an RMD enzyme.

### V. Production and purification of the antibody molecule of the present disclosure

In one embodiment, the present disclosure provides a method for preparing the antibody molecule of the present disclosure, wherein the method comprises culturing the host cell under conditions suitable for expressing the nucleic acid encoding the antibody molecule of the present disclosure, and optionally isolating the antibody. In a certain embodiment, the method further comprises recovering the antibody molecule of the present disclosure from the host cell.

In one embodiment, provided is a method for preparing the antibody molecule of the present disclosure, wherein the method comprises culturing the host cell comprising a nucleic acid encoding the antibody (e.g., any one and/or more polypeptide chains) or an expression vector comprising the nucleic acid, as provided above, under conditions suitable for expressing antibodies, and optionally recovering the antibody from the host cell (or the host cell medium).

For recombinant production of the antibody molecule of the present disclosure, a nucleic acid encoding the antibody (e.g., the antibody described above, e.g., any one and/or more polypeptide chains) is isolated and inserted into one or more vectors for further cloning and/or expressing in the host cell. Such nucleic acids can be easily isolated and sequenced by using conventional procedures (e.g., by using oligonucleotide probes that are capable of specifically binding to genes encoding heavy and light chains of antibodies).

The antibody molecule prepared as described herein can be purified by known prior art such as high-performance liquid chromatography, ion exchange chromatography, gel electrophoresis, affinity chromatography, and size exclusion chromatography. The actual conditions used to purify a particular protein also depend on factors such as net charge, hydrophobicity, and hydrophilicity, and these will be apparent to those skilled in the art. The purity of the antibody molecule of the present disclosure can be determined by any one of a variety of well-known analytical methods including size exclusion chromatography, gel electrophoresis, high-performance liquid chromatography, and the like.

### VI. Assay

The antibody molecule provided herein can be identified, screened, or characterized for physical/chemical properties and/or biological activities thereof through a variety of assays known in the art. In one aspect, the antibody of the present disclosure is tested for the antigen-binding activity, for example, by known methods such as ELISA and Western blotting. The binding to the bound antigen can be determined by methods known in the art, and exemplary methods are disclosed herein, such as bio-layer interferometry and SPR.

The present disclosure also provides an assay for identifying antibodies having biological activities. Biological activities may include, for example, binding to an antigen, binding to an antigen on the cell surface, inhibitory or activating effects on an antigen, and the like. Also provided are antibodies having such biological activities *in vivo* and/or *in vitro.*

In certain embodiments, the antibody of the present disclosure is characterized for such biological activities.

The present disclosure also provides a method for identifying properties of antibodies, e.g., druggability-related properties. Such druggability-related properties include, for example, thermal stability (e.g., long-term thermal stability).

Cells for use in any of the *in-vitro* assays described above include cell lines that naturally express antigens or are engineered to express antigens. Such cells also include cell lines that express antigens and cell lines transfected with DNA encoding antigens that do not normally express antigens.

It can be understood that any of the above assays can be performed by using the immunoconjugate of the present disclosure in place of or in addition to the antibody molecule of the present disclosure.

It can also be understood that any of the above assays can be performed on the antibody molecule of the present disclosure and other active agents.

In some embodiments, the antigen is EGFR (e.g., human EGFR) and/or B7-H3 (e.g., human B7-H3).

### VII. Pharmaceutical composition and pharmaceutical formulation

In some embodiments, the present disclosure provides a composition comprising any of the antibody molecules or the fragments (preferably the antigen-binding fragments) thereof, or the immunoconjugates thereof described herein; preferably, the composition is a pharmaceutical composition. In one embodiment, the composition further comprises a pharmaceutical supplementary material. In one embodiment, the composition, e.g., the pharmaceutical composition, comprises the antibody molecule or the fragment thereof, or the immunoconjugate thereof of the present disclosure, and a combination of one or more additional therapeutic agents.

In some embodiments, the additional therapeutic agent is selected from an anti-angiogenic agent, a chemotherapeutic agent, an additional antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is a small molecule drug, and preferably, the small molecule drug is selected from a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.

In some embodiments, the composition is for use in the prevention or treatment of a disease, such as acute and chronic inflammatory diseases, infection (e.g., chronic infection), and a tumor. For example, the disease is a tumor (e.g., cancer) or an infection. In some embodiments, the tumor is a tumor immune escape. Preferably, the tumor is a cancer of epithelial origin, e.g., a gastrointestinal tumor, a lung tumor, or a skin tumor, e.g., a skin cancer (e.g., cutaneous squamous cell carcinoma, or head and neck cancer such as squamous cell carcinoma of the head and neck), an esophageal cancer (e.g., esophageal squamous cell carcinoma), an intestinal cancer (e.g., colon cancer, rectal cancer, or colorectal cancer), or a lung cancer (e.g., non-small cell lung cancer, lung squamous cell carcinoma, or lung adenocarcinoma). In some embodiments, the infection is a chronic infection. In some embodiments, the infection is, e.g., a bacterial infection, a viral infection, a fungal infection, a protozoal infection, and the like.

The present disclosure also includes a composition (including a pharmaceutical composition or a pharmaceutical formulation) comprising the antibody or the immunoconjugate thereof of the present disclosure, and/or a composition (including a pharmaceutical composition or a pharmaceutical formulation) comprising a polynucleotide encoding the antibody of the present disclosure. In certain embodiments, the composition comprises one or more of the antibodies or the fragments thereof of the present disclosure, or one or more polynucleotides encoding one or more of the antibodies or the fragments thereof of the present disclosure.

Such compositions can further comprise a suitable pharmaceutical supplementary material, such as a pharmaceutical carrier and a pharmaceutical excipient known in the art, including buffers.

As used herein, the "pharmaceutical carrier" includes any and all solvents, dispersion media, isotonic agents and absorption delaying agents, and the like that are physiologically compatible. The pharmaceutical carrier suitable for use in the present disclosure may be sterile liquid, such as water and oil, including those of petroleum, animal, plant, or synthetic origin, such as peanut oil, soybean oil, mineral oil, or sesame oil. Water is a preferred carrier when the pharmaceutical composition is administered intravenously. Saline solutions, aqueous dextrose, and glycerol solutions can also be used as liquid carriers, particularly for injectable solutions.

Suitable excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. For use and application of excipients, see also "Handbook of Pharmaceutical Excipients", 5th edition., R. C. Rowe, P. J. Seskey and S. C. Owen, Pharmaceutical Press, London, Chicago.

The composition may further comprise a small amount of wetting agent, emulsifier, or pH buffer, if desired. Such compositions may be in the form of a solution, a suspension, an emulsion, a tablet, a pill, a capsule, a powder, a sustained release formulation, or the like. Oral formulations may comprise a standard pharmaceutical carrier and/or excipient such as pharmaceutical-grade mannitol, lactose, starch, magnesium stearate, and saccharin.

The composition of the present disclosure may be in a variety of forms. Such forms include, for example, liquid, semi-solid and solid dosage forms, such as liquid solutions (e.g., injectable solutions and infusible solutions), dispersions or suspensions, liposomes, and suppositories. The preferred form depends on the intended mode of administration and therapeutic use. Commonly preferred compositions are in the form of injectable solutions or infusible solutions. The preferred mode of administration is parenteral (e.g., intravenous, subcutaneous, intraperitoneal (i.p.) and intramuscular) injection. In one preferred embodiment, the antibody molecule is administered by intravenous infusion or injection. In another preferred embodiment, the antibody molecule is administered by intramuscular, intraperitoneal or subcutaneous injection.

The pharmaceutical formulation, preferably in the form of a lyophilized formulation or an aqueous solution, comprising the antibody described herein can be prepared by mixing the antibody of the present disclosure of desired purity with one or more optional pharmaceutical supplementary materials ("Remington's Pharmaceutical Sciences", 16th Ed., Osol, A. ed. (1980)).

The pharmaceutical composition or formulation of the present disclosure may further comprise more than one active ingredient required by a particular indication treated, preferably those having complementarity activity without adversely affecting one another. For example, it is desirable to further provide an additional therapeutic agent. In some embodiments, the additional therapeutic agent is selected from an anti-angiogenic agent, a chemotherapeutic agent, an additional antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is a small molecule drug, and preferably, the small molecule drug is selected from a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.

A sustained release formulation can be prepared. Suitable examples of the sustained release formulation include a semipermeable matrix of a solid hydrophobic polymer containing an antibody. The matrix is in the form of a shaped article, such as a film or a microcapsule.

The pharmaceutical composition of the present disclosure is suitable for intravenous, intramuscular, subcutaneous, parenteral, rectal, spinal or epidermal administration (e.g., by injection or infusion).

Therapeutic compositions generally should be sterile and stable under the conditions of manufacture and storage. The compositions can be prepared into solutions, microemulsions, dispersions, liposomes, or lyophilized forms. Sterile injectable solutions can be prepared by adding a required amount of an active compound (i.e., antibody molecule) to a suitable solvent, and then filtering and disinfecting the resulting mixture. Generally, dispersions are prepared by incorporating the active compound into a sterile vehicle, which comprises a basic dispersion medium and other ingredients. Coating agents such as lecithin can be used. In the case of dispersions, the proper fluidity of a solution can be maintained by using a surfactant. Prolonged absorption of injectable compositions can be caused by including in the compositions a substance that delays absorption such as monostearate and gelatin.

A kit comprising the antibody molecule described herein is also within the scope of the present disclosure. The kit may include one or more other elements, including, for example, package insert; other reagents, such as a marker or a reagent for coupling; a pharmaceutical carrier; and a device or other materials for administration to a subject.

### VIII. Combination product or kit

In some embodiments, the present disclosure also provides a combination product, which comprises the antibody or the antigen-binding fragment thereof, or the immunoconjugate thereof of the present disclosure, and one or more additional therapeutic agents. In some embodiments, the additional therapeutic agent is selected from an anti-angiogenic agent, a chemotherapeutic agent, an additional antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is a small molecule drug, and preferably, the small molecule drug is selected from a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.

In some embodiments, the combination product is for use in the prevention or treatment of a disease, such as acute and chronic inflammatory diseases, an infection (e.g., chronic infection), and a tumor. For example, the disease is a tumor (e.g., cancer) or an infection. In some embodiments, the tumor is a tumor immune escape. Preferably, the tumor is a cancer of epithelial origin, e.g., a gastrointestinal tumor, a lung tumor, or a skin tumor, e.g., a skin cancer (e.g., cutaneous squamous cell carcinoma, or head and neck cancer such as squamous cell carcinoma of the head and neck), an esophageal cancer (e.g., esophageal squamous cell carcinoma), an intestinal cancer (e.g., colon cancer, rectal cancer, or colorectal cancer), or a lung cancer (e.g., non-small cell lung cancer, lung squamous cell carcinoma, or lung adenocarcinoma). In some embodiments, the infection is a chronic infection. In some embodiments, the infection is, e.g., a bacterial infection, a viral infection, a fungal infection, a protozoal infection, and the like.

In some embodiments, two or more ingredients in the combination product may be administered to a subject in combination sequentially, separately, or simultaneously.

In some embodiments, the present disclosure also provides a kit, which comprises the antibody, the pharmaceutical composition, the immunoconjugate or the combination product of the present disclosure, and optionally a package insert directing administration.

In some embodiments, the present disclosure also provides a pharmaceutical product, which comprises the antibody, the pharmaceutical composition, the immunoconjugate or the combination product of the present disclosure, and optionally a package insert directing administration.

### IX. Use of the antibody molecule of the present disclosure

In another aspect, the present disclosure relates to a method for preventing or treating a tumor (e.g., cancer) in a subject, wherein the method comprises administering to the subject an effective amount of the antibody molecule, the pharmaceutical composition, the immunoconjugate, the combination product or the kit of the present disclosure. In some embodiments, the tumor in the subject of the present disclosure includes solid tumors and hematological tumors. In some embodiments, the tumor is a tumor immune escape. In some embodiments, the tumor is cancer.

In some embodiments, compared with normal cells of the same tissue or an adjacent normal tissue in the same subject, or compared with normal cells of the same tissue in a healthy subject, tumor cells of the tumor have one or more of the following characteristics:
(i) overexpressing wild-type EGFR (e.g., wild-type EGFR with an increased nucleic acid or protein level) and/or expressing mutant EGFR, e.g., mutant EGFR comprising mutations listed in Passaro A, et al., Nat Cancer. 2021, wherein preferably, the mutant EGFR comprises one or more mutations selected from R521K, L858R, T790M, G719X, C797S, Y1069C, Exon19 deletion (Del19), Exon20ins (e.g., S768_D770dup), and preferably, the mutant EGFR comprises R521K/Y1069C, R521K, L858R/T790M/C797S, Del19/T790M/C797S or S768_D770dup, compared with normal cells of an adjacent tissue or compared with normal cells of the same tissue in a healthy subject;
(ii) overexpressing wild-type KRAS (e.g., wild-type KRAS with an increased nucleic acid or protein level) or expressing mutant KRAS compared with normal cells of an adjacent tissue or compared with normal cells of the same tissue in a healthy subject, wherein preferably, the mutant KRAS comprises a mutation at position G12 or G13, e.g., G12D or G12C;
(iii) having B7-H3 with an increased nucleic acid or protein level compared with normal cells of an adjacent tissue or compared with normal cells of the same tissue in a healthy subject; and/or
(iv) the tumor cells being resistant to a tyrosine kinase inhibitor, e.g., to the first-generation (erlotinib) and the third-generation (osimertinib), e.g., to osimertinib.

In another aspect, the present disclosure relates to a method for preventing or treating an infectious disease in a subject, wherein the method comprises administering to the subject an effective amount of the antibody molecule, the pharmaceutical composition, the immunoconjugate, the combination product or the kit of the present disclosure. In one embodiment, the infectious disease is a chronic infection.

The subject may be a mammal, e.g., a primate, preferably a higher primate, e.g., a human (e.g., a patient suffering from or at risk of suffering from the disease described herein). In one embodiment, the subject suffers from or is at risk of suffering from the disease described herein (e.g., the tumor, infection or autoimmune disease described herein). In certain embodiments, the subject is receiving or has received other therapies, e.g., chemotherapy and/or radiotherapy.

In other aspects, the present disclosure provides use of the antibody molecule or the fragment thereof, the immunoconjugate thereof, the combination product or the kit in the manufacture or preparation of a drug for treating the related diseases or conditions mentioned herein.

In some embodiments, the antibody or the fragment thereof, the immunoconjugate, the composition, the combination product or the kit of the present disclosure delays the onset of the conditions and/or symptoms related to the conditions.

In some embodiments, the antibody, the pharmaceutical composition, the immunoconjugate, the combination product or the kits of the present disclosure can also be administered in combination with one or more additional therapies, such as therapeutic modalities and/or additional therapeutic agents, for the prevention and/or treatment described herein.

In some embodiments, the therapeutic modality includes surgery (e.g., tumor resection) or radiotherapy.

In some embodiments, the therapeutic agent is selected from an anti-angiogenic agent, a chemotherapeutic agent, an additional antibody, a cytotoxic agent, a vaccine, an anti-infective active agent, a small molecule drug, or an immunomodulatory agent.

In some embodiments, the small molecule drug is selected from a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.

The immunomodulatory agents include an inhibitor of an immune checkpoint molecule and an activator of a co-stimulatory molecule.

In some further embodiments, the antibody or the fragment thereof of the present disclosure is used in combination with a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.

In some embodiments, the antibody or the fragment thereof of the present disclosure can be administered in combination with a therapy comprising adoptive transfer of T cells (e.g., cytotoxic T lymphocytes or CTLs) expressing a chimeric antigen receptor (CAR).

In some embodiments, the antibody or the fragment thereof of the present disclosure can be administered in combination with an anti-tumor agent.

In some embodiments, the antibody or the fragment thereof of the present disclosure can be administered in combination with a cytokine. The cytokine can be administered as a fusion molecule with the antibody molecule of the present disclosure, or as a separate composition. In one embodiment, the antibody of the present disclosure is administered in combination with one, two, three or more cytokines (e.g., as a fusion molecule or as a separate composition).

In some embodiments, the antibody or the fragment thereof of the present disclosure can be combined with cancer therapies conventional in the art, including, but not limited to: (i) radiotherapies; (ii) chemotherapies, or the application of cytotoxic drugs, which generally affect rapidly dividing cells; (iii) targeted therapies, or agents that specifically affect the deregulation of cancer cell proteins; (iv) immunotherapy, or the enhancement of immune responses in a host (e.g., vaccine); (v) hormonal therapies, or the blocking of hormones (e.g., when a tumor is hormone sensitive), (vi) angiogenesis inhibitors, or the blocking of angiogenesis and growth, and (vii) palliative care.

In some embodiments, the antibody or the fragment thereof of the present disclosure can be combined with conventional methods for enhancing an immune function in a host.

### The various combination therapies described above can be further combined for treatment.

Such combination therapies encompass both combined administration (wherein two or more therapeutic agents are contained in the same formulation or separate formulations), and separate administrations (wherein the antibody of the present disclosure can be administered prior to, concurrently with, and/or subsequent to the administration of additional therapies, e.g., therapeutic modalities or therapeutic agents). The antibody molecule and/or additional therapies, e.g., therapeutic agents or therapeutic modalities, can be administered when a disease is active or when the disease is in remission or less active. The antibody molecule can be administered prior to, concurrently with, subsequent to other therapies, or during remission of a disease.

The antibody (the pharmaceutical composition or the immunoconjugate comprising the same, and any additional therapeutic agent) of the present disclosure can be administered by any suitable method, including parenteral administration, intrapulmonary administration, intranasal administration, and, if required by locoregional treatment, intralesional administration. Parenteral infusion includes intramuscular, intravenous, intra-arterial, intraperitoneal, or subcutaneous administration. The administration may be performed by any suitable route, such as injection, e.g., intravenous or subcutaneous injection, to some extent depending on short-term or long-term administration. Various administration schedules are encompassed herein, including, but not limited to, single administration or multiple administrations at multiple time points, bolus injection, and pulse infusion.

In order to prevent or treat diseases, the appropriate dosage of the antibody of the present disclosure (when used alone or in combination with one or more additional therapeutic agents) will depend on types of diseases to be treated, types of antibodies, severity, and progression of the disease, purpose of administration (prophylactic or therapeutic) of the antibody, previous treatments, clinical histories of patients, responses to the antibody, and the discretion of an attending physician. The antibody is suitably administered to a patient through a single treatment or through a series of treatments.

The dosage and regimen of the antibody molecule of the present disclosure can be determined by those skilled. In some embodiments, the dosage regimen is adjusted to provide an optimal desired response (e.g., therapeutic response).

In some embodiments, the antibody (and the pharmaceutical composition or the immunoconjugate comprising the same) of the present disclosure, alone or in combination with other therapeutic agents, can be administered twice a week, or once a week, or once every two weeks.

It will be understood that any treatment can be performed using the immunoconjugate, the composition, the combination product or the kit disclosed herein in place of or in addition to the antibody disclosed herein.

### Example 1. Construction of Anti-B7-H3/EGFR Bispecific Antibodies

The anti-B7-H3/EGFR bispecific antibody molecules of the present disclosure were prepared by assembling an anti-B7-H3 antibody parent and an anti-EGFR antibody parent into an antibody format of IgG1 (as shown in FIG. 1) by the protein science Innobody technology platform of Innovent Biologics (Suzhou) Co., Ltd. (application number: PCT/CN2021/143141, title of the invention: Protein containing heterodimer antibody Fc and preparation method therefor) or by conventional methods in the art. The bispecific antibody format contains four polypeptide chains and can bind to two antigens, antigen A (EGFR) and antigen B (B7-H3).

The parent antibodies used for constructing the bispecific antibodies were the anti-EGFR monoclonal antibody anti-zalutumumab (hereinafter referred to as Zalu, publication number: WO02100348A2, title of the invention: Human monoclonal antibodies to epidermal growth factor receptor (EGFR)) and anti-B7-H3 monoclonal antibodies. The antigen-binding region sequences of the anti-B7-H3 monoclonal antibodies Hz20G5.26 and Hz19A2.25 were obtained through antibody humanization from the hybridoma technology screening platform of Innovent Biologics (Hz20G5 and Hz19A2 in application number: PCT/CN2021/140449, title of the invention: Anti-B7-H3 antibody and use thereof); the antigen-binding region sequence of the anti-B7-H3 monoclonal antibody Hz5C2.9 was also obtained through antibody humanization from the hybridoma technology screening platform of Innovent Biologics. Meanwhile, the Innobody technology was adopted to perform mutations in the Fc region to improve the formation of a heterodimer of specific antibodies (application number: PCT/CN2021/143141, title of the invention: Protein containing heterodimer antibody Fc and preparation method therefor). Three bispecific antibodies that simultaneously bind to B7H3 and EGFR were obtained by screening and were numbered as Hz5C2.9/Zalu bsAb, Hz19A2.25/Zalu bsAb, and Hz20G5.26/Zalu bsAb bispecific antibody molecules, respectively. For the specific amino acid sequence numbers, refer to Table 1 and Table A - Sequence Information. All three bispecific antibodies are of the IgG-like structure. For the specific schematic diagram of the structure, refer to FIG. 1.

**Table 1. Details of sequences of B7H3/EGFR bsAb molecules and positive control Gp120/Zalu**

| Bispecific antibody molecule | B7H3 antibody parent | Heavy and light chains involved | EGFR antibody parent | Heavy and light chains involved |
|---|---|---|---|---|
| Hz20G5.26/Zalu | Hz20G5.26 | Heavy chain 2: SEQ ID NO: 35 | Zalu | Heavy chain 1: SEQ ID NO: 33, |
| | | Light chain 2: SEQ ID NO: 36 | | Light chain 1: SEQ ID NO: 34; |
| Hz19A2.25/Zalu | Hz19A2.25 | Heavy chain 2: SEQ ID NO: 37 | Zalu | Heavy chain 1: SEQ ID NO: 33, |
| | | Light chain 2: SEQ ID NO: 38 | | Light chain 1: SEQ ID NO: 34; |
| Hz5C2.9/Zalu | Hz5C2.9 | Heavy chain 2: SEQ ID NO: 39 | Zalu | Heavy chain 1: SEQ ID NO: 33, |
| | | Light chain 2: SEQ ID NO: 40 | | Light chain 1: SEQ ID NO: 34; |
| Gp120/Zalu | ¹Gp120 | | Zalu | Heavy chain 1: SEQ ID NO: 33, |
| | | | | Light chain 1: SEQ ID NO: 34; |
| Hz20G5 26/Gp 1 20 | | | | |
| | Hz20G5.26 | Heavy chain 2: SEQ ID NO: 35 | Gp120 | |
| | | Light chain 2: SEQ ID NO: 36 | | |

| | | | | |
|---|---|---|---|---|
| ¹ Zhou, T., Xu, L., Dey, B. et al., Structural definition of a conserved neutralization epitope on HIV-1 gp120. Nature 445, 732-737 (2007) | | | | |

### Example 2. Preparation of Anti-B7-H3/EGFR Bispecific Antibody Molecules

### 1. Plasmid construction of bispecific antibody molecules

The heavy chain sequences of the anti-EGFR antibodies, the light chain sequences of the anti-EGFR antibodies, the heavy chain sequences of the anti-B7-H3 antibodies, and the light chain sequences of the anti-B7-H3 antibodies listed in Table 1 were separately inserted into vectors pcDNA3.1 (Invitrogen, V790-20) to obtain heavy chain plasmids and light chain plasmids of an anti-EGFR end and heavy chain plasmids and light chain plasmids of an anti-B7-H3 end, respectively.

### 2. Preparation process of bispecific antibodies

By using the GlymaxX technology (ProBioGen AG, see patent publication number: WO2011035884A1, the entire content of which is incorporated herein by reference in its entirety for purposes of the present disclosure), RMD enzyme (GDP-6-deoxy-D-lyxo-4-hexylose reductase, sequence: SEQ ID NO: 41) plasmids were transiently co-transfected with the heavy and light chain plasmids of anti-EGFR parent antibodies and the heavy and the light chain plasmids of anti-B7-H3 parent antibodies, respectively, into ExpiCHO (Invitrogen, A29133) cells by PEI MAX (Polysciences, A804355) to express antibody parents of the anti-EGFR and anti-B7-H3 ends with a low fucose content. After 7 days, the cell fermentation broths were harvested, filtered for clarification, and separately captured on a Hitrap Mabselect Sure chromatographic column (GE Healthcare, 11-0034-95) to obtain the anti-EGFR parent antibodies and the B7H3 parent antibodies, respectively.

After the concentration was detected by using an A280 method, the antibody parents were mixed at a molar ratio of 1:1. An appropriate amount of reducing agent GSH was added, and the mixture was reacted overnight at room temperature. The reducing agent was removed by ultrafiltration, and the reaction was stopped. Fine purification was performed using a MonoS cation exchange chromatographic column (GE Healthcare, 17-5168-01), with a 20 mM sodium phosphate buffer (pH 6.6) as an anti-B7-H3 parent solution and a 20 mM sodium phosphate buffer (pH 6.6) containing 1 M sodium chloride as an anti-EGFR parent solution. The elution gradient was 0-50% (30 column volumes). The eluted protein solutions were ultrafiltered and buffer-exchanged into PBS (Gibco, 70011-044). The purity was determined by SEC-HPLC.

### Example 3. Assay of Affinity of Hz20G5.26/Zalu bsAb Bispecific Antibody Molecule

The affinities (KD) of the bispecific antibodies of the present disclosure for binding to B7H3 and EGFR were determined by using bio-layer interferometry (BLI).

Half an hour before the start of the experiment, an appropriate number of AHC sensors (18-5060, Sartorius) were taken according to the number of samples and soaked in an SD buffer (1× PBS, 0.1% BSA, 0.05% Tween-20). The antibodies prepared above, and human B7H3 (B73-H52E2, Acro Biosystem) and human EGFR (EGR-H5222, Acro Biosystem) were each diluted to 100 nM.

The SD buffer, antibody solutions, human B7H3, and human EGFR were added to a 96-well black polystyrene microplate (Greiner, 655209). Detection was performed using Fortebio Octet Red96e, and the sensors were arranged according to the positions of the samples on the plate. The instrument set-up parameters were as follows: operation steps: baseline equilibration for 120 s, sample loading for antibody immobilization for 100 s, baseline equilibration for 120 s, antigen binding for 100 s, and dissociation for 120 s, at a rotation speed of 1000 rpm and at a temperature of 30 °C. After the experiment was completed, KD values were analyzed using ForteBio Octet analysis software. The results are shown in Table 2 below.

**Table 2: Assay of affinity of B7H3/EGFR bispecific antibody molecules**

| **Name of antibody** | **Parent** | **KD(M)** | **Kon(1/Ms)** | **Kdis(1/s)** |
|---|---|---|---|---|
| **Hz20G5.26/Zalu** | **EGFR** | 4.59E-09 | 2.70E+05 | 1.24E-03 |
| | **B7H3** | 2.48E-09 | 6.55E+05 | 1.63E-03 |
| **Hz19A2.25/Zalu** | **EGFR** | 4.19E-09 | 2.89E+05 | 1.21E-03 |
| | **B7H3** | 6.73E-09 | 3.26E+05 | 2.19E-03 |
| **Hz5C2.9/Zalu** | **EGFR** | 4.69E-09 | 2.64E+05 | 1.24E-03 |
| | **B7H3** | 8.25E-09 | 4.27E+05 | 3.52E-03 |

### Example 4. Screening of Anti-B7-H3/EGFR Bispecific Antibody Molecules

The anti-B7-H3 and anti-EGFR antibody parent sequences were expressed and assembled into Hz5C2.9/Zalu bsAb, Hz19A2.25/Zalu bsAb, and Hz20G5.26/Zalu bsAb bispecific antibody molecules by using the Innobody technology platform. The B7H3/Zalu bsAb bispecific antibody molecules were tested for *in-vitro* activity against non-small cell lung cancer (NSCLC) and head and neck squamous cell carcinoma (HNSCC) by an antibody-to-cell proliferation inhibition assay (growth inhibition assay) and an antibody-dependent cell-mediated cytotoxicity assay (ADCC cell report). Meanwhile, safety verification was performed in a human cutaneous squamous cell carcinoma cell line (A431).

Cell line sources and media:
PC9 (NSCLC), Shanghai Yubo Biotechnology Co., Ltd., YB-H3210D; culture medium: MEM + 10% FBS + 1% Pen/strep
TE-1 (HNSCC): CoBioer, CBP60655; medium: RPMI 1640 + 10% FBS + 1% Pen/strep
SK-MES-1 (NSCLC), CoBioer, CBP60152; medium: MEM + 1% NEAA + 1 mM Sodium Pyruvate + 10% FBS + 1% Pen/strep
A431: cutaneous squamous cell carcinoma cell, ATCC, CRL-1555; culture medium: DMEM + 10% FBS + 1% Pen/strep
Construction of PC9 + hB7H3 and CHO-S + hB7H3 cell lines: a Lentvirus + hB7H3 lentivirus (hB7H3 (UniProt, Q5ZPR3-1), and Lentvirus (PPL public plasmid/protein library, BC000141)) was constructed and packaged, PC9 (NSCLC, Shanghai Yubo Biotechnology Co., Ltd., YB-H3210) and CHO-S (Thermo) were separately infected with Lentvirus + hB7H3, and PC9 + hB7H3 and CHO-S + hB7H3 stably transfected cell lines were obtained through pressurized screening and sorting.

### Experimental method:

### 1. Proliferation inhibition experiment (growth inhibition assay)

(1) NSCLC: plated at 1500-2500 cells/100 µL in a 96-well low-adsorption plate (Corning, CLS7007-24EA) for 3D cell culture.
   HNSCC: plated at 1500-2000 cells/100 µL in a 96-well white-bottom plate (NUNC, 136101) for 2D cell culture.
(2) The antibody molecules prepared in advance by dilution (the highest concentration: 300 nM, 3.16-fold dilution) were added to the corresponding cell well plate, mixed well, and cultured at 37 °C with 5% CO₂ for 5 days.
(3) After 5 days of continuous culture for proliferation inhibition assay, the Cell-Titer reagent (Promega, G7572) prepared in advance was added to the corresponding wells, the plate was left to stand at room temperature for 15-25 min in the dark, and then HNSCC cells were directly detected by a multimode microplate reader (Molecular Devices, Spectra MAXi3).
(4) NSCLC: Cell-Titer and the cell mixture were transferred to a 96-well white-bottom plate (NUNC, 136101) and detected by a multimode microplate reader (Molecular Devices, Spectra MAXi3).

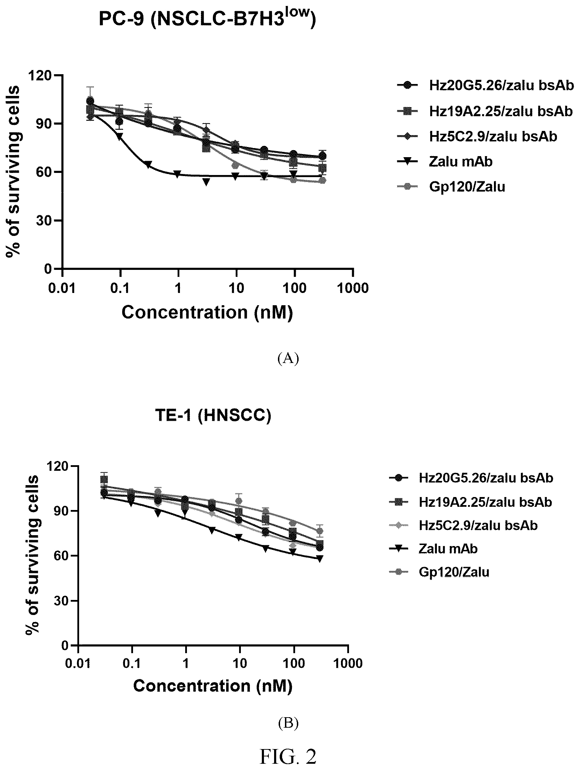

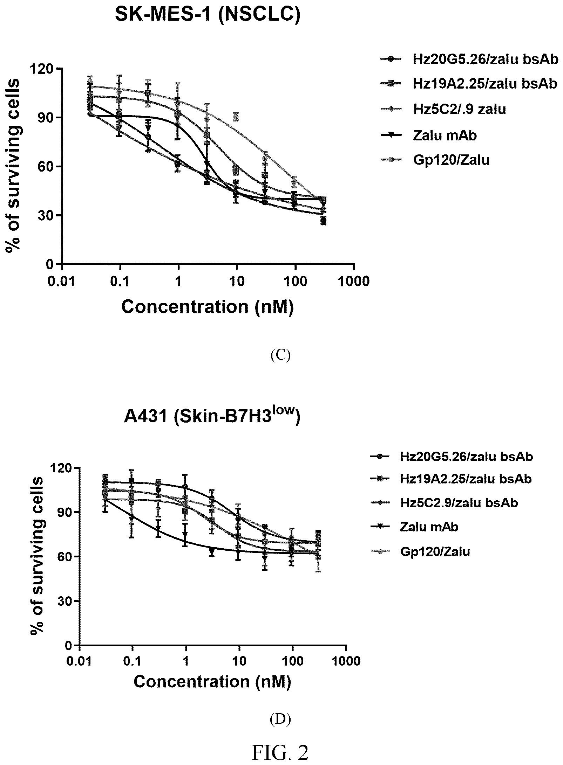

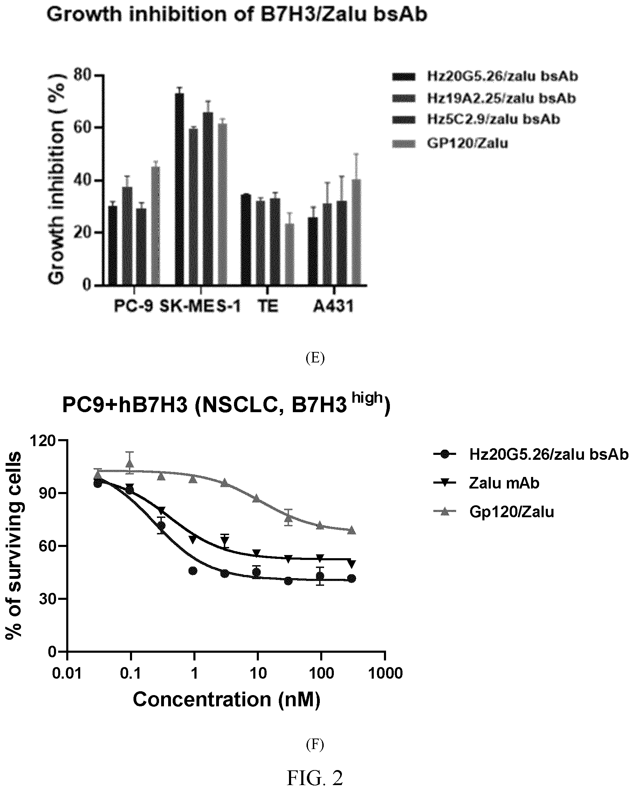

The results are shown in FIG. 2. The percentage of surviving cells (% of surviving cells) (FIGs. 2A, B, C, D and F) was the relative survival rate of the cells tested. For the labeling of the corresponding cells and the categories to which they belonged, see FIG. 2 and the description of the figures, for example, PC9 is a type of NSCLC, and TE-1 is a type of HNSCC. Among them, A431 is a cutaneous squamous cell carcinoma cell that does not respond to antibody therapy and is intended to be a measure of drug side effects.

The percentage of growth inhibition (growth inhibition %) in FIG. 2E was obtained through the analysis and statistics based on the results in FIG. 2 (% of surviving cells), specifically as follows: under the highest antibody concentration of 300 nM, the maximum cell inhibition rate of the drug in each type of cell was calculated as: 300 nM, (1 - % of surviving cells) × 100%.

In HNSCC and NSCLC cell lines, the results for the proliferation inhibition experiment on three bispecific antibody molecules of Hz5C2.9/Zalu bsAb, Hz19A2.25/Zalu bsAb, and Hz20G5.26/Zalu bsAb (FIGs. 2A, B and C) showed that the three bispecific antibody molecules exhibited significant tumor-killing effects in both HNSCC and NSCLC, among which the bispecific antibody molecule Hz20G5.26/Zalu bsAb not only had superior efficacy, but also had poor *in-vitro* efficacy in A431 (FIG. 2D), indicating that Hz20G5.26/Zalu bsAb could reduce or decrease side reactions such as skin toxicity associated with the clinical use of EGFR antibody series.

### 2. ADCC report assay (ADCC cell report assay)

(1) Target cells (CHO-S + hB7H3 constructed above or NCI-H522 (CoBioer, CBP60140)) and ADCC effector cells (Promega, G7102) were mixed well and added to a 96-well white-bottom plate (NUNC, 136101) according to an effector/target ratio (10:1).
(2) The antibody molecules prepared in advance by dilution (for the detection of CHO-S + HB7H3: among the final concentrations after antibody dilution, the highest was 6.25 nM, with a 4-fold serial dilution; for the detection of NCI-H522: among the final concentrations after antibody dilution, the highest was 100 nM, with a 4-fold serial dilution) were added to the corresponding cell well plate, mixed well, and cultured at 37 °C with 5% CO₂ for 20 h.
(3) The Bio-glo reagent (Promega, G755B) prepared in advance was added to the cell well plate, the plate was left to stand at room temperature for 10-15 min in the dark, and then the cells were detected by a multimode microplate reader (Molecular Devices, Spectra MAXi3).

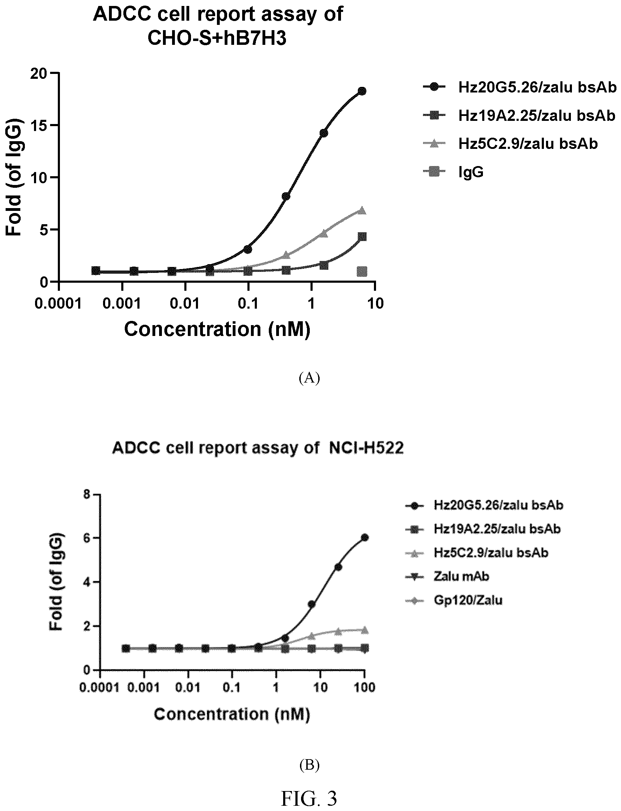

### The experimental results are shown in FIG. 3, where the ordinate indicates the multiples relative to IgG1 obtained with IgG1 as a control.

In NSCLC and HNSCC tumor cell lines, through the cell proliferation inhibition experiment (FIG. 2) and the ADCC report assay (FIG. 3), the experimental results showed that Hz20G5.26/Zalu bsAb exhibited the overall best *in-vitro* efficacy; in the A431 cutaneous squamous cell carcinoma cell line, Hz20G5.26/Zalu bsAb exhibited the lowest *in-vitro* toxic activity (as a measure of toxicity of EGFR inhibitors to human skin). Therefore, the bispecific antibodies of the present disclosure, especially Hz20G5.26/Zalu bsAb, had lower toxicity to human skin, and had a larger effective and safe drug dosage when causing no or weak toxic and side effects, that is, they had a wider efficacy selection window, and are safer in terms of efficacy.

### Example 5. In-Vitro Pharmacodynamic Activity of Proliferation Inhibition by Hz20G5.26/Zalu bsAb Bispecific Antibody Molecule

After an EGFR ligand binds to EGFR on the surface of tumor cells, EGFR can form a homodimer, which transmits signals into the cells, and the tumor cells will exhibit biological activities such as proliferation, invasion, metastasis, and apoptosis resistance through various cascade reactions.

After the antigen-binding moiety against EGFR in Hz20G5.26/Zalu bsAb binds to EGFR on the surface of tumor cells, in one aspect, the binding of the EGFR ligand to EGFR can be blocked, and thus the biological signal transmission can be inhibited, and the corresponding biological activities of the tumor can be blocked; in another aspect, the endocytosis of EGFR is stimulated, and thus the final degradation of EGFR by intracellular lysosomes and the like is enabled. EGFR is an epithelial-derived broad-spectrum tyrosine kinase receptor, and Hz20G5.26/Zalu bs Ab adopts an EGFR antibody parent sequence with low affinity for EGFR, thereby greatly reducing the toxic and side effects of the EGFR monoclonal antibodies on normal epithelial tissues such as the skin. B7-H3 is highly expressed in various tumor cell lines but is lowly expressed in normal tissues and organs, and is TAA with extremely high selectivity and a wide expression profile. Hz20G5.26/Zalu bs Ab incorporates a B7-H3 high-affinity Hz20G5.26 parent on the basis of low affinity for EGER, thereby greatly improving the blocking activity against EGFR signaling and improving the pharmacodynamic biological activity and pharmacodynamic safety window of Hz20G5.26/Zalu bs Ab.

### Example 5.1 Detection of B7-H3 and EGFR Expression in Tumor Cell Lines

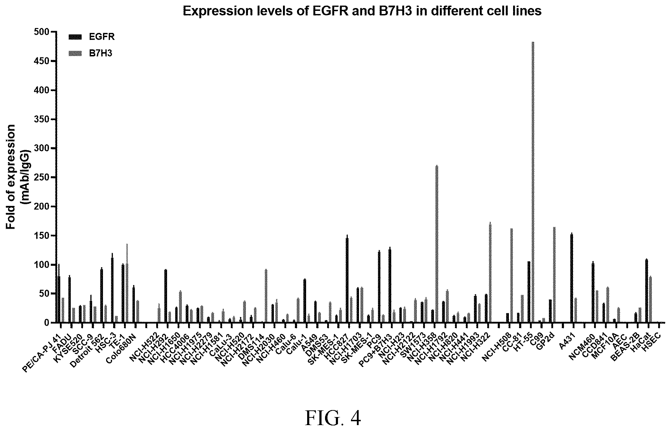

There is a certain positive correlation between the drug sensitivity of Hz20G5.26/Zalu bsAb to tumor cell lines and the abundance of receptor expression on the surface of tumor cells. Some cell lines were selected from NSCLC (non-small cell lung cancer), HNSCC (head and neck squamous cell carcinoma), CRC (colorectal cancer), and normal cell lines for the detection of relative expressions of B7-H3 and EGFR, and the *in-vitro* pharmacodynamic activity of Hz20G5.26/Zalu bsAb was detected based on the abundance of B7-H3 and EGFR expressions on the cell surface. The experimental results are shown in FIG. 4.

### Experimental method:

1. Various cells were resuspended in an FACS buffer, 100,000-200,000 cells were plated in a 96-well plate (Corning, CLS3799-50EA), then 10 µg/mL EGFR antibody (Cetuximab), 10 µg/mL B7-H3 antibody (Hz20G5.26mAb) and 10 µg/mL IgG were added to the cell well plate, and the plate was incubated at 4 °C for 1 h.
2. The plate was washed twice with PBS, then the prepared APC-anti human Fc antibody (Biolegend, 410712) was added to the corresponding cell well plate, and the plate was incubated at 4 °C for 30-40 min.
3. The plate was washed twice with PBS, and then FACS detection (BD, Celesta) was performed. The results are shown in FIG. 4.

FIG. 4 showed the expression levels of B7H3 and EGFR in different cell lines, in which the cell lines with higher B7H3 expression levels and dependent on the EGFR signaling pathway (e.g., a cell line containing EGFR mutations obtained subsequently by sequencing analysis (results not shown) or an EGFR-overexpressing cell line) could be selected for the activity detection of bispecific antibodies.

### Example 5.2 Proliferation Inhibition Experiment by Hz20G5.26/Zalu bsAb Bispecific Antibody Molecule in Multiple Tumor Cell Lines

EGFR is highly expressed on the surface of various solid tumor cells, such as lung cancer (30%-80%), head and neck squamous carcinoma (36% -100%), colorectal cancer (25%-77%), and esophageal cancer (43%-89%), and is a broad-spectrum anti-tumor tyrosine kinase target protein. EGFR, as a broad-spectrum anti-tumor target protein, also has the highest mutation rate among the mutation types in non-small cell lung cancer patients (the global average mutation rate is about 35%, reaching 40% in China), and in NSCLC patients carrying EGFR mutations, classical mutations (L858R, T790M, or Exon19 deletion) account for 85%-90%; uncommon mutations such as EGFR exon 20ins, T790M primary point mutations, and compound mutations, as well as other point mutations located between exons 18-21 represented by G719X and sequence repeat mutations thereof, account for about 10% of the EGFR mutations.

Construction of H1975-EGFR^{L858R/T790M/C797S}, PC9+B7H3-EGFR^{Del19/T790M/C797S}, and H322-EGFR^{S768_D770dup} cell lines: Lentvirus + EGFR^{L858R/T790M/C797S}, Lentvirus + EGFR^{Del19/T790M/C797S}, and Lentvirus + EGFR^{S768_D770dup}viruses were separately constructed and packaged, NCI-H1975 (ATCC, CRL-5908), PC9 + hB7H3 (constructed as described above), and H322 were infected with Lentvirus + EGFR^{L858R/T790M/C797S}, Lentvirus + EGFR^{Del19/T790M/C797S}, and Lentvirus + EGFR^{S768_D770du} viruses, respectively, and H1975-EGFR^{L858R/T790M/C797S}, PC9+B7H3-EGFR^{Del19/T790M/C797S}, and H322-EGFR^{S768_D770dup} stably transfected cell lines were obtained through pressurized screening.

### Experimental method:

### Proliferation inhibition experiment

(1) NSCLC: plated at 1500-2500 cells/100 µL in a 96-well low-adsorption plate (Corning, CLS7007-24EA) for 3D cell culture, in which the following cell lines and specific amounts were used:
   NSCLC: 2000 cells/100 µL/well
   NCI-H292: Cell Bank of the Chinese Academy of Sciences, SCSP-582
   NCI-H322: Cobioer, CBP60134
   NCI-H1650: ATCC, CRL-5883
   NCI-H1975: ATCC, CRL-5908
   SK-MES-1: CoBioer, CBP60152
   NCI-H1703: ATCC, HTB-43
   PC9 + hB7H3: constructed as described above
   CRC and HNSCC: plated at 1500-2000 cells/100 µL in a 96-well white-bottom plate (NUNC, 136101) for 2D cell culture, in which the following cell lines and specific amounts were used:
      CRC: 1500 cells/100 µL/well
      CCK-81: CoBioer, CBP60581
      HT-55: CoBioer, CBP60012
      H508: CoBioer, CBP60795
      LS180: CoBioer, CBP60034
      HNSCC: 1500 cells/100 µL/well
      TE-1: CoBioer, CBP60655
      Colo680: CoBioer, CBP60452. (2) The antibody molecules prepared in advance by dilution were added to the corresponding cell well plate, mixed well, and cultured in an incubator at 37 °C with 5% CO₂ for 5 days.
(3) After 5 days of continuous culture for proliferation inhibition assay, the Cell-Titer reagent (Promega, G7572) prepared in advance was added to cell wells, the plate was left to stand at room temperature for 15-25 min in the dark, and then CRC and HNSCC cells were directly detected by a multimode microplate reader (Molecular Devices, Spectra MAXi3).
(4) NSCLC: Cell-Titer and the cell mixture were transferred to a 96-well white-bottom plate (NUNC, 136101) and detected by a multimode microplate reader (Molecular Devices, Spectra MAXi3).

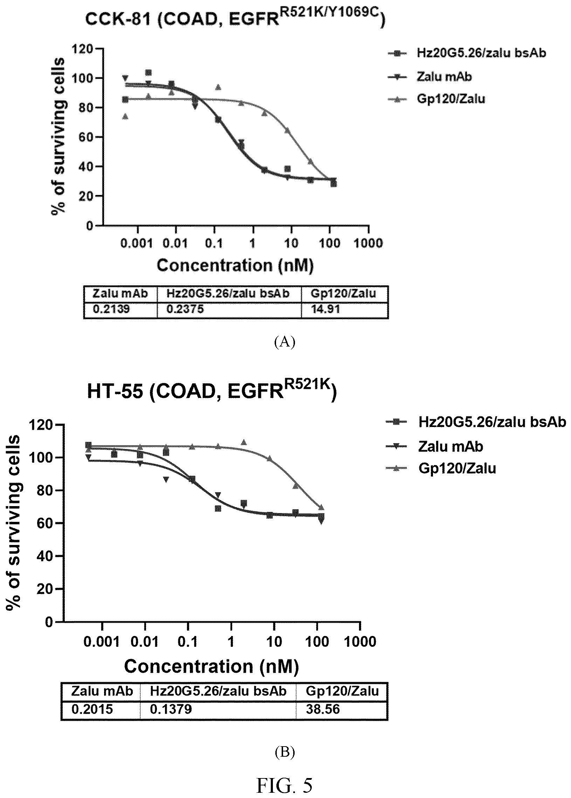

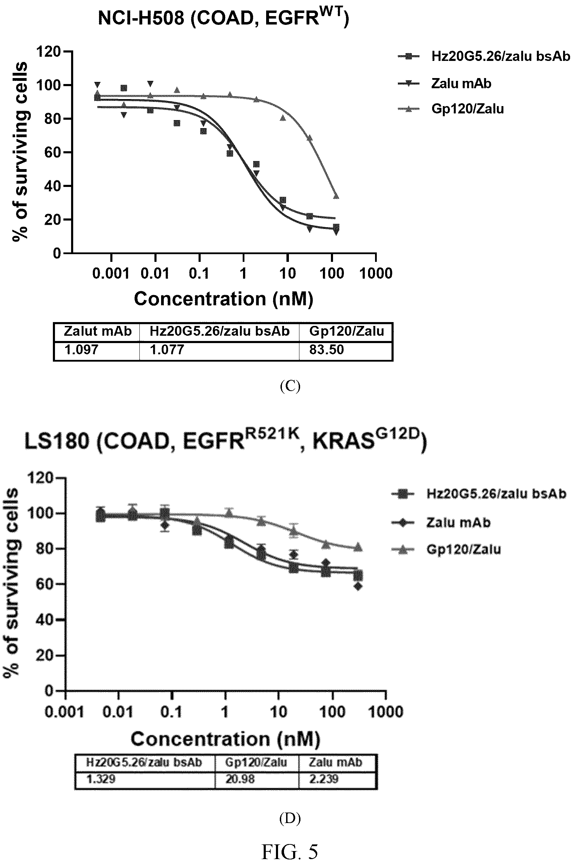

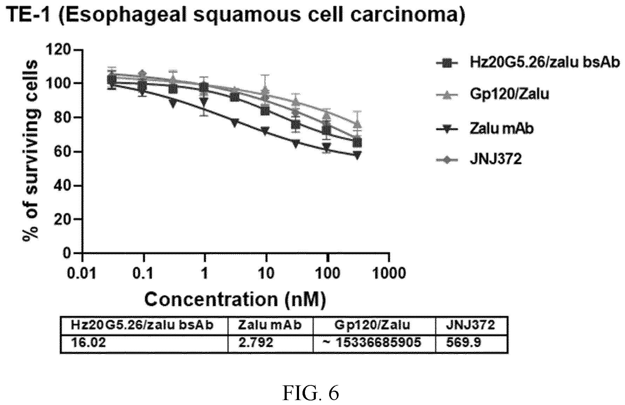

EGFR was used as a broad-spectrum anti-tumor target protein. In terms of the anti-tumor efficacy of *in-vitro* proliferation inhibition, it was found that Hz20G5.26/Zalu bsAb exhibited an anti-tumor effect in esophageal cancer tumor cell lines (as shown in FIG. 6). Among them, in the TE-1 esophageal cancer tumor cell line, Hz20G5.26/Zalu bsAb exhibited a better anti-tumor effect than JNJ372. In colon cancer and other tumor cell lines (as shown in FIG. 5), Hz20G5.26/Zalu bsAb could achieve the anti-tumor effect of the EGFR monoclonal antibody. Particularly, in the LS180 colon cancer tumor cell line, its *in-vitro* anti-tumor effect was superior to that of the EGFR monoclonal antibody; in the NCI-H508 colon cancer tumor cell line, the maximum anti-tumor killing rate reached 84%.

In lung cancer, not all cancer tumor patients develop and progress due to primary gene mutations of EGFR. In non-small cell lung cancer patients, the average mutation rate of EGFR is about 35%. EGFR-TKI small molecules (such as erlotinib (Selleck Chemicals, cat: S7786) or osimertinib (Selleck Chemicals, cat: S7297)) serving as treatment standards mainly target patients with sensitive gene mutations, but often become resistant and ineffective due to gene mutations. Therefore, it is necessary to continuously develop new targeted drugs for new mutation sites. In lung cancer, Hz20G5.26/Zalu bsAb is not only effective against NSCLC tumor cell lines with EGFR mutations, but also exhibits *in-vitro* anti-tumor effects on wild-type EGFR and abnormally amplified EGFR non-small cell lung cancer tumor cell lines. In NSCLC tumor cell lines, the overall *in-vitro* anti-tumor efficacy of Hz20G5.26/Zalu bsAb is far superior to the *in-vitro* anti-tumor effect of JNJ-372.

In the NSCLC-EGFR^{WT} tumor cell line (FIG. 7.1), the maximum *in-vitro* anti-tumor killing rate of NCI-H292 was 74.2% ± 2.8%; in the NSCLC-EGFR classical mutation (L858R, T790M, or Exon19 deletion) tumor cell lime (FIG. 7.2), for NCI-H1975 (EGFR^{L858R/T790M}) (FIG. 7.2B), the maximum *in-vitro* anti-tumor inhibition or killing rate was 51.2% ± 1.4%, with IC50 of 0.99 nM, and the *in-vitro* efficacy was superior to that of JNJ-372 and the EGFR monoclonal antibody; in the NSCLC-EGFR abnormally amplified tumor cell line (FIG. 7.3), for SK-MES-1, the maximum *in-vitro* anti-tumor killing rate was 60.7% ± 15.9%, with IC50 of 0.40 nM, and the overall *in-vitro* efficacy was superior to that of JNJ-372 and the EGFR monoclonal antibody. Moreover, these NSCLC-EGFR abnormally amplified tumor cell lines SK-MES-1 and NCI-H1703 were resistant and not sensitive to the first-generation (erlotinib) and third-generation (osimertinib) drugs. The third-generation EGFR-TKI small molecule inhibitor osimertinib, which has been marketed and used for treating T790M mutation, has poor efficacy against the rare mutation EGFR^{exon20ins}. In order to investigate the efficacy of Hz20G5.26/Zalu bsAb against EGFR^{exon20ins} tumors, we constructed a H322-EGFR^{S768_D770dup} stably transfected cell line on the basis of NCI-H322-EGFR^{WT} cells. The anti-tumor efficacy of *in-vitro* proliferation inhibition (FIG. 7.4) showed that Hz20G5.26/Zalu bsAb exhibited *in-vitro* efficacy activity against EGFR^{exon20ins}. With the administration and treatment of EGFR-TKI small molecule inhibitors, NSCLC patients are constantly developing double (such as Del19/T790M or L858R/T790M) or triple (such as Del19/T790M/C797S or L858R/T790M/C797S) drug-resistant mutations. This also presents an important development direction for the next-generation EGFR-TKI. In order to verify the pharmacodynamic activity of Hz20G5.26/Zalu bsAb against triple drug-resistant mutations of EGFR-TKI, we constructed H1975-EGFR^{L858R/T790M/C797S} and PC9+B7H3-EGFR^{Del19/T790M/C797S} stably transfected cell lines. The anti-tumor efficacy of *in-vitro* proliferation inhibition (FIG. 7.4) showed that Hz20G5.26/Zalu bsAb exhibited certain *in-vitro* pharmacodynamic activity against EGFR^{L858R/T790M/C797S} and EGFR^{Del19/T790M/C797S}.

To further verify the *in-vitro* pharmacological activity of Hz20G5.26/Zalu bsAb, a comparative analysis of the *in-vitro* proliferation inhibition efficacy was performed on Hz20G5.26/Zalu bsAb and a combination of gp120/Zalu and gp120/Hz20G5.26 (FIG. 7.5). The results showed that in the NCI-H292 (Lung adeno, EGFR^{WT}), SK-MES-1 (sqNSCLC, EGFR^{Amp}) NSCLC tumor cell lines and the LS180 CRC tumor cell line, the *in-vitro* efficacy of Hz20G5.26/Zalu bsAb was far superior to that of the combination of gp120/Zalu and gp120/Hz20G5.26. The results for proliferation inhibition and tumor killing experiments on Hz20G5.26/Zalu bsAb are shown in Table 3.

**Table 3. Results for proliferation inhibition and tumor killing experiments on Hz20G5.26/Zalu bsAb**

| Cell line | EGFR State | KRAS State | IC50(nM) | | | Max inhibition (%) | | |
|---|---|---|---|---|---|---|---|---|
| | | | | | | bsAb | Zalu | JNJ372 |
| | | | bsAb | Zalu | JNJ372 | Mean±SD | Mean±SD | Mean±SD |
| CCK-81 | R521/K | WT | 0.24 | 0.21 | NA | 71.7 | 69.5 | NA |
| | Y1069C | | | | | | | |
| HT-55 | R521K | WT | 0.14 | 0.20 | NA | 35.8 | 39.0 | NA |
| H508 | WT | WT | 1.08 | 1.10 | NA | 84.4 | 87.7 | NA |
| LS180 | R521K | G12D | 1.33 | 2.24 | NA | 35.0±5.2 | 41.0±2.2 | NA |
| TE-1 | WT | WT | 16.02 | 2.80 | 569.90 | 34.6±0.5 | 42.2±1.1 | 32.2±4.7 |
| Colo-680N | WT | WT | 12.29 | 0.09 | 25.53 | 21.8±2.4 | 24.9±1.1 | 26.5±2.2 |
| H292 | WT | WT | 57.84 | 8.68 | 140.90 | 74.2±2.8 | 79.4±1.6 | 64.5±2.5 |
| H322 | WT | WT | 0.51 | 1.44 | 8.06 | 41.7±4.3 | 39.9±0.8 | 38.2±6.7 |
| H1650 | Amplification | WT | 4.86 | 3.02 | 20.59 | 32.9±0.6 | 26.8±2.6 | 31.0±1.4 |
| | Del19 | | | | | | | |
| H1975 | L858R | WT | 0.99 | 1.78 | 5.67 | 51.2±1.4 | 51.0±2.3 | 47.9±3.0 |
| | T790M | | | | | | | |
| SK-MES-1 | Amplification | WT | 0.40 | 1.10 | 9.82 | 60.7±15.9 | 67.8±4.1 | 66.1±3.7 |
| H1703 | Amplification | WT | 0.04 | 0.04 | 0.70 | 50.2±1.3 | 45.6±3.8 | 44.3±3.4 |

### Example 5.3 Synergistic Effect of Combination of Hz20G5.26/Zalu bsAb Bispecific Antibody Molecule and KRAS Small Molecule Inhibitor

In cancer tumor patients, in addition to the abnormal activation of EGFR leading to carcinogenesis, the activation due to KRAS gene mutations is a common driver of cancer tumors. About 25% of human cancers are related to KRAS mutations, and KRAS mutations are highly relevant to the prognosis and treatment of cancer. KRAS-G12D and KRAS-G12C are common abnormal mutations in CRC and NSCLC tumors. Now, with the use of KRAS small molecule inhibitors, it is expected that KRAS-acquired drug resistance will emerge subsequently. The combination of KRAS small molecule inhibitors with other drugs (such as immunotherapy drugs or targeted therapy drugs) may be a new drug research and development direction.

### Experimental method:

### Proliferation inhibition experiment

(1) NSCLC (H358) (Cobioer, CBP60136) and CRC (LS180) (Cobioer, CBP60034): plated at 1500-2500 cells/100 µL in a 96-well low-adsorption plate (Corning, CLS7007-24EA) for 3D cell culture.
(2) The antibody molecules and small molecule inhibitors prepared in advance by dilution were added to the corresponding cell well plate, mixed well, and cultured at 37 °C with 5% CO₂ for 5 days.
(3) After 5 days of continuous culture for proliferation inhibition assay, the Cell-Titer reagent (Promega, G7572) prepared in advance was added to cell wells, and the plate was left to stand at room temperature for 15-25 min in the dark.
(4) Cell-Titer and the above cell mixture were transferred to a 96-well white-bottom plate (NUNC, 136101) and detected by a multimode microplate reader (Molecular Devices, Spectra MAXi3).

It was found from the results of the **NCI-H358 (EGFR^{WT}, KRAS^{G12C}) NSCLC** tumor cell line (FIG. 8A) that the maximum *in-vitro* anti-tumor killing rate of Hz20G5.26/Zalu bsAb in H358 was 66.9% ± 1.9%, with IC₅₀ of 0.48 nM, and the anti-tumor efficacy was far superior to that of the EGFR monoclonal antibody (Zalu monoclonal antibody) and JNJ-372, and was superior to that of the combination of Gp120/Zalu and Gp120/Hz20G5.26. In combination with AMG510 (KRAS-G12C small molecule inhibitor) (MedChem Express, HY-114277), it was found that there was a synergistic effect between Hz20G5.26/Zalu and AMG510, and under the action of 10 nM Hz20G5.26/Zalu bsAb, the killing rate of H358 cells was increased from 22.51% to 67.12% by 0.98 nM AMG510, and the anti-tumor effect was enhanced in H358 cells (FIG. 8B). In order to demonstrate the synergistic effect, pharmacodynamic synergy scores were further calculated in SynergyFinder (https://synergyfinder.fimm.fi/synergy/20210817124619827928/) based on FIG. 8B. The results are in FIGs. 8C and 8D. FIG. 8D showed the sum of the scores for the different dimensions, with a total score of 11.895, greater than 10, indicating that there was a synergistic effect between the two drugs.

It was found from the results of the LS180 (EGFR^{R521K}, KRAS ^{G12D}) CRC tumor cell line that the *in-vitro* anti-tumor efficacy of Hz20G5.26/Zalu bsAb was superior to that of the EGFR monoclonal antibody Zalu, JNJ372, and the combination of Gp120/Zalu and gp120/Hz20G5.26 (FIG. 7.5); moreover, in combination with MRTX1133 (KRAS-G12D small molecule inhibitor), there was a significant synergistic effect, and under the condition of 1.56 nM Hz20G5.26/Zalu bsAb, the anti-LS180 tumor efficacy was increased from 36.45% to 61.56% by 15.62 nM MRTX1133, and the anti-tumor effect was significantly enhanced (FIG. 9A). Synergy scores were further calculated in FIGs. 9B and 9C using SynergyFinder, with a total score of 37.714, indicating that there was a synergistic effect between the two drugs.

### Example 5.4. Mechanism Study on In-Vitro Efficacy of Proliferation Inhibition by Hz20G5.26/Zalu bsAb Bispecific Antibody Molecule

In the proliferation inhibition experiment, Hz20G5.26/Zalu bsAb further enhanced the blocking of EGFR signals under the help of the B7H3 antibody, thereby realizing a stronger anti-tumor killing effect. On NCI-H358 cells, we conducted studies in two directions, i.e., the inhibition and blocking of EGFR signals and the blocking of EGFR ligands, by Western blotting, to investigate the reason why the *in-vitro* anti-tumor effect of Hz20G5.26/Zalu bsAb was superior to that of the EGFR monoclonal antibody and the combination of Gp120/Zalu and Gp120/Hz20G5.26.

### Experimental method:

### 1. EGFR signal blocking experiment (signal blocking)

### (1) Cell treatment

<1> NCI-H358 (Cobioer, CBP60136) was taken and plated at 7.0E5 cells/well in a 6-well plate (NEST, 703011) for overnight culture.
<2> The medium (RPMI1640 complete medium (RPMI1640 + 10% FBS)) was removed, a serum-free medium RPMI1640 (assay medium), and the cells were subjected to starvation treatment overnight.
<3> The medium in <2> was removed, a serum-free medium (assay medium) containing 200 nM Hz20G5.26/Zalu bsAb, 200 nM Gp120/Zalu, 200 nM Gp120/Hz20G5.26, 200 nM Zalu mAb, and 200 nM Gp120/Zalu + 200 nM Gp120/Hz20G5.26 was added to the corresponding cell well plate, and a blank group (medium), a bsAb group (200 nM Hz20G5.26/Zalu bsAb), a Gp120/Zalu group (200 nM Gp120/Zalu), a Gp120/B7H3 group (200 nM Gp120/Hz20G5.26), a Zalu group (200 nM Zalu mAb), a Gp120/Zalu + Gp120B7H3 group (200 nM Gp120/Zalu + 200 nM Gp120/Hz20G5.26) were set. The plate was left to stand at 37 °C with 5% CO₂ for 2 h.

### (2) Protein extraction and total protein quantification

<1> A cell lysis buffer was prepared in advance, and the composition of the cell lysis buffer was as follows: 100-200 µL/tube RIPA (Thermo, 89900) + 1:100 phosphatase inhibitor (abcam, ab201112) + 1:20 protease inhibitor (Roche, 11836170001).
<2> The lysis buffer prepared in advance was added to the cells obtained in (1) above to digest the cells. Then, the cells were lysed on ice for 20 min.
<3> The centrifuge was pre-cooled at 4 °C in advance. Then the lysate was centrifuged at 12,000 rpm/min at 4 °C for 15 min, and the supernatant was collected.
<4> The concentration of the extracted total protein was calibrated by using a BCA reagent (Beyotime, P0012).
<5> The corresponding total protein was diluted with an LDS sample buffer (Invitrogen, 2201446), denatured at 70 °C for 10 min, and stored at -40 °C for later use.

### (3) Western blotting

<1> The protein sample obtained in (3) and a protein marker (Prestained protein ladder (Thermo, 26620)) were added to a pre-gel (Thermo, NP0321BOX); the gel was run at 200 V for about 50 min using an electrophoresis apparatus (BIO-RAD, TRANS SD CELL).
<2> The membrane was taken out and transferred using a transfer apparatus (Invitrogen, IBCOT2).
<3> The membrane was slowly blocked with the prepared blocking buffer (containing 5% of skim milk powder in TBST) for 1-2 h; EGF receptor rabbit mAb (CST, 4267), pEGF receptor rabbit mAb (CST, 3777), and GAPDH rabbit mAb (CST, 2118) antibodies were added and incubated at 4 °C overnight.
<4> The membrane was washed three times with TBST, each time for 8-10 min, with the rotating speed of the shaker controlled to be 80-100 rpm/min.
<5> The membrane was incubated with labeled HRP-goat anti-rabbit IgG (abcam, Ab205718) at room temperature for 1-2 h.
<6> The membrane was washed three times with TBST, each time for 8-10 min, with the rotating speed of the shaker controlled to be 80-100 rpm/min.
<7> The membrane was developed using an ECL luminescence reagent (Beyotime, P0018AM), and was exposed to light using a developing apparatus (BIO-RAD, chemi Doc MP).

The results are shown in FIG. 10.

### 2. EGFR ligand blocking experiment (signal blocking)

### (1) Cell treatment

<1> NCI-H358 (H358) was taken and plated at 7.0E5 cells/well in a 6-well plate (NEST, 703011) for overnight culture.
<2> The medium (RPMI1640 complete medium (RPMI1640 + 10% FBS)) was removed, a serum-free medium RPMI1640, and the cells were subjected to starvation treatment overnight.
<3> The medium was removed, a medium (to 2 cell wells) and a medium containing 200nM Hz20G5.26/Zalu bsAb, 200 nM Gp120/Zalu, 200nM Gp120/Hz20G5.26, 200 nM Zalu mAb, and 200 nM Gp120/Zalu + 200 nM Gp120/Hz20G5.26 were added to the corresponding cell well plate, and a blank group (medium), a control group (medium), a bsAb group (200 nM Hz20G5.26/Zalu bsAb), a Gp120/Zalu group (200 nM Gp120/Zalu), a Gp120B7H3 group (200 nM Gp120/Hz20G5.26), a Zalu group (200 nM Zalu mAb), a Gp120/Zalu + Gp120B7H3 group (200 nM Gp120/Zalu + 200 nM Gp120/Hz20G5.26) were set. The plate was left to stand at 37 °C with 5% CO₂ for 1 h.
<4> After 1 h, 30 nM EGF (ACRO, EGF-H52b) (upper panel in FIG. 11) and 30 nM TGF-α (R&D, 239-A-100) (lower panel in FIG. 11) were added to the blank group (medium), the bsAb group (200 nM Hz20G5.26/Zalu bsAb), the Gp120/Zalu group (200 nM Gp120/Zalu), the Gp120/B7H3 group (200 nM Gp120/Hz20G5.26), the Zalu group (200 nM Zalu mAb), and the Gp120/Zalu + Gp120/B7H3 group (200 nM Gp120/Zalu + 200 nM Gp120/Hz20G5.26), and the plate was left to stand at 37 °C with 5% CO₂ for 1 h.

### (2) Protein extraction and total protein quantification

<1> A cell lysis buffer was prepared in advance, and the composition of the cell lysis buffer was as follows: 100-200 µL/tube RIPA (Thermo, 89900) + 1:100 phosphatase inhibitor (abcam, ab201112) + 1:20 protease inhibitor (Roche, 11836170001).
<2> The lysis buffer prepared in advance was added to the cells obtained in (1) above to digest the cells. Then, the cells were lysed on ice for 20 min.
<3> The centrifuge was pre-cooled at 4 °C in advance. Then the lysate was centrifuged at 12,000 rpm/min at 4 °C for 15 min, and the supernatant was collected.
<4> The concentration of the extracted total protein was calibrated by using a BCA reagent (Beyotime, P0012).
<5> The corresponding total protein was diluted with an LDS sample buffer (Invitrogen, 2201446), denatured at 70 °C for 10 min, and stored at -40 °C for later use.

### (3) Western blotting

<1> The protein sample and a protein marker (Prestained protein ladder (Thermo, 26620)) were added to a pre-gel (Thermo, NP0321BOX); the gel was run at 200 V for about 50 min using an electrophoresis apparatus (BIO-RAD, TRANS SD CELL).
<2> The membrane was taken out and transferred using a transfer apparatus (Invitrogen, IBCOT2).
<3> The membrane was slowly blocked with the prepared blocking buffer (containing 5% of skim milk powder in TBST) for 1-2 h; EGF receptor rabbit mAb (CST, 4267), pEGF receptor rabbit mAb (CST, 3777), and GAPDH rabbit mAb (CST, 2118) antibodies were added and incubated at 4 °C overnight.
<4> The membrane was washed three times with TBST, each time for 8-10 min, with the rotating speed of the shaker controlled to be 80-100 rpm/min.
<5> The membrane was incubated with labeled HRP-goat anti-rabbit IgG (abcam, Ab205718) at room temperature for 1-2 h.
<6> The membrane was washed three times with TBST, each time for 8-10 min, with the rotating speed of the shaker controlled to be 80-100 rpm/min.
<7> The membrane was developed using an ECL luminescence reagent (Beyotime, P0018AM), and was exposed to light using a developing apparatus (BIO-RAD, chemi Doc MP).

The results are shown in FIG. 11.

It was found from the results for the study on signal blocking mechanism (FIG. 10 and FIG. 11) that Hz20G5.26/Zalu bsAb had stronger signal blocking and ligand blocking effects than the EGFR monoclonal antibody Zalu and the combination of Gp120/Zalu and Gp120/Hz20G5.26. The results also revealed the reason why Hz20G5.26/Zalu bsAb had better *in-vitro* anti-CRC, NSCLC and HNSCC tumor effects than the EGFR monoclonal antibody, and Gp120/Zalu and Gp120/Hz20G5.26.

### Example 6. In-Vitro Pharmacodynamic Activity of ADCC of Hz20G5.26/Zalu bsAb Bispecific Antibody Molecule

Antibody-dependent cell-mediated cytotoxicity (ADCC) is an important mechanism by which anti-tumor antibodies exert their anti-tumor effects. The principle of ADCC is that a Fab segment of an antibody binds to an epitope on the surface of a tumor cell, an Fc segment of the antibody binds to an FcR on the surface of a killer immune cell (NK cell, macrophage, neutrophil, and the like), and the tumor cell is directly killed through the mediation of the immune cell. The ADCC is mainly realized through Fc of an antibody and an FcRIIIa receptor on the surface of NK.

Due to the introduction of the Hz20G5.26 parent, not only the blocking activity against the EGFR antibody was improved in Hz20G5.26/Zalu bsAb, but also the ADCC effect of Hz20G5.26/Zalu bsAb was improved as a whole (it is presumed that Hz20G5.26 binds to a membrane-proximal specific epitope of B7H3, and thus a strong ADCC function is triggered); meanwhile, the GlymaxX low fucose technology is adopted, so that the ADCC effect of Hz20G5.26/Zalu bsAb is further enhanced.

Therefore, the Hz20G5.26/Zalu bsAb bispecific antibody molecule can jointly exert various anti-tumor pharmacodynamic effects through two mechanisms of action of EGFR signal blocking and ADCC. The specific experimental steps were as follows:

### Example 6.1 ADCC report assay

In the ADCC report assay, ADCC effector cells ((Promega, G7102)) are engineered cells in which FcRIIIa (V158) receptor is overexpressed into Jurkat T cells, which rapidly respond to ADCC through the NFAT-RE driving element within the Jurkat T cells.

### Experimental method:

(1) Tumor cells (cell lines listed in Table 3) and ADCC effector cells (Promega, G7102) were mixed well (1.5E4 cells:1.5E5 cells/well/100 µL (1.5E5:1.5E6 cells/mL)) according to an effector/target ratio (10:1), and added to a 96-well white plate (NUNC, 136101).
(2) The antibody molecules prepared in advance by dilution were added to the corresponding cell well plates, mixed well, and cultured at 37 °C with 5% CO₂ for 20 h.
(3) The Bio-glo reagent (Promega, G755B) prepared in advance was added to the cell well plate, the plate was left to stand at room temperature for 10-15 min in the dark, and then the cells were detected by a multimode microplate reader (Molecular Devices, Spectra MAXi3).

We selected tumor cell lines with different EGFR types (EGFR wild type, EGFR abnormally amplified type, EGFR mutation type, and EGFR wild type and KRAS mutation type) among NSCLC tumor cell lines, and verified the ADCC activity of Hz20G5.26/Zalu bsAb by the ADCC report assay. It was found from the results for the ADCC report assay (FIG. 12 and Table 4) that the ADCC activity of Hz20G5.26/Zalu bsAb was stronger than that of JNJ373 and the EGFR monoclonal antibody among the different NSCLC-EGFR tumor cell lines. Meanwhile, it was found from the experimental results of H292 (NSCLC-EGFR wild-type) and H358 (NSCLC-EGFR wild-type and KRAS mutant type) that the ADCC activity of Hz20G5.26/Zalu bsAb was stronger than that of JNJ372 and the EGFR monoclonal antibody, but also stronger than that of the combination of Gp120/Zalu and Gp120/Hz20G5.26.

**Table 4. Results for ADCC reporter gene assay of Hz20G5.26/Zalu bsAb**

| **Cell line** | **EGFR State** | **KRAS State** | **EC50(nM)** | | | **Max Fold(of IgG)** | | |
|---|---|---|---|---|---|---|---|---|
| | | | **bsAb** | **Zalu** | **JNJ372** | **bsAb** | **Zalu** | **JNJ372** |
| **H292** | **WT** | **WT** | **0.023** | **0.009** | **0.083** | **6.23** | **4.07** | **3.73** |
| **H322** | **WT** | **WT** | **0.006** | **0.006** | **0.106** | **5.07** | **3.65** | **4.04** |
| **H1650** | **Amplification** | **WT** | **0.038** | **0.021** | **0.102** | **9.23** | **5.46** | **4.98** |
| | **DeI19** | | | | | | | |
| **H1975** | **L858R** | **WT** | **0.107** | **0.029** | **0.063** | **3.63** | **3.19** | **2.41** |
| | **T790M** | | | | | | | |
| **SK-MES-1** | **Amplification** | **WT** | **0.040** | **0.011** | **0.144** | **7.26** | **4.91** | **4.61** |
| **H1703** | **Amplification** | **WT** | **0.034** | **0.007** | **0.119** | **8.33** | **3.52** | **2.96** |
| **H358** | **WT** | **G12C** | **0.005** | **0.004** | **0.018** | **5.73** | **4.49** | **3.95** |

### Example 6.2 huPBMC ADCC assay

It was found from the above results for the ADCC report assay that the ADCC activity of Hz20G5.26/Zalu bsAb was stronger than that of JNJ372, the EGFR monoclonal antibody Zalu, and the combination of Gp120/Zalu and Gp120/Hz20G5.26. In order to more truly and effectively reflect the ADCC activity of Hz20G5.26/Zalu bsAb, we designed a huPBMC ADCC assay, in which ADCC activity was verified by using normal human PBMC.

### Experimental method:

(1) A CTS medium (Gibco, A3021002) was preheated at 37 °C. huPBMC (Miaotong Biotechnology, PB100C-W) was quickly thawed in a water bath, and then the cells were added slowly to 8 mL of CTS medium (containing 1% DNase).
(2) The mixture was centrifuged at 300 g for 8 min. The supernatant was removed, and the cells were resuspended in 30 mL of CTS (containing 10 µL of DNase), transferred to a T75 flask, and incubated in an incubator at 37 °C overnight.
(3) The suspension cells cultured overnight were taken and centrifuged at 300 g for 8 min. The supernatant was removed, and the cell density was adjusted with CTS. According to an effector/target ratio of effector cells (huPBMCs) to target cells (tumor cells in Table 5) of 50:1, huPBMCs and the tumor cells prepared in advance (Target:huPBMC = 1.5E4/7.5E5 cells/well/100 µL) were plated in a 96-well low-adsorption plate (Corning, CLS7007-24EA).
(4) The diluted antibody drug was added to the corresponding cell well plate and cultured at 37 °C with 5% CO₂ for 8 h.
(5) The mixture was centrifuged at 300 g for 5 min. 50 µL of the supernatant was transferred to a 96-well transparent flat-bottom plate ((NUNC, 136101). 50 µL of the LDH reagent (Promega, G1780) prepared in advance was added to the corresponding well plate, and the plate was left to stand at room temperature for 15-30 min in the dark.
(6) 50 µL LDH stop solution (Promega, G1780) was taken and detected by a multimode microplate reader (Molecular Devices, Spectra MAXi3) at 490 nm for readings.

The results are shown in FIG. 13 and Table 5. The results for ADCC experiment on Hz20G5.26/Zalu bsAb in huPBMC could more truly and effectively reflect the actual efficacy result. It could be found from the huPBMC ADCC results (FIG. 13 and Table 5) that the ADCC activity results of Hz20G5.26/Zalu bsAb in huPBMC were substantially consistent with the reported ADCC activity results. In a number of different NSCLC-EGFR tumor cell lines, the huPBMC ADCC activity of Hz20G5.26/Zalu bsAb was stronger than that of JNJ373 and the EGFR monoclonal antibody, and stronger than that of the combination of Gp120/Zalu and Gp120/Hz20G5.26; through the huPBMC ADCC effect, the anti-tumor killing rate of Hz20G5.26/Zalu bsAb in H1975 was the lowest, but still reached 36.53%; the anti-tumor killing rate of Hz20G5.26/Zalu bsAb in H322 was the highest and reached 100%.

Therefore, on the basis of enhancing EGFR signal blocking, Hz20G5.26/Zalu bsAb could further exert its anti-tumor pharmacodynamic activity in a number of cancer tumors by involving immune cells in ADCC.

**Table 5. Results for huPBMC ADCC experiment on Hz20G5.26/Zalu bsAb**

| **Cell line** | **EGFR State** | **KRAS State** | **EC50(nM)** | | | **Max cytotoxicity (%)** | | |
|---|---|---|---|---|---|---|---|---|
| | | | **bsAb** | **Zalu** | **JNJ372** | **bsAb** | **Zalu** | **JNJ372** |
| **H292** | **WT** | **WT** | **0.002** | **0.001** | **0.004** | **67.32** | **64.71** | **63.44** |
| **H322** | **WT** | **WT** | **0.006** | **0.014** | **0.032** | **100.00** | **77.12** | **76.29** |
| **H1650** | **Amplification** | **WT** | **0.107** | **0.571** | **0.427** | **58.11** | **30.66** | **429.40** |
| | **DeI19** | | | | | | | |
| **H1975** | **L858R** | **WT** | **0.125** | **0.118** | **0.117** | **36.53** | **17.18** | **18.47** |
| | **T790M** | | | | | | | |
| **SK-MES-1** | **Amplification** | **WT** | **0.020** | **0.014** | **0.072** | **58.11** | **51.33** | **47.78** |
| **H1703** | **Amplification** | **WT** | **0.007** | **0.007** | **0.040** | **70.00** | **64.34** | **63.94** |
| **H358** | **WT** | **G12C** | **0.007** | **0.007** | **0.030** | **73.18** | **55.91** | **58.99** |

### Example 7. In-Vitro Study of Drug Safety of Hz20G5.26/Zalu bsAb Bispecific Antibody Molecule

EGFR is an epithelial-derived epidermal growth factor, and in addition to overexpression or abnormal activation leading to cancer tumorigenesis, EGFR expression is found in some epithelial cells and keratinocytes. Relative to the adverse reactions of the EGFR-TKI small molecule inhibitor, such as rash, diarrhea, paronychia, oral mucositis, liver injury, and interstitial lung diseases, the EGFR monoclonal antibody also has certain adverse reactions, mainly manifested as skin toxicity, such as pustular rash, paronychia, and dry and itching skin, which greatly affects the life quality and treatment compliance of tumor patients.

We selected a human cutaneous squamous cell carcinoma cell line (A431, ATCC, CRL-1555) and human skin keratinocytes (HaCat, Cell lines service, 300493) as study models for adverse reactions of Hz20G5.26/Zalu bs Ab, to investigate the tolerance of Hz20G5.26/Zalu bs Ab in an *in-vitro* experiment.

### Experimental method

(1) A431 and HaCat cells were plated at 1500-2000 cells/100 µL in a 96-well low-adsorption plate (Corning, CLS7007-24EA) for 3D cell culture, in which DMEM + 10% FBS + 1% Pen/strep was used as a medium.
(2) The antibody molecules prepared in advance by dilution were added to the corresponding cell well plate, mixed well, and cultured in an incubator at 37 °C with 5% CO₂ for 5 days.
(3) After 5 days of continuous culture for proliferation inhibition assay, the Cell-Titer reagent (Promega, G7572) prepared in advance was added to cell wells, and the plate was left to stand at room temperature for 15-25 min in the dark.
(4) Cell-Titer and the cell mixture were transferred to a 96-well white-bottom plate (NUNC, 136101) and detected by a multimode microplate reader (Molecular Devices, Spectra MAXi3).

The results are shown in FIG. 14. It was found from the experimental results that the *in-vitro* efficacy of the Hz20G5.26/Zalu bs Ab against A431 and HaCat cells was far lower than that of the EGFR monoclonal antibody, indicating that the cells had high tolerance to Hz20G5.26/Zalu bs Ab and the adverse reactions were much lower than those of the EGFR monoclonal antibody; meanwhile, the tolerance of the cells to Hz20G5.26/Zalu bs Ab was superior to that of JNJ372.

Therefore, on the basis of reducing the EGER affinity, the B7H3 high-affinity Hz20G5.26 parent was applied to Hz20G5.26/Zalu bs Ab, so that the pharmacodynamic biological activity and the pharmacodynamic safety window of Hz20G5.26/Zalu bs Ab were improved.

### Example 8. In-Vivo Pharmacodynamic Activity of Hz20G5.26/Zalu bsAb Bispecific Antibody Molecule

In order to demonstrate the *in-vivo* efficacy of the Hz20G5.26/Zalu bispecific antibody molecule, Balb/c Nude mice were inoculated with NCI-H292 cells (ATCC) of an NSCLC-EGFR^{WT} tumor cell line to determine the anti-tumor efficacy of the Hz20G5.26/Zalu bispecific antibody of the present disclosure. SPF female Balb/c Nude mice (purchased from Beijing Vital River Laboratory Animal Technology Co., Ltd.) with certificate No. 110011211108430747 were used in the experiment.

NCI-H292 cells were routinely subcultured for the subsequent *in-vivo* experiment. The cells were collected by centrifugation. Then the NCI-H292 cells were resuspended in an equal-proportion mixture of PBS (1×) and Matrigel Matrix (Corning) to prepare a cell suspension with a cell concentration of 20×10⁶ cells/mL. On day 0, 0.2 mL of the cell suspension was taken and subcutaneously inoculated to the right abdominal area of Balb/c Nude mice to establish NCI-H292 tumor-bearing mouse models.

Four days after the tumor cell inoculation, the tumor volume in each mouse was measured. The mice were grouped (with 5 mice in each group). The dosages and routes of administration are shown in Table 6.

**Table 6. Groups, and dosages and routes of administration in the in vivo experiment**

| Group | Dosage of administration | Administration frequency | Route of administration |
|---|---|---|---|
| h-IgG* | 5 mg/kg | Q3-4D x4 | Intraperitoneal injection |
| JNJ372 | 5 mg/kg | Q3-4D x4 | Intraperitoneal injection |
| Gp120/Zalu | 5 mg/kg | Q3-4D x4 | Intraperitoneal injection |
| Hz20G5.26/Zalu | 5 mg/kg | Q3-4D x4 | Intraperitoneal injection |
| Zalu mAb | 5 mg/kg | Q3-4D x4 | Intraperitoneal injection |

| | | | |
|---|---|---|---|
| *: h-IgG was an isotype control antibody, purchased from Equitech-Bio, lot No. 210112-0356. | | | |

h-IgG, JNJ372, Gp120/Zalu, Hz20G5.26/Zalu, and Zalu mAb were all used at a concentration of 0.5 mg/mL, and were administered once every 3 to 4 days for a total of 4 times (Q3-4D × 4). Administration was performed on days 4, 8, 12, and 15 after the inoculation of NCI-H292 cells. Mice were monitored for tumor volume and body weight twice a week. As shown in FIG. 15A, the monitoring ended after 59 days. Since death of mice due to tumors occurred earlier in some groups, relative tumor growth inhibition (TGI%) was calculated based on the tumor volume on day 22 after the inoculation by the following formula: TGI% = 100% × (control group tumor volume - treatment group tumor volume)/(control group tumor volume - control group tumor volume before administration).

Tumor volume measurement: The maximum length of major axis (L) and maximum length of minor axis (W) of tumors were measured with a vernier caliper, and tumor volume was calculated by the following formula: V = L × W²/2.

The mice were weighed using an electronic balance. Mice with tumor volumes above 2000 mm³ or weight loss above 20% were euthanized.

The tumor growth inhibition results shown in Table 7 showed that on day 22 after the inoculation, the tumor growth inhibition rates of JNJ372, Gp120/Zalu, Hz20G5.26/Zalu, and Zalu mAb were 100%, 72%, 108%, and 107%, respectively, compared with that of the h-IgG, 5 mg/kg group. There was one mouse with complete tumor response in each of the Hz20G5.26/Zalu and Zalu mAb groups. The survival curve of the mice shown in FIG. 15B showed that Hz20G5.26/Zalu could significantly prolong the survival of the mice. In conclusion, the anti-tumor efficacy of Hz20G5.26/Zalu on NCI-H292 tumor-bearing mice is comparable to that of the parental Zalu mAb, and is superior to that of JNJ372 and the non-targeted GP120/Zalu.

Meanwhile, the monitoring results of the body weight of mice (FIG. 15C) showed that there was no significant decrease in the body weight of the mice in the Hz20G5.26/Zalu group 59 days after the inoculation.

**Table 7. Anti-tumor efficacy statistics**

| Group | Tumor volume (mm³) | Tumor growth inhibition (%) @ Day22 | Complete response rate* @ Day59 |
|---|---|---|---|
| h-IgG | 1427 | N/A | 0/5 |
| JNJ372 | 177 | 100 | 0/5 |
| Gp120/Zalu | 532 | 72 | 0/5 |
| Hz20G5.26/Zalu | 76 | 108 | 1/5 |
| Zalu mAb | 96 | 107 | 1/5 |

| | | | |
|---|---|---|---|
| *Complete response rate: the tumor was completely regressed with a tumor volume of 0. | | | |

In order to demonstrate the *in-vivo* efficacy of the Hz20G5.26/Zalu bispecific antibody molecule against EGFR abnormally amplified NSCLC, Balb/c Nude mice were inoculated with SK-MES-1 cells (CoBioer) to determine the anti-tumor efficacy of the Hz20G5.26/Zalu bispecific antibody molecule of the present disclosure. SPF female Balb/c Nude mice (purchased from Beijing Vital River Laboratory Animal Technology Co., Ltd.) with certificate No. 110011211108966881 were used in the experiment.

SK-MES-1 cells were routinely subcultured for the subsequent *in-vivo* experiment. The cells were collected by centrifugation. Then the SK-MES-1 cells were resuspended in an equal-proportion mixture of PBS (1×) and Matrigel Matrix (Corning) to prepare a cell suspension with a cell concentration of 20×10⁶ cells/mL. On day 0, 0.2 mL of the cell suspension was taken and subcutaneously inoculated to the right abdominal area of Balb/c Nude mice to establish SK-MES-1 tumor-bearing mouse models.

Six days after the tumor cell inoculation, the tumor volume in each mouse was measured. The mice were grouped (with 7 mice in each group). The dosages and routes of administration are shown in Table 8.

**Table 8. Groups, and dosages and routes of administration in the in vivo experiment**

| Group | Dosage of administration | Administration frequency | Route of administration |
|---|---|---|---|
| h-IgG* | 1 mg/kg | Single dose | Intraperitoneal injection |
| Hz20G5.26/Zalu | 1 mg/kg | Single dose | Intraperitoneal injection |
| JNJ372 | 1 mg/kg | Single dose | Intraperitoneal injection |
| Zalu mAb | 1 mg/kg | Single dose | Intraperitoneal injection |
| Gp120/Zalu | 1 mg/kg | Single dose | Intraperitoneal injection |

| | | | |
|---|---|---|---|
| *: h-IgG was an isotype control antibody, purchased from Equitech-Bio, lot No. 210112-0356. | | | |

h-IgG, Hz20G5.26/Zalu, JNJ372, Zalu mAb and Gp120/Zalu were all used at a concentration of 0.1 mg/mL. Administration was performed once on day 6 after the inoculation of SK-MES-1 cells. Mice were monitored for tumor volume and body weight twice a week. As shown in FIG. 16A, the monitoring ended after 24 days.

On day 24 after inoculation, the relative tumor growth inhibition (TGI%) was calculated by the following formula: TGI% = 100% × (control group tumor volume - treatment group tumor volume)/(control group tumor volume - control group tumor volume before administration).

Tumor volume measurement: The maximum length of major axis (L) and maximum length of minor axis (W) of tumors were measured with a vernier caliper, and tumor volume was calculated by the following formula: V = L × W²/2. The mice were weighed using an electronic balance.

The tumor growth inhibition results shown in Table 9 showed that on day 24 after the inoculation, the tumor growth inhibition rates of Hz20G5.26/Zalu, JNJ372, Zalu mAb, and Gp120/Zalu were 143%, 122%, 145%, and 113%, respectively, compared with that of the h-IgG, 1 mg/kg group. Meanwhile, the monitoring results of the body weight of mice (FIG. 16B) showed that there was no significant difference in the body weight of the mice 24 days after the inoculation.

**Table 9. Anti-tumor efficacy statistics on day 24**

| Group | Tumor volume (mm³) | Tumor growth inhibition (%) | Complete response rate* |
|---|---|---|---|
| h-IgG | 419 | N/A | 0/7 |
| Hz20G5.26/Zalu | 28 | 143 | 2/7 |
| JNJ372 | 87 | 122 | 0/7 |
| Zalu mAb | 23 | 145 | 2/7 |
| Gp120/Zalu | 112 | 113 | 0/7 |

| | | | |
|---|---|---|---|
| *Complete response rate: the tumor was completely regressed with a tumor volume of 0. | | | |

To further demonstrate the advantages of the Hz20G5.26/Zalu molecule as the bispecific antibody in terms of *in-vivo* efficacy, Balb/c Nude mice were inoculated with SK-MES-1 cells (CoBioer) to determine the anti-tumor efficacy of the Hz20G5.26/Zalu bispecific antibody molecule of the present disclosure. SPF female Balb/c Nude mice (purchased from Beijing Vital River Laboratory Animal Technology Co., Ltd.) with certificate No. 110011221103705231 were used in the experiment.

SK-MES-1 cells were routinely subcultured for the subsequent *in-vivo* experiment. The cells were collected by centrifugation. Then the SK-MES-1 cells were resuspended in an equal-proportion mixture of PBS (1×) and Matrigel Matrix (Corning) to prepare a cell suspension with a cell concentration of 25×10⁶ cells/mL. On day 0, 0.2 mL of the cell suspension was taken and subcutaneously inoculated to the right abdominal area of Balb/c Nude mice to establish SK-MES-1 tumor-bearing mouse models.

14 days after the tumor cell inoculation, the tumor volume in each mouse was measured. The mice were grouped (with 6 mice in each group). The dosages and routes of administration are shown in Table 10.

**Table 10. Groups, and dosages and routes of administration in the in vivo experiment**

| Group | Dosage of administration | Administration frequency | Route of administration |
|---|---|---|---|
| h-IgG* | 0.6 mg/kg | Single dose | Intraperitoneal injection |
| Hz20G5.26/Zalu | 0.3 mg/kg | Single dose | Intraperitoneal injection |
| Gp120/Zalu | 0.3 mg/kg | Single dose | Intraperitoneal injection |
| Gp120/Hz20G5.26 | 0.3 mg/kg | Single dose | Intraperitoneal injection |
| Gp120/Zalu + Gp120/Hz20G5.26 | 0.3 + 0.3 mg/kg | Single dose | Intraperitoneal injection |

| | | | |
|---|---|---|---|
| *: h-IgG was an isotype control antibody, purchased from Equitech-Bio, lot No. 210112-0356. | | | |

h-IgG was used at a concentration of 0.06 mg/mL, and Hz20G5.26/Zalu, Gp120/Zalu, and Gp120/Hz20G5.26 were all used at a concentration of 0.03 mg/mL. Administration was performed once on day 14 after the inoculation of SK-MES-1 cells. Mice were monitored for tumor volume and body weight twice a week. As shown in FIG. 17A, the monitoring ended after 35 days.

On day 35 after inoculation, the relative tumor growth inhibition (TGI%) was calculated by the following formula: TGI% = 100% × (control group tumor volume - treatment group tumor volume)/(control group tumor volume - control group tumor volume before administration).

Tumor volume measurement: The maximum length of major axis (L) and maximum length of minor axis (W) of tumors were measured with a vernier caliper, and tumor volume was calculated by the following formula: V = L × W²/2. The mice were weighed using an electronic balance.

The tumor growth inhibition results shown in Table 11 showed that on day 35 after the inoculation, the tumor growth inhibition rates of Hz20G5.26/Zalu, Gp120/Zalu, Gp120/Hz20G5.26, and Gp120/Zalu + Gp120/Hz20G5.26 were 121%, 31%, 1%, and 16%, respectively, compared with that of the h-IgG, 0.6 mg/kg group. The anti-tumor efficacy of Hz20G5.26/Zalu was superior to that of the control non-targeted monoclonal antibody Gp120/Zalu, Gp120/Hz20G5.26, or the combination of the two, demonstrating the unique mechanism of Hz20G5.26/Zalu serving as the bispecific antibody, namely the pulling effect of Hz20G5.26 on the blocking of EGFR signals by zalu. Meanwhile, the monitoring results of the body weight of mice (FIG. 17B) showed that there was no significant difference in the body weight of the mice 24 days after the inoculation.

**Table 11. Anti-tumor efficacy statistics on day 35**

| Group | Tumor volume (mm³) | Tumor growth inhibition (%) |
|---|---|---|
| h-IgG | 483 | N/A |
| Hz20G5.26/Zalu | 179 | 121 |
| Gp120/Zalu | 404 | 31 |
| Gp120/Hz20G5.26 | 481 | 1 |
| Gp120/Zalu + Gp120/Hz20G5.26 | 443 | 16 |

### Example 9. In-Vivo Pharmacodynamic Activity of Combination of Hz20G5.26/Zalu bsAb Bispecific Antibody Molecule and KRAS Small Molecule Inhibitor

In order to demonstrate the *in-vivo* efficacy of the combination of the Hz20G5.26/Zalu bispecific antibody molecule and a KRAS small molecule inhibitor, NOG mice were inoculated with an NSCLC tumor cell line NCI-H358 (CoBioer) with a KRAS^{G12C} mutation to determine the anti-tumor efficacy of the combination of the Hz20G5.26/Zalu bispecific antibody of the present disclosure and a KRAS small molecule inhibitor AMG510. SPF female NOG mice (purchased from Beijing Vital River Laboratory Animal Technology Co., Ltd.) with certificate No. 1100112211001153625 were used in the experiment.

NCI-H358 cells were routinely subcultured for the subsequent *in-vivo* experiment. The cells were collected by centrifugation. Then the NCI-H358 cells were resuspended in an equal-proportion mixture of PBS (1×) and Matrigel Matrix (Corning) to prepare a cell suspension with a cell concentration of 25×10⁶ cells/mL. On day 0, 0.2 mL of the cell suspension was taken and subcutaneously inoculated to the right abdominal area of NOG mice to establish NCI-H358 tumor-bearing mouse models.

Six days after the tumor cell inoculation, the tumor volume in each mouse was measured. The mice were grouped (with 7 mice in each group). The dosages and routes of administration are shown in Table 12.

**Table 12. Groups, and dosages and routes of administration in the in vivo experiment**

| Group | Dosage of administration | Administration frequency | Route of administration |
|---|---|---|---|
| h-IgG* | 20 mg/kg | Q3-4D x4 | Intraperitoneal injection |
| AMG510 | 10 mg/kg | QD x14 | Intragastric administration |
| Hz20G5.26/Zalu | 10 mg/kg | Q3-4D x4 | Intraperitoneal injection |
| AMG510 + Hz20G5.26/Zalu | 10 + 10 mg/kg | QD x14 + Q3-4D x4 | Intragastric administration + intraperitoneal injection |
| JNJ372 | 10 mg/kg | Q3-4D x4 | Intraperitoneal injection |
| Zalu mAb | 10 mg/kg | Q3-4D x4 | Intraperitoneal injection |

| | | | |
|---|---|---|---|
| *: h-IgG was an isotype control antibody, purchased from Equitech-Bio, lot No. 210112-0356. | | | |

h-IgG was used at a concentration of 2 mg/mL, and Hz20G5.26/Zalu, JNJ372, and Zalu mAb were all used at a concentration of 1 mg/mL, and they were administered once every 3 to 4 days for a total of 4 times (Q3-4D × 4). Administration was performed on days 6, 9, 12, and 15 after the inoculation of NCI-H358 cells. Mice were monitored for tumor volume and body weight twice a week. AMG510 was used at a concentration of 1 mg/mL, and was administered once every day for a total of 14 times (QD × 14), starting on day 6 after the inoculation of NCI-H358 cells. Mice were monitored for tumor volume twice a week, and were monitored for body weight every day during the administration, and twice a week after the end of administration. As shown in FIG. 18A, the monitoring ended after 33 days. On day 33 after the inoculation, the relative tumor growth inhibition (TGI%) was calculated by the following formula: TGI% = 100% × (control group tumor volume - treatment group tumor volume)/(control group tumor volume - control group tumor volume before administration).

Tumor volume measurement: The maximum length of major axis (L) and maximum length of minor axis (W) of tumors were measured with a vernier caliper, and tumor volume was calculated by the following formula: V = L × W²/2. The mice were weighed using an electronic balance.

The tumor growth inhibition results shown in Table 13 showed that on day 33 after the inoculation, the tumor growth inhibition rates of AMG510, Hz20G5.26/Zalu, AMG510 + Hz20G5.26/Zalu, JNJ372, and Zalu mAb were 89%, 110%, 130%, 82%, and 111%, respectively, compared with that of the h-IgG, 20 mg/kg group. In each group, the combination of Hz20G5.26/Zalu and AMG510 had the optimal anti-tumor effect on NCI-H358 tumor-bearing mice and had a synergistic effect.

Meanwhile, the monitoring results of the body weight of mice (FIG. 18B) showed that the administration of AMG510 caused a gradual decrease in the body weight of mice. After the suspension of administration, the body weight of mice recovered, and the body weight of mice in all groups was normal on day 33.

**Table 13. Tumor growth inhibition on day 33**

| Group | Tumor volume (mm³) | Tumor growth inhibition (%) |
|---|---|---|
| h-IgG* | 400 | N/A |
| AMG510 | 180 | 89 |
| Hz20G5.26/Zalu | 128 | 110 |
| AMG510 + Hz20G5.26/Zalu | 78 | 130 |
| JNJ372 | 196 | 82 |
| Zalu mAb | 126 | 111 |

### Sequence listing: sequence information of anti-B7-H3/EGFR bispecific antibodies

| Name | Sequence No. | Sequence |
|---|---|---|
| VH of zalutumumab antibody | SEQ ID NO:1 | |
| VL of zalutumumab antibody | SEQ ID NO:2 | |
| VH of Hz20G5.26 antibody | SEQ ID NO:3 | QVQLVQSGAEVKKPGASVKVSCKASGYTFTEYIMHWVRQ APGQRLEWMGGINPGTGGTTYNQKFKDRVTITVDTSASTA YMELSSLRSEDTAVYYCTRRTPPWHFAVWGQGTLVTVSS |
| VL of Hz20G5.26 antibody | SEQ ID NO:4 | DIQLTQSPSFLSASVGDRVTITCSASSSVSYIHWYQQKPGKA PKRWIYDTSRLASGVPSRFSGSGSGTEFTLTISSLQPEDFATY YCQQWSSAPLTFGGGTKVEIK |
| VH of Hz19A2.25 antibody | SEQ ID NO:5 | QVQLVQSGAEVKKPGASVKVSCKASGYIFTSYWIHWVRQ APGQGLEWMGRIYPGTESTFYNEKFKGRVTMTRDTSTST VYMELSSLRSEDTAVYYCHFITASDWYFDVWGQGTLVTVS S |
| VL of Hz19A2.25 antibody | SEQ ID NO:6 | EIVLTQSPATLSLSPGERATLSCSVSSSVQSNYLYWYQQKPG QAPRLLIYGTSNLASGIPARFSGSGSGTDFTLTISSLEPEDFAV YYCYQWSSYPFTFGQGTKLEIK |
| VH of Hz5C2.9 antibody | SEQ ID NO:7 | QVQLVQSGAEVKKPGSSVKVSCKASGYTFSDYVISWVRQA PGQGLEWMGEIYPRGGIIHYNEKFKARVTITADKSTSTAY MELSSLRSEDTAVYYCARLWDWYFDVWGQGTLVTVSS |
| VL of Hz5C2.9 antibody | SEQ ID NO:8 | EIVLTQSPGTLSLSPGERATLSCRSSQSLVHSQGITYLDWYQ |
| | | QKPGQAPRLLIYKVSNRFSGIPDRFSGSGSGTDFTLTISRLEP EDFAVYYCSQGTHVPPWTFGGGTKVEIK |
| HCDR1 of zalutumumab antibody | SEQ ID NO:9 | TYGMH(Kabat scheme) |
| HCDR2 of zalutumumab antibody | SEQ ID NO:10 | VIWDDGSYKYYGDSVKG(Kabat scheme) |
| HCDR3 of zalutumumab antibody | SEQ ID NO:11 | DGITMVRGVMKDY(Kabat scheme) |
| LCDR1 of zalutumumab antibody | SEQ ID NO:12 | RASQDISSALV(Kabat scheme) |
| LCDR2 of zalutumumab antibody | SEQ ID NO:13 | DASSLES(Kabat scheme) |
| LCDR3 of zalutumumab antibody | SEQ ID NO:14 | QQFNSYPLT(Kabat scheme) |
| HCDR1 of Hz20G5.26 antibody | SEQ ID NO:15 | GYTFTEYIMH(AbM scheme) |
| HCDR2 of Hz20G5.26 antibody | SEQ ID NO:16 | GINPGTGGTTYNQKFKD(Kabat scheme) |
| HCDR3 of Hz20G5.26 antibody | SEQ ID NO:17 | RTPPWHFAV(Kabat scheme) |
| LCDR1 of Hz20G5.26 antibody | SEQ ID NO:18 | SASSSVSYIH(Kabat scheme) |
| LCDR2 of Hz20G5.26 antibody | SEQ ID NO:19 | DTSRLAS(Kabat scheme) |
| LCDR3 of Hz20G5.26 antibody | SEQ ID NO:20 | QQWSSAPLT(Kabat scheme) |
| HCDR1 of Hz19A2.25 antibody | SEQ ID NO:21 | GYIFTSYWIH(AbM scheme) |
| HCDR2 of Hz19A2.25 antibody | SEQ ID NO:22 | RIYPGTESTFYNEKFKG(Kabat scheme) |
| HCDR3 of Hz19A2.25 antibody | SEQ ID NO:23 | ITASDWYFDV(Kabat scheme) |
| LCDR1 of Hz19A2.25 antibody | SEQ ID NO:24 | SVSSSVQSNYLY(Kabat scheme) |
| LCDR2 of Hz19A2.25 antibody | SEQ ID NO:25 | GTSNLAS(Kabat scheme) |
| LCDR3 of Hz19A2.25 antibody | SEQ ID NO:26 | YQWSSYPFT(Kabat scheme) |
| HCDR1 of Hz5C2.9 antibody | SEQ ID NO:27 | GYTFSDYVIS(AbM scheme) |
| HCDR2 of Hz5C2.9 antibody | SEQ ID NO:28 | EIYPRGGIIHYNEKFKA(Kabat scheme) |
| HCDR3 of Hz5C2.9 antibody | SEQ ID NO:29 | ARLWDWYFDV(Kabat scheme) |
| LCDR1 of Hz5C2.9 antibody | SEQ ID NO:30 | RSSQSLVHSQGITYLD(Kabat scheme) |
| LCDR2 of Hz5C2.9 antibody | SEQ ID NO:31 | KVSNRFS(Kabat scheme) |
| LCDR3 of Hz5C2.9 antibody | SEQ ID NO:32 | SQGTHVPPWT(Kabat scheme) |
| HC of peptide chain #1 of anti-HZ205.26/Z alu bispecific antibody | SEQ ID NO:33 | QVQLVESGGGVVQPGRSLRLSCAASGFTFSTYGMHWVRQA PGKGLEWVAVIWDDGSYKYYGDSVKGRFTISRDNSKNTLY LQMNSLRAEDTAVYYCARDGITMVRGVMKDYFDYWGQGT LVTVSSASTKGPSVFPLAPSSKSTSGGTAALGCLVKDYFPEP VTVSWNSGALTSGVHTFPAVLQSSGLYSLSSVVTVPSSSLGT QTYICNVNHKPSNTKVDKKVEPKSCDKTHTCPPCPAPELLG GPSVFLFPPKPKDTLMISRTPEVTCVVVDVSHEDPEVKFNW YVDGVEVHNAKTKPREEQYNSTYRVVSVLTVLHQDWLNG KEYKCKVSNKALPAPIEKTISKAKGQPREPQVTTLPPSRDEL TKNQVSLTCLVSGFYPSDIAVEWESNGQPENNYKTTPPVLDS DGSFFLYSDLTVDKSRWQQGNVFSCSVMHEALHNHYTQKS LSLSPGK |
| LC of peptide chain #1 of anti-HZ205.26/Z alu bispecific antibody | SEQ ID NO:34 | AIQLTQSPSSLSASVGDRVTITCRASQDISSALVWYQQKPGK APKLLIYDASSLESGVPSRFSGSESGTDFTLTISSLQPEDFATY YCQQFNSYPLTFGGGTKVEIKRTVAAPSVFIFPPSDEQLKSGT ASVVCLLNNFYPREAKVQWKVDNALQSGNSQESVTEQDSK DSTYSLSSTLTLSKADYEKHKVYACEVTHQGLSSPVTKSFN RGEC |
| HC of peptide chain #2 of anti-HZ205.26/Z alu bispecific antibody | SEQ ID NO:35 | QVQLVQSGAEVKKPGASVKVSCKASGYTFTEYIMHWVRQ APGQRLEWMGGINPGTGGTTYNQKFKDRVTITVDTSASTAY MELSSLRSEDTAVYYCTRRTPPWHFAVWGQGTLVTVSSAST KGPSVFPLAPSSKSTSGGTAALGCLVKDYFPEPVTVSWNSG ALTSGVHTFPAVLQSSGLYSLSSVVTVPSSSLGTQTYICNVN HKPSNTKVDKKVEPKSCDKTHTCPPCPAPELLGGPSVFLFPP KPKDTLMISRTPEVTCVVVDVSHEDPEVKFNWYVDGVEVH NAKTKPREEQYNSTYRVVSVLTVLHQDWLNGKEYKCKVS NKALPAPIEKTISKAKGQPREPQVYTLPPSRDELTKNQVRLT CLVKGFYPSDIAVEWESNGQPENNYKTTPPVLKSDGSFFLYS KLTVDKSRWQQGNVFSCSVMHEALHNHYTQKSLSLSPGK |
| LC of peptide chain #2 of anti-HZ205.26/Z alu bispecific antibody | SEQ ID NO:36 | DIQLTQSPSFLSASVGDRVTITCSASSSVSYIHWYQQKPGKA PKRWIYDTSRLASGVPSRFSGSGSGTEFTLTISSLQPEDFATY |
| | | YCQQWSSAPLTFGGGTKVEIKRTVAAPSVFIFPPSDEQLKSG TASVVCLLNNFYPREAKVQWKVDNALQSGNSQESVTEQDS KDSTYSLSSTLTLSKADYEKHKVYACEVTHQGLSSPVTKSF NRGEC |
| HC of peptide chain #1 of anti-Hz19A2.25/ Zalu bispecific antibody | SEQ ID NO:33 | QVQLVESGGGVVQPGRSLRLSCAASGFTFSTYGMHWVRQA PGKGLEWVAVIWDDGSYKYYGDSVKGRFTISRDNSKNTLY LQMNSLRAEDTAVYYCARDGITMVRGVMKDYFDYWGQGT LVTVSSASTKGPSVFPLAPSSKSTSGGTAALGCLVKDYFPEP VTVSWNSGALTSGVHTFPAVLQSSGLYSLSSVVTVPSSSLGT QTYICNVNHKPSNTKVDKKVEPKSCDKTHTCPPCPAPELLG GPSVFLFPPKPKDTLMISRTPEVTCVVVDVSHEDPEVKFNW YVDGVEVHNAKTKPREEQYNSTYRVVSVLTVLHQDWLNG KEYKCKVSNKALPAPIEKTISKAKGQPREPQVTTLPPSRDEL TKNQVSLTCLVSGFYPSDIAVEWESNGQPENNYKTTPPVLDS DGSFFLYSDLTVDKSRWQQGNVFSCSVMHEALHNHYTQKS LSLSPGK |
| LC of peptide chain #1 of anti-Hz19A2.25/ Zalu bispecific antibody | SEQ ID NO:34 | AIQLTQSPSSLSASVGDRVTITCRASQDISSALVWYQQKPGK APKLLIYDASSLESGVPSRFSGSESGTDFTLTISSLQPEDFATY YCQQFNSYPLTFGGGTKVEIKRTVAAPSVFIFPPSDEQLKSGT ASVVCLLNNFYPREAKVQWKVDNALQSGNSQESVTEQDSK DSTYSLSSTLTLSKADYEKHKVYACEVTHQGLSSPVTKSFN RGEC |
| HC of peptide chain #2 of anti-Hz19A2.25/ Zalu bispecific antibody | SEQ ID NO:37 | QVQLVQSGAEVKKPGASVKVSCKASGYIFTSYWIHWVRQA PGQGLEWMGRIYPGTESTFYNEKFKGRVTMTRDTSTSTVY MELSSLRSEDTAVYYCHFITASDWYFDVWGQGTLVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKKVEPKSCDKTHTCPPCPAPELLGGPSVFLFP PKPKDTLMISRTPEVTCVVVDVSHEDPEVKFNWYVDGVEV HNAKTKPREEQYNSTYRVVSVLTVLHQDWLNGKEYKCKV SNKALPAPIEKTISKAKGQPREPQVYTLPPSRDELTKNQVRL TCLVKGFYPSDIAVEWESNGQPENNYKTTPPVLKSDGSFFLY SKLTVDKSRWQQGNVFSCSVMHEALHNHYTQKSLSLSPGK |
| LC of peptide chain #2 of anti-Hz19A2.25/ Zalu bispecific antibody | SEQ ID NO:38 | EIVLTQSPATLSLSPGERATLSCSVSSSVQSNYLYWYQQKPG QAPRLLIYGTSNLASGIPARFSGSGSGTDFTLTISSLEPEDFAV YYCYQWSSYPFTFGQGTKLEIKRTVAAPSVFIFPPSDEQLKS GTASVVCLLNNFYPREAKVQWKVDNALQSGNSQESVTEQD SKDSTYSLSSTLTLSKADYEKHKVYACEVTHQGLSSPVTKSF |
| | | NRGEC |
| HC of peptide chain #1 of anti-Hz5C2.9/Za lu bispecific antibody | SEQ ID NO:33 | QVQLVESGGGVVQPGRSLRLSCAASGFTFSTYGMHWVRQA PGKGLEWVAVIWDDGSYKYYGDSVKGRFTISRDNSKNTLY LQMNSLRAEDTAVYYCARDGITMVRGVMKDYFDYWGQGT LVTVSSASTKGPSVFPLAPSSKSTSGGTAALGCLVKDYFPEP VTVSWNSGALTSGVHTFPAVLQSSGLYSLSSVVTVPSSSLGT QTYICNVNHKPSNTKVDKKVEPKSCDKTHTCPPCPAPELLG GPSVFLFPPKPKDTLMISRTPEVTCVVVDVSHEDPEVKFNW YVDGVEVHNAKTKPREEQYNSTYRVVSVLTVLHQDWLNG KEYKCKVSNKALPAPIEKTISKAKGQPREPQVTTLPPSRDEL TKNQVSLTCLVSGFYPSDIAVEWESNGQPENNYKTTPPVLDS DGSFFLYSDLTVDKSRWQQGNVFSCSVMHEALHNHYTQKS LSLSPGK |
| LC of peptide chain #1 of Hz5C2.9/Zalu bispecific antibody | SEQ ID NO:34 | AIQLTQSPSSLSASVGDRVTITCRASQDISSALVWYQQKPGK APKLLIYDASSLESGVPSRFSGSESGTDFTLTISSLQPEDFATY YCQQFNSYPLTFGGGTKVEIKRTVAAPSVFIFPPSDEQLKSGT ASVVCLLNNFYPREAKVQWKVDNALQSGNSQESVTEQDSK DSTYSLSSTLTLSKADYEKHKVYACEVTHQGLSSPVTKSFN RGEC |
| HC of peptide chain #2 of Hz5C2.9/Zalu bispecific antibody | SEQ ID NO:39 | QVQLVQSGAEVKKPGSSVKVSCKASGYTFSDYVISWVRQA PGQGLEWMGEIYPRGGIIHYNEKFKARVTITADKSTSTAYME LSSLRSEDTAVYYCARLWDWYFDVWGQGTLVTVSSASTKG PSVFPLAPSSKSTSGGTAALGCLVKDYFPEPVTVSWNSGALT SGVHTFPAVLQSSGLYSLSSVVTVPSSSLGTQTYICNVNHKP SNTKVDKKVEPKSCDKTHTCPPCPAPELLGGPSVFLFPPKPK DTLMISRTPEVTCVVVDVSHEDPEVKFNWYVDGVEVHNAK TKPREEQYNSTYRVVSVLTVLHQDWLNGKEYKCKVSNKAL PAPIEKTISKAKGQPREPQVYTLPPSRDELTKNQVRLTCLVK GFYPSDIAVEWESNGQPENNYKTTPPVLKSDGSFFLYSKLTV DKSRWQQGNVFSCSVMHEALHNHYTQKSLSLSPGK |
| LC of peptide chain #2 of Hz5C2.9/Zalu bispecific antibody | SEQ ID NO:40 | EIVLTQSPGTLSLSPGERATLSCRSSQSLVHSQGITYLDWYQQ KPGQAPRLLIYKVSNRFSGIPDRFSGSGSGTDFTLTISRLEPE DFAVYYCSQGTHVPPWTFGGGTKVEIKRTVAAPSVFIFPPSD EQLKSGTASVVCLLNNFYPREAKVQWKVDNALQSGNSQES VTEQDSKDSTYSLSSTLTLSKADYEKHKVYACEVTHQGLSS PVTKSFNRGEC |
| RMD enzyme | SEQ ID NO:41 | MTQRLFVTGLSGFVGKHLQAYLAAAHTPWALLPVPHRYDL |
| | | LEPDSLGDLWPELPDAVIHLAGQTYVPEAFRDPARTLQINLL GTLNLLQALKARGFSGTFLYISSGDVYGQVAEAALPIHEELI PHPRNPYAVSKLAAESLCLQWGITEGWRVLVARPFNHIGPG QKDSFVIASAARQIARMKQGLQANRLEVGDIDVSRDFLDV QDVLSAYLRLLSHGEAGAVYNVCSGQEQKIRELIELLADIAQ VELEIVQDPARMRRAEQRRVRGSHARLHDTTGWKPEITIKQ SLRAILSDWESRVREE |
| Wild-type IgG1-CH1 | SEQ ID NO:42 | ASTKGPSVFPLAPSSKSTSGGTAALGCLVKDYFPEPVTVSW NSGALTSGVHTFPAVLQSSGLYSLSSVVTVPSSSLGTQTYICN VNHKPSNTKVDKKV |
| Hinge region | SEQ ID NO:43 | EPKSCDKTHTCPPCP |
| Wild-type IgG1 CH2 | SEQ ID NO:44 | APELLGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAK |
| Wild-type IgG1 CH3 | SEQ ID NO:45 | GQPREPQVYTLPPSRDELTKNQVSLTCLVKGFYPSDIAVEWE SNGQPENNYKTTPPVLDSDGSFFLYSKLTVDKSRWQQGNVF SCSVMHEALHNHYTQKSLSLSPGK |
| Wild-type IgG1-Fc | SEQ ID NO:46 | APELLGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVYTLP PSRDELTKNQVSLTCLVKGFYPSDIAVEWESNGQPENNYKT TPPVLDSDGSFFLYSKLTVDKSRWQQGNVFSCSVMHEALHN HYTQKSLSLSPGK |
| CH2 + CH3 (without hinge region) | | |
| Wild-type IgG1-Fc | SEQ ID NO:47 | EPKSCDKTHTCPPCPAPELLGGPSVFLFPPKPKDTLMISRTPE VTCVVVDVSHEDPEVKFNWYVDGVEVHNAKTKPREEQYN STYRVVSVLTVLHQDWLNGKEYKCKVSNKALPAPIEKTISK AKGQPREPQVYTLPPSRDELTKNQVSLTCLVKGFYPSDIAVE WESNGQPENNYKTTPPVLDSDGSFFLYSKLTVDKSRWQQG NVFSCSVMHEALHNHYTQKSLSLSPGK |
| CH2 + CH3 (with hinge region) | | |
| CH3 mutations (CH3 of the Fc moiety linked to the EGFR antigen-binding region), K370S, Y349T, and K409D | SEQ ID NO:48 | GQPREPQVTTLPPSRDELTKNQVSLTCLVSGFYPSDIAVEWE SNGQPENNYKTTPPVLDSDGSFFLYSDLTVDKSRWQQGNVF SCSVMHEALHNHYTQKSLSLSPGK |
| Fc region (without hinge region) with K370S, Y349T, and K409D | SEQ ID NO:49 | APELLGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVTTLP PSRDELTKNQVSLTCLVSGFYPSDIAVEWESNGQPENNYKTT PPVLDSDGSFFLYSDLTVDKSRWQQGNVFSCSVMHEALHN HYTQKSLSLSPGK |
| Fc region (with hinge region) with K370S, Y349T, and K409D | SEQ ID NO:50 | EPKSCDKTHTCPPCPAPELLGGPSVFLFPPKPKDTLMISRTPE VTCVVVDVSHEDPEVKFNWYVDGVEVHNAKTKPREEQYN STYRVVSVLTVLHQDWLNGKEYKCKVSNKALPAPIEKTISK AKGQPREPQVTTLPPSRDELTKNQVSLTCLVSGFYPSDIAVE WESNGQPENNYKTTPPVLDSDGSFFLYSDLTVDKSRWQQG NVFSCSVMHEALHNHYTQKSLSLSPGK |
| CH3 comprising S364R and D399K | SEQ ID NO:51 | GQPREPQVYTLPPSRDELTKNQVRLTCLVKGFYPSDIAVEWE SNGQPENNYKTTPPVLKSDGSFFLYSKLTVDKSRWQQGNVF SCSVMHEALHNHYTQKSLSLSPGK |
| Fc region (without hinge region) with S364R and D399K | SEQ ID NO:52 | APELLGGPSVFLFPPKPKDTLMISRTPEVTCVVVDVSHEDPE VKFNWYVDGVEVHNAKTKPREEQYNSTYRVVSVLTVLHQ DWLNGKEYKCKVSNKALPAPIEKTISKAKGQPREPQVYTLP PSRDELTKNQVRLTCLVKGFYPSDIAVEWESNGQPENNYKT TPPVLKSDGSFFLYSKLTVDKSRWQQGNVFSCSVMHEALHN HYTQKSLSLSPGK |
| Fc region (with hinge region) with S364R and D399K | SEQ ID NO:53 | EPKSCDKTHTCPPCPAPELLGGPSVFLFPPKPKDTLMISRTPE VTCVVVDVSHEDPEVKFNWYVDGVEVHNAKTKPREEQYN STYRVVSVLTVLHQDWLNGKEYKCKVSNKALPAPIEKTISK AKGQPREPQVYTLPPSRDELTKNQVRLTCLVKGFYPSDIAVE WESNGQPENNYKTTPPVLKSDGSFFLYSKLTVDKSRWQQG NVFSCSVMHEALHNHYTQKSLSLSPGK |
| Light chain constant region | SEQ ID NO:54 | |

## Claims

1. A bispecific antibody binding to EGFR and B7-H3, comprising a first antigen-binding region and a second antigen-binding region, wherein the first antigen-binding region specifically binds to EGFR, and the second antigen-binding region specifically binds to B7H3.

2. The bispecific antibody according to claim 1, wherein the second antigen-binding region comprises sequences of an HCDR1, an HCDR2, and an HCDR3 of a heavy chain variable region set forth in SEQ ID NO: 3, 5, or 7, and sequences of an LCDR1, an LCDR2, and an LCDR3 of a light chain variable region set forth in SEQ ID NO: 4, 6, or 8.

3. The bispecific antibody according to claim 1, wherein the second antigen-binding region comprises an HCDR1, an HCDR2, and an HCDR3 of a heavy chain variable region VH, and an LCDR1, an LCDR2, and an LCDR3 of a light chain variable region VL, wherein
(i) the HCDR1, the HCDR2, and the HCDR3 are three complementarity determining regions HCDR1, HCDR2, and HCDR3 comprised in a VH set forth in SEQ ID NO: 3; and the LCDR1, the LCDR2, and the LCDR3 are three complementarity determining regions LCDR1, LCDR2, and LCDR3 comprised in a VL set forth in SEQ ID NO: 4;
(ii) the HCDR1, the HCDR2, and the HCDR3 are three complementarity determining regions HCDR1, HCDR2, and HCDR3 comprised in a VH set forth in SEQ ID NO: 5; and the LCDR1, the LCDR2, and the LCDR3 are three complementarity determining regions LCDR1, LCDR2, and LCDR3 comprised in a VL set forth in SEQ ID NO: 6; or
(iii) the HCDR1, the HCDR2, and the HCDR3 are three complementarity determining regions HCDR1, HCDR2, and HCDR3 comprised in a VH set forth in SEQ ID NO: 7; and the LCDR1, the LCDR2, and the LCDR3 are three complementarity determining regions LCDR1, LCDR2, and LCDR3 comprised in a VL set forth in SEQ ID NO: 8.

4. The bispecific antibody according to claim 1, wherein the second antigen-binding region comprises an HCDR1, an HCDR2, and an HCDR3 of a heavy chain variable region VH, and an LCDR1, an LCDR2, and an LCDR3 of a light chain variable region VL, wherein
(i) the HCDR1 comprises or consists of an amino acid sequence set forth in SEQ ID NO: 15, the HCDR2 comprises or consists of an amino acid sequence set forth in SEQ ID NO: 16, and the HCDR3 comprises or consists of an amino acid sequence set forth in SEQ ID NO: 17;
the LCDR1 comprises or consists of an amino acid sequence set forth in SEQ ID NO: 18, the LCDR2 comprises or consists of an amino acid sequence set forth in SEQ ID NO: 19, and the LCDR3 comprises or consists of an amino acid sequence set forth in SEQ ID NO: 20; or,
(ii) the HCDR1 comprises or consists of an amino acid sequence set forth in SEQ ID NO: 21, the HCDR2 comprises or consists of an amino acid sequence set forth in SEQ ID NO: 22, and the HCDR3 comprises or consists of an amino acid sequence set forth in SEQ ID NO: 23;
the LCDR1 comprises or consists of an amino acid sequence set forth in SEQ ID NO: 24, the LCDR2 comprises or consists of an amino acid sequence set forth in SEQ ID NO: 25, and the LCDR3 comprises or consists of an amino acid sequence set forth in SEQ ID NO: 26; or,
(iii) the HCDR1 comprises or consists of an amino acid sequence set forth in SEQ ID NO: 27, the HCDR2 comprises or consists of an amino acid sequence set forth in SEQ ID NO: 28, and the HCDR3 comprises or consists of an amino acid sequence set forth in SEQ ID NO: 29;
the LCDR1 comprises or consists of an amino acid sequence set forth in SEQ ID NO: 30, the LCDR2 comprises or consists of an amino acid sequence set forth in SEQ ID NO: 31, and the LCDR3 comprises or consists of an amino acid sequence set forth in SEQ ID NO: 32.

5. The bispecific antibody according to any one of claims 1-4, wherein the second antigen-binding region comprises a heavy chain variable region VH, wherein the VH comprises or consists of an amino acid sequence set forth in SEQ ID NO: 3, 5, or 7, or an amino acid sequence having at least 80%, 85%, 90%, 91%, 92%, 93%, 94%, 95%, 96%, 97%, 98%, or 99% sequence identity to the amino acid sequence set forth in SEQ ID NO: 3, 5, or 7.

6. The bispecific antibody according to any one of claims 1-5, wherein the second antigen-binding region comprises a light chain variable region VL, wherein the VL comprises or consists of an amino acid sequence set forth in SEQ ID NO: 4, 6, or 8, or an amino acid sequence having at least 80%, 85%, 90%, 91%, 92%, 93%, 94%, 95%, 96%, 97%, 98%, or 99% sequence identity to the amino acid sequence set forth in SEQ ID NO: 4, 6, or 8.

7. The bispecific antibody according to any one of claims 1-6, wherein the second antigen-binding region comprises a heavy chain variable region VH and a light chain variable region VL, wherein
(i) the VH comprises or consists of an amino acid sequence set forth in SEQ ID NO: 3 or an amino acid sequence having at least 80%, 85%, 90%, 91%, 92%, 93%, 94%, 95%, 96%, 97%, 98%, or 99% identity to the amino acid sequence of SEQ ID NO: 3, and the VL comprises or consists of an amino acid sequence set forth in SEQ ID NO: 4 or an amino acid sequence having at least 80%, 85%, 90%, 91%, 92%, 93%, 94%, 95%, 96%, 97%, 98%, or 99% identity to the amino acid sequence of SEQ ID NO: 4;
(ii) the VH comprises or consists of an amino acid sequence set forth in SEQ ID NO: 5 or an amino acid sequence having at least 80%, 85%, 90%, 91%, 92%, 93%, 94%, 95%, 96%, 97%, 98%, or 99% identity to the amino acid sequence of SEQ ID NO: 5, and the VL comprises or consists of an amino acid sequence set forth in SEQ ID NO: 6 or an amino acid sequence having at least 80%, 85%, 90%, 91%, 92%, 93%, 94%, 95%, 96%, 97%, 98%, or 99% identity to the amino acid sequence of SEQ ID NO: 6; or
(iii) the VH comprises or consists of an amino acid sequence set forth in SEQ ID NO: 7 or an amino acid sequence having at least 80%, 85%, 90%, 91%, 92%, 93%, 94%, 95%, 96%, 97%, 98%, or 99% identity to the amino acid sequence of SEQ ID NO: 7, and the VL comprises or consists of an amino acid sequence set forth in SEQ ID NO: 8 or an amino acid sequence having at least 80%, 85%, 90%, 91%, 92%, 93%, 94%, 95%, 96%, 97%, 98%, or 99% identity to the amino acid sequence of SEQ ID NO: 8.

8. The bispecific antibody according to any one of claims 1-7, wherein the second antigen-binding region comprises or consists of a heavy chain variable region VH and a light chain variable region VL, wherein the VH and the VL comprise or consist of, respectively, amino acid sequences set forth in:
SEQ ID NO: 3 and SEQ ID NO: 4;
SEQ ID NO: 5 and SEQ ID NO: 6; or
SEQ ID NO: 7 and SEQ ID NO: 8.

9. The bispecific antibody according to any one of claims 1-7, wherein the first antigen-binding region comprises an HCDR1, an HCDR2, and an HCDR3 of a heavy chain variable region VH, and an LCDR1, an LCDR2, and an LCDR3 of a light chain variable region VL, wherein
the HCDR1, the HCDR2, and the HCDR3 are three complementarity determining regions HCDR1, HCDR2, and HCDR3 comprised in a VH set forth in SEQ ID NO: 1; and the LCDR1, the LCDR2, and the LCDR3 are three complementarity determining regions LCDR1, LCDR2, and LCDR3 comprised in a VL set forth in SEQ ID NO: 2.

10. The bispecific antibody according to claim 9, wherein the HCDR1 of the first antigen-binding region comprises or consists of an amino acid sequence of SEQ ID NO: 9; the HCDR2 comprises or consists of an amino acid sequence of SEQ ID NO: 10; the HCDR3 comprises or consists of an amino acid sequence of SEQ ID NO: 11; and the LCDR1 of the first antigen-binding region comprises or consists of an amino acid sequence of SEQ ID NO: 12; the LCDR2 comprises or consists of an amino acid sequence of SEQ ID NO: 13; and the LCDR3 comprises or consists of an amino acid sequence of SEQ ID NO: 14.

11. The bispecific antibody according to claim 9 or 10, wherein the first antigen-binding region comprises a heavy chain variable region VH, wherein the VH comprises or consists of an amino acid sequence set forth in SEQ ID NO: 1 or an amino acid sequence having at least 80%, 85%, 90%, 91%, 92%, 93%, 94%, 95%, 96%, 97%, 98%, or 99% identity to the amino acid sequence of SEQ ID NO: 1.

12. The bispecific antibody according to any one of claims 9-11, wherein the first antigen-binding region comprises a light chain variable region VL, wherein the VL comprises or consists of an amino acid sequence set forth in SEQ ID NO: 2 or an amino acid sequence having at least 80%, 85%, 90%, 91%, 92%, 93%, 94%, 95%, 96%, 97%, 98%, or 99% identity to the amino acid sequence of SEQ ID NO: 2.

13. The bispecific antibody according to any one of claims 9-12, wherein the first antigen-binding region comprises a heavy chain variable region VH and a light chain variable region VL, wherein the VH comprises or consists of an amino acid sequence set forth in SEQ ID NO: 1 or an amino acid sequence having at least 80%, 85%, 90%, 91%, 92%, 93%, 94%, 95%, 96%, 97%, 98%, or 99% identity to the amino acid sequence of SEQ ID NO: 1, and the VL comprises or consists of an amino acid sequence set forth in SEQ ID NO: 2 or an amino acid sequence having at least 80%, 85%, 90%, 91%, 92%, 93%, 94%, 95%, 96%, 97%, 98%, or 99% identity to the amino acid sequence of SEQ ID NO: 2.

14. The bispecific antibody according to any one of claims 9-13, wherein the first antigen-binding region comprises or consists of a heavy chain variable region VH and a light chain variable region VL, wherein the VH and the VL comprise or consist of, respectively, amino acid sequences set forth in: SEQ ID NO: 1 and SEQ ID NO: 2.

15. The bispecific antibody according to any one of claims 1-14, wherein the first antigen-binding region specifically binds to EGFR, and comprises an HCDR1, an HCDR2, and an HCDR3 of a heavy chain variable region VH, and an LCDR1, an LCDR2, and an LCDR3 of a light chain variable region VL, wherein
the HCDR1 comprises or consists of an amino acid sequence of SEQ ID NO: 9;
the HCDR2 comprises or consists of an amino acid sequence of SEQ ID NO: 10;
the HCDR3 comprises or consists of an amino acid sequence of SEQ ID NO: 11;
the LCDR1 comprises or consists of an amino acid sequence of SEQ ID NO: 12;
the LCDR2 comprises or consists of an amino acid sequence of SEQ ID NO: 13; and
the LCDR3 comprises or consists of an amino acid sequence of SEQ ID NO: 14;
and
the second antigen-binding region specifically binds to B7H3, and comprises an HCDR1, an HCDR2, and an HCDR3 of a heavy chain variable region VH, and an LCDR1, an LCDR2, and an LCDR3 of a light chain variable region VL, wherein
(i) the HCDR1 comprises or consists of an amino acid sequence of SEQ ID NO: 15;
the HCDR2 comprises or consists of an amino acid sequence of SEQ ID NO: 16;
the HCDR3 comprises or consists of an amino acid sequence of SEQ ID NO: 17;
the LCDR1 comprises or consists of an amino acid sequence of SEQ ID NO: 18;
the LCDR2 comprises or consists of an amino acid sequence of SEQ ID NO: 19; and
the LCDR3 comprises or consists of an amino acid sequence of SEQ ID NO: 20;
(ii) the HCDR1 comprises or consists of an amino acid sequence of SEQ ID NO: 21;
the HCDR2 comprises or consists of an amino acid sequence of SEQ ID NO: 22;
the HCDR3 comprises or consists of an amino acid sequence of SEQ ID NO: 23;
the LCDR1 comprises or consists of an amino acid sequence of SEQ ID NO: 24;
the LCDR2 comprises or consists of an amino acid sequence of SEQ ID NO: 25; and
the LCDR3 comprises or consists of an amino acid sequence of SEQ ID NO: 26; or
(iii) the HCDR1 comprises or consists of an amino acid sequence of SEQ ID NO: 27;
the HCDR2 comprises or consists of an amino acid sequence of SEQ ID NO: 28;
the HCDR3 comprises or consists of an amino acid sequence of SEQ ID NO: 29;
the LCDR1 comprises or consists of an amino acid sequence of SEQ ID NO: 30;
the LCDR2 comprises or consists of an amino acid sequence of SEQ ID NO: 31; and
the LCDR3 comprises or consists of an amino acid sequence of SEQ ID NO: 32.

16. The bispecific antibody according to claim 15, wherein the first antigen-binding region comprises a VH comprising or consisting of an amino acid sequence set forth in SEQ ID NO: 1 and a VL comprising or consisting of an amino acid sequence set forth in SEQ ID NO: 2, and the second antigen-binding region comprises a VH and a VL comprising or consisting of, respectively, amino acid sequences set forth in:
SEQ ID NO: 3 and SEQ ID NO: 4;
SEQ ID NO: 5 and SEQ ID NO: 6; or
SEQ ID NO: 7 and SEQ ID NO: 8.

17. The bispecific antibody according to any one of claims 1-16, comprising an Fc region, wherein preferably, the Fc region has low fucosylation, e.g., low fucosylation obtained after treatment by the GlymaxX technology.

18. The bispecific antibody according to claim 17, comprising a first Fc region and a second Fc region, wherein the first Fc region and the second Fc region are identical or different.

19. The bispecific antibody according to claim 17 or 18, wherein the first Fc region and the second Fc region are each a human IgG Fc, e.g., human IgG1 Fc, human IgG2 Fc, human IgG3 Fc, or human IgG4 Fc, e.g., comprising or consisting of an amino acid sequence set forth in SEQ ID NO: 46 or 47, or an amino acid sequence having at least 90% identity, e.g., 95%, 96%, 97%, 99% or more identity, thereto.

20. The bispecific antibody according to claim 18 or 19, wherein mutations that promote heterodimerization of the first Fc region and the second Fc region are introduced in the first Fc region and the second Fc region.

21. The bispecific antibody according to claim 20, wherein the mutations are introduced on the basis of the Innobody technology.

22. The bispecific antibody according to claim 21, wherein a CH3 of one Fc region comprises S364R and D399K mutations, and a CH3 of the other Fc region comprises Y349T, K370S, and K409D mutations.

23. The bispecific antibody according to claim 22, wherein
a) one Fc region polypeptide comprises or consists of an amino acid sequence set forth in SEQ ID NO: 49 or 50, and the other Fc region polypeptide comprises or consists of an amino acid sequence set forth in SEQ ID NO: 52 or 53;
b) one Fc region polypeptide comprises an amino acid sequence having at least 85%, 90%, 95%, 96%, 97%, 98%, or 99% identity to the amino acid sequence set forth in SEQ ID NO: 49 or 50, and the other Fc region polypeptide comprises an amino acid sequence having at least 85%, 90%, 95%, 96%, 97%, 98%, or 99% identity to the amino acid sequence set forth in SEQ ID NO: 52 or 53; or
c) one Fc region polypeptide comprises an amino acid sequence having at least 85%, 90%, 95%, 96%, 97%, 98%, or 99% identity to the amino acid sequence set forth in SEQ ID NO: 49 or 50 and comprises mutations Y349T, K370S, and K409D, and the other Fc region comprises an amino acid sequence having at least 85%, 90%, 95%, 96%, 97%, 98%, or 99% identity to the amino acid sequence set forth in SEQ ID NO: 52 or 53 and comprises mutations S364R and D399K.

24. The bispecific antibody according to claim 20, wherein the mutations are introduced based on the Knob-into-Hole technology, wherein corresponding Knob and Hole mutations are introduced in the first Fc region and the second Fc region.

25. The bispecific antibody according to claim 24, wherein
a) one Fc region polypeptide comprises a mutation T366W, and the other Fc region polypeptide comprises T366S, L368A, and Y407V (numbering according to EU index), or
b) one Fc region comprises amino acid substitutions S354C and T366W, and the other Fc region comprises amino acid substitutions Y349C, T366S, L368A, and Y407V (numbering according to EU index).

26. The bispecific antibody according to any one of claims 1-25, wherein the first and/or second antigen-binding regions (e.g., the heavy chain variable region therein) can also be linked to 1 or 2 heavy chain constant regions (e.g., a heavy chain constant region of human IgG1, human IgG2, human IgG3, or human IgG4) comprising a CH1 and an Fc region, via or not via a hinge region, for example, the C-terminus of the heavy chain variable region is linked to the N-terminus of the CH1 of the heavy chain constant region.

27. The bispecific antibody according to claim 26, wherein the CH1 comprises or consists of an amino acid sequence set forth in SEQ ID NO: 42 or an amino acid sequence having at least 80%, 85%, 90%, 91%, 92%, 93%, 94%, 95%, 96%, 97%, 98%, or 99% identity to the amino acid sequence of SEQ ID NO: 42.

28. The bispecific antibody according to claims 1-27, wherein the first and/or second antigen-binding regions (e.g., the light chain variable region therein) can be further linked to a light chain constant region, e.g., the C-terminus of the light chain variable region is linked to the N-terminus of the light chain constant region.

29. The bispecific antibody according to claim 28, wherein the light chain constant region is a kappa light chain constant region or a lambda light chain constant region.

30. The bispecific antibody according to claim 20, wherein the light chain constant region comprises or consists of an amino acid sequence set forth in SEQ ID NO: 54 or an amino acid sequence having at least 80%, 85%, 90%, 91%, 92%, 93%, 94%, 95%, 96%, 97%, 98%, or 99% identity to the amino acid sequence of SEQ ID NO: 54.

31. The bispecific antibody according to any one of claims 1-30, wherein the bispecific antibody is an IgG-like antibody having a configuration shown in FIG. 1.

32. The bispecific antibody according to claim 31, comprising a heavy chain 1 and a light chain 1, and a heavy chain 2 and a light chain 2, wherein the heavy chain 1 and the light chain 1 constitute a first half antibody, and the heavy chain 2 and the light chain 2 constitute a second half antibody, wherein
the heavy chain 1 comprises the heavy chain variable region of the first antigen-binding region and a first heavy chain constant region; the light chain 1 comprises the light chain variable region of the first antigen-binding region and a first light chain constant region; and the heavy chain 2 comprises the heavy chain variable region of the second antigen-binding region and a second heavy chain constant region; the light chain 2 comprises the light chain variable region of the second antigen-binding region and a second light chain constant region.

33. The bispecific antibody according to claim 32, wherein the heavy chain 1 comprises or consists of an amino acid sequence set forth in SEQ ID NO: 33 or an amino acid sequence having at least 80%, 85%, 90%, 91%, 92%, 93%, 94%, 95%, 96%, 97%, 98%, or 99% identity to the amino acid sequence of SEQ ID NO: 33.

34. The bispecific antibody according to claim 32 or 33, wherein the light chain 1 comprises or consists of an amino acid sequence set forth in SEQ ID NO: 34 or an amino acid sequence having at least 80%, 85%, 90%, 91%, 92%, 93%, 94%, 95%, 96%, 97%, 98%, or 99% identity to the amino acid sequence of SEQ ID NO: 34.

35. The bispecific antibody according to any one of claims 32-34, wherein the heavy chain 1 comprises or consists of an amino acid sequence set forth in SEQ ID NO: 33 or an amino acid sequence having at least 80%, 85%, 90%, 91%, 92%, 93%, 94%, 95%, 96%, 97%, 98%, or 99% identity to the amino acid sequence of SEQ ID NO: 33, and the light chain 1 comprises or consists of an amino acid sequence set forth in SEQ ID NO: 34 or an amino acid sequence having at least 80%, 85%, 90%, 91%, 92%, 93%, 94%, 95%, 96%, 97%, 98%, or 99% identity to the amino acid sequence of SEQ ID NO: 34.

36. The bispecific antibody according to any one of claims 32-35, wherein the heavy chain 2 comprises or consists of an amino acid sequence set forth in SEQ ID NO: 35, 37, or 39 or an amino acid sequence having at least 80%, 85%, 90%, 91%, 92%, 93%, 94%, 95%, 96%, 97%, 98%, or 99% identity to the amino acid sequence set forth in SEQ ID NO: 35, 37, or 39.

37. The bispecific antibody according to any one of claims 32-36, wherein the light chain 2 comprises or consists of an amino acid sequence set forth in SEQ ID NO: 36, 38, or 40 or an amino acid sequence having at least 80%, 85%, 90%, 91%, 92%, 93%, 94%, 95%, 96%, 97%, 98%, or 99% identity to the amino acid sequence set forth in SEQ ID NO: 36, 38, or 40.

38. The bispecific antibody according to any one of claims 32-37, wherein
(1) the heavy chain 2 comprises or consists of an amino acid sequence set forth in SEQ ID NO: 35 or an amino acid sequence having at least 80%, 85%, 90%, 91%, 92%, 93%, 94%, 95%, 96%, 97%, 98%, or 99% identity to the amino acid sequence set forth in SEQ ID NO: 35; and the light chain 2 comprises or consists of an amino acid sequence set forth in SEQ ID NO: 36 or an amino acid sequence having at least 80%, 85%, 90%, 91%, 92%, 93%, 94%, 95%, 96%, 97%, 98%, or 99% identity to the amino acid sequence set forth in SEQ ID NO: 36;
(2) the heavy chain 2 comprises or consists of an amino acid sequence set forth in SEQ ID NO: 37 or an amino acid sequence having at least 80%, 85%, 90%, 91%, 92%, 93%, 94%, 95%, 96%, 97%, 98%, or 99% identity to the amino acid sequence set forth in SEQ ID NO: 37; and the light chain 2 comprises or consists of an amino acid sequence set forth in SEQ ID NO: 38 or an amino acid sequence having at least 80%, 85%, 90%, 91%, 92%, 93%, 94%, 95%, 96%, 97%, 98%, or 99% identity to the amino acid sequence set forth in SEQ ID NO: 38;
(3) the heavy chain 2 comprises or consists of an amino acid sequence set forth in SEQ ID NO: 39 or an amino acid sequence having at least 80%, 85%, 90%, 91%, 92%, 93%, 94%, 95%, 96%, 97%, 98%, or 99% identity to the amino acid sequence set forth in SEQ ID NO: 39; and the light chain 2 comprises or consists of an amino acid sequence set forth in SEQ ID NO: 40 or an amino acid sequence having at least 80%, 85%, 90%, 91%, 92%, 93%, 94%, 95%, 96%, 97%, 98%, or 99% identity to the amino acid sequence set forth in SEQ ID NO: 40.

39. The bispecific antibody according to any one of claims 32-38, wherein
the heavy chain 1 comprises or consists of an amino acid sequence set forth in SEQ ID NO: 33 or an amino acid sequence having at least 80%, 85%, 90%, 91%, 92%, 93%, 94%, 95%, 96%, 97%, 98%, or 99% identity to the amino acid sequence of SEQ ID NO: 33, and the light chain 1 comprises or consists of an amino acid sequence set forth in SEQ ID NO: 34 or an amino acid sequence having at least 80%, 85%, 90%, 91%, 92%, 93%, 94%, 95%, 96%, 97%, 98%, or 99% identity to the amino acid sequence of SEQ ID NO: 34; and the heavy chain 2 and the light chain 2 comprise or consist of, respectively, amino acid sequences set forth in the following SEQ ID NOs or amino acid sequences having at least 85%, 90%, 95%, 96%, 97%, 98%, or 99% identity thereto:
i) SEQ ID NO: 35 and SEQ ID NO: 36;
ii) SEQ ID NO: 37 and SEQ ID NO: 38;
iii) SEQ ID NO: 39 and SEQ ID NO: 40.

40. The bispecific antibody according to any one of claims 32-39, wherein,
(i) the heavy chain 1 comprises or consists of an amino acid sequence set forth in SEQ ID NO: 33, and the light chain 1 comprises or consists of an amino acid sequence set forth in SEQ ID NO: 34;
the heavy chain 2 comprises or consists of an amino acid sequence set forth in SEQ ID NO: 35, and the light chain 2 comprises or consists of an amino acid sequence set forth in SEQ ID NO: 36; or,
(ii) the heavy chain 1 comprises or consists of an amino acid sequence set forth in SEQ ID NO: 33, and the light chain 1 comprises or consists of an amino acid sequence set forth in SEQ ID NO: 34;
the heavy chain 2 comprises or consists of an amino acid sequence set forth in SEQ ID NO: 37, and the light chain 2 comprises or consists of the amino acid sequence set forth in SEQ ID NO: 38; or,
(iii) the heavy chain 1 comprises or consists of an amino acid sequence set forth in SEQ ID NO: 33, and the light chain 1 comprises or consists of an amino acid sequence set forth in SEQ ID NO: 34;
the heavy chain 2 comprises or consists of an amino acid sequence set forth in SEQ ID NO: 39, and the light chain 2 comprises or consists of the amino acid sequence set forth in SEQ ID NO: 40.

41. The bispecific antibody or the antigen-binding fragment thereof binding to EGFR and B7-H3 according to any one of claims 1-40, wherein the antibody or the antigen-binding fragment thereof has one or more of the following properties:
(i) in one aspect, the antibody can block the binding of an EGFR ligand to EGFR, thereby inhibiting biological signaling and blocking the corresponding biological activity of a tumor; in another aspect, the antibody stimulates the endocytosis of EGFR and thus enables the final degradation of EGFR by intracellular lysosomes and the like;
(ii) the antibody adopts an EGFR antibody parent sequence with a low affinity for EGFR, thereby greatly reducing toxic and side effects of a series of monoclonal EGFR antibodies on normal epithelial tissues such as the skin;
(iii) the antibody introduces a parent antibody sequence with a high affinity for B7-H3 on the basis of the low affinity for EGER, thereby greatly improving the blocking activity against EGFR signaling and improving the pharmacodynamic biological activity and the efficacy and safety windows of the bispecific antibody of the present disclosure;
(iv) the antibody is a low-fucosylated antibody;
(ii) the antibody has relatively high pharmacodynamic biological activity and safety;
(v) the antibody has excellent tumor killing and inhibitory effects;
(vi) the antibody has excellent *in-vivo* and *in-vitro* ADCC pharmacodynamic activity;
(vii) the antibody has an excellent synergistic anti-tumor effect when used in combination with a KRAS small-molecule inhibitor.

42. An isolated nucleic acid encoding any one of the chains of the bispecific antibody binding to EGFR and B7-H3 according to any one of claims 1-41.

43. A vector, comprising the nucleic acid according to claim 42, wherein preferably, the vector is an expression vector; preferably, the expression vector is a pcDNA, e.g., pcDNA3.1.

44. A host cell, comprising the nucleic acid according to claim 42 or the vector according to claim 43, wherein preferably, the host cell is prokaryotic or eukaryotic, more preferably a yeast cell or a mammalian cell (e.g., a 293 cell or a CHO cell, e.g., a 293F cell, a 293T cell, or a CHO-S cell).

45. The host cell according to claim 44, wherein the host cell is glycoengineered to express an RMD enzyme; preferably, the host cell is a CHO cell.

46. The host cell according to claim 45, comprising a nucleic acid encoding the RMD enzyme.

47. The host cell according to claim 46, wherein the RMD enzyme comprises or consists of an amino acid sequence set forth in SEQ ID NO: 41 or an amino acid sequence having at least 90% identity thereto; preferably, the RMD enzyme is derived from *Pseudomonas aeruginosa.*

48. A method for preparing the bispecific antibody binding to EGFR and B7-H3, wherein the method comprises culturing the host cell according to any one of claims 44-47 under conditions suitable for expression of a nucleic acid encoding the bispecific antibody according to any one of the preceding claims 1-41, and optionally, isolating the antibody or the antigen-binding fragment thereof, and optionally, the method further comprises recovering the antibody or the antigen-binding fragment thereof from the host cell (or host cell culture medium).

49. An immunoconjugate, comprising the bispecific antibody according to any one of claims 1-41 conjugated to a therapeutic agent or a diagnostic agent.

50. A pharmaceutical composition, comprising: the bispecific antibody according to any one of claims 1-41 or the immunoconjugate according to claim 49, and optionally, a pharmaceutical supplementary material.

51. The pharmaceutical composition according to claim 50, further comprising a second therapeutic agent, wherein preferably, the second therapeutic agent is selected from an anti-angiogenic agent, a chemotherapeutic agent, an additional antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is selected from a KRAS small molecule inhibitor, e.g., a KRAS G12C inhibitor (e.g., AMG510 (sotorasib) or GFH925), or a KRAS G12D (e.g., MRTX1133) or KRAS G12S inhibitor.

52. A pharmaceutical combination product, comprising the bispecific antibody according to any one of claims 1-41 or the immunoconjugate according to claim 49 or the pharmaceutical composition product according to claim 50, and one or more second therapeutic agents, wherein preferably, the second therapeutic agent is selected from an anti-angiogenic agent, a chemotherapeutic agent, an additional antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is selected from a KRAS small molecule inhibitor, e.g., a KRAS G12C inhibitor (e.g., AMG510 (sotorasib) or GFH925), or a KRAS G12D (e.g. MRTX1133) or KRAS G12S inhibitor.

53. A method for preventing or treating a tumor or an infectious disease in a subject, wherein the method comprises administering to the subject an effective amount of the bispecific antibody according to any one of claims 1-41, or the immunoconjugate according to claim 49, or the pharmaceutical composition according to claim 50.

54. The method according to claim 53, further comprising co-administering to the subject one or more additional therapies, wherein the therapy, for example, comprises a therapeutic modality and/or an additional therapeutic agent, wherein preferably, the therapeutic modality comprises surgical treatment and/or radiotherapy, or the therapeutic agent is selected from an anti-angiogenic agent, a chemotherapeutic agent, an additional antibody, a cytotoxic agent, a vaccine, an anti-infective active agent, a small molecule drug, or an immunomodulatory agent (e.g., an activator of a co-stimulatory molecule or an inhibitor of an immune checkpoint molecule); preferably, the second therapeutic agent is selected from a KRAS small molecule inhibitor, e.g., a KRAS G12C inhibitor (e.g. AMG510 (sotorasib) or GFH925), or a KRAS G12D (e.g., MRTX1133) or KRAS G12S inhibitor.

55. A method for preventing or treating a tumor or an infectious disease in a subject, wherein the method comprises administering to the subject an effective amount of the pharmaceutical composition according to claim 51 or the pharmaceutical combination product according to claim 52.

56. The method according to any one of claims 53 to 55, wherein the tumor is a cancer, e.g., a solid tumor or a hematological tumor, including a cancer of epithelial origin, e.g., a gastrointestinal tumor, a lung tumor, or a skin tumor, e.g., a skin cancer (e.g., cutaneous squamous cell carcinoma, or head and neck cancer such as squamous cell carcinoma of the head and neck), an esophageal cancer (e.g., esophageal squamous cell carcinoma), an intestinal cancer (e.g., colon cancer, rectal cancer, or colorectal cancer), or a lung cancer (e.g., non-small cell lung cancer, lung squamous cell carcinoma, or lung adenocarcinoma).

57. The method according to any one of claims 53-56, wherein tumor cells of the tumor
(i) overexpress wild-type EGFR (e.g., wild-type EGFR with an increased nucleic acid or protein level) and/or express mutant EGFR, wherein preferably, the mutant EGFR comprises one or more mutations selected from R521K, L858R, T790M, G719X, C797S, Y1069C, Exon19 deletion (Del19), Exon20ins (e.g., S768_D770dup), and preferably, the mutant EGFR comprises R521K/Y1069C, R521K, L858R/T790M/C797S, Del19/T790M/C797S or S768_D770dup, compared with normal cells of an adjacent tissue or compared with normal cells of the same tissue in a healthy subject;
(ii) overexpress wild-type KRAS (e.g., wild-type KRAS with an increased nucleic acid or protein level) or express mutant KRAS compared with normal cells of an adjacent tissue or compared with normal cells of the same tissue in a healthy subject, wherein preferably, the mutant KRAS comprises a mutation at position G12 or G13, e.g., G12D or G12C;
(iii) have B7-H3 with an increased nucleic acid or protein level compared with normal cells of an adjacent tissue or compared with normal cells of the same tissue in a healthy subject; and/or
(iv) the tumor cells are resistant to a tyrosine kinase inhibitor, e.g., to the first-generation (erlotinib) and the third-generation (osimertinib), e.g., to osimertinib.

58. The method according to claim 57, wherein the tumor cells of the tumor express the mutant EGFR and the mutant KRAS, e.g., comprise EGFR having a mutation R521K and KRAS having a mutation G120D.

59. A method for detecting antigens EGFR and/or B7-H3 in a sample, wherein the method comprises
(a) bringing a sample into contact with the bispecific antibody according to any one of claims 1-41; and
(b) detecting a complex formed by the antibody or the antigen-binding fragment thereof with EGFR and/or B7-H3, wherein optionally, the antibody is detectably labeled.

60. Use of the antibody or the antigen-binding fragment thereof according to any one of claims 1-41, and/or the isolated nucleic acid according to claim 42, and/or the vector according to claim 43, and/or the host cell according to any one of claims 44-47, and/or the immunoconjugate according to claim 49, and/or the pharmaceutical composition according to claim 50 or 51 or the pharmaceutical combination product according to claim 52, in the preparation of a drug for preventing and/or treating a disease in a subject.
